# EUROPEAN PATENT SPECIFICATION

(11) **EP 1 575 966 B1**
(45) Date of publication and mention of the grant of the patent: **29.10.2008**
(21) Application number: 03789562.0
(22) Date of filing: 22.12.2003
(51) Int. Cl.: C07F 9/6558, C07F 9/6512, A61K 31/675, A61P 35/00

(54) **THERAPEUTIC QUINAZOLINE DERIVATIVES**
THERAPEUTISCHE QUINAZOLIN-DERIVATE
DERIVES THERAPEUTIQUES DE QUINAZOLINE

(30) Priority: 24.12.2002 EP 02293240
(43) Date of publication of application: 21.09.2005
(73) Proprietor: AstraZeneca AB, 151 85 Södertälje (SE)
(72) Inventor: MORTLOCK, Andrew, Austen, AstraZeneca R & D, Macclesfield, Cheshire SK10 4TG (GB)
(86) International application number: PCT/GB2003/005640
(87) International publication number: WO 2004/058782

(56) References cited:
- WO-A-00/56720
- WO-A-01/21594
- WO-A-01/21597
- WO-A-03/000288

## Description

The present invention relates to certain quinazoline derivatives for use in the treatment of certain diseases in particular to proliferative disease such as cancer and in the preparation of medicaments for use in the treatment of proliferative disease, to novel quinazoline compounds and to processes for their preparation, as well as pharmaceutical compositions containing them as active ingredient.

Cancer (and other hyperproliferative disease) is characterised by uncontrolled cellular proliferation. This loss of the normal regulation of cell proliferation often appears to occur as the result of genetic damage to cellular pathways that control progress through the cell cycle.

In eukaryotes, an ordered cascade of protein phosphorylation is thought to control the cell cycle. Several families of protein kinases that play critical roles in this cascade have now been identified. The activity of many of these kinases is increased in human tumours when compared to normal tissue. This can occur by either increased levels of expression of the protein (as a result of gene amplification for example), or by changes in expression of co activators or inhibitory proteins.

The first identified, and most widely studied of these cell cycle regulators have been the cyclin dependent kinases (or CDKs). Activity of specific CDKs at specific times is essential for both initiation and coordinated progress through the cell cycle. For example, the CDK4 protein appears to control entry into the cell cycle (the G0-G1-S transition) by phosphorylating the retinoblastoma gene product pRb. This stimulates the release of the transcription factor E2F from pRb, which then acts to increase the transcription of genes necessary for entry into S phase. The catalytic activity of CDK4 is stimulated by binding to a partner protein, Cyclin D. One of the first demonstrations of a direct link between cancer and the cell cycle was made with the observation that the Cyclin D 1 gene was amplified and cyclin D protein levels increased (and hence the activity of CDK4 increased) in many human tumours (Reviewed in Sherr, 1996, Science 274: 1672-1677; Pines, 1995, Seminars in Cancer Biology 6: 63-72). Other studies (Loda et al., 1997, Nature Medicine 3(2): 231-234; Gemma et al., 1996, International Journal of Cancer 68(5): 605-11; Elledge et al. 1996, Trends in Cell Biology 6; 388-392) have shown that negative regulators of CDK function are frequently down regulated or deleted in human tumours again leading to inappropriate activation of these kinases.

More recently, protein kinases that are structurally distinct from the CDK family have been identified which play critical roles in regulating the cell cycle and which also appear to be important in oncogenesis. These include the newly identified human homologues of the *Drosophila* aurora and *S.cerevisiae* Ipl1 proteins. The three human homologues of these genes Aurora-A, Aurora-B and Aurora-C (also known as aurora2, aurora1 and aurora3 respectively) encode cell cycle regulated serine-threonine protein kinases (summarised in Adams et al., 2001, Trends in Cell Biology. 11 (2): 49-54). These show a peak of expression and kinase activity through G2 and mitosis. Several observations implicate the involvement of human aurora proteins in cancer. The Aurora-A gene maps to chromosome 20q13, a region that is frequently amplified in human tumours including both breast and colon tumours. Aurora-A may be the major target gene of this amplicon, since Aurora-A DNA is amplified and mRNA overexpressed in greater than 50% of primary human colorectal cancers. In these tumours Aurora-A protein levels appear greatly elevated compared to adjacent normal tissue. In addition, transfection of rodent fibroblasts with human Aurora-A leads to transformation, conferring the ability to grow in soft agar and form tumours in nude mice (Bischoff et al., 1998, The EMBO Journal. 17(11): 3052-3065). Other work (Zhou et al., 1998, Nature Genetics. 20(2): 189-93) has shown that artificial overexpression of Aurora-A leads to an increase in centrosome number and an increase in aneuploidy, a known event in the development of cancer. Further work has shown an increase in expression of Aurora-B (Adams et al., 2001, Chromsoma. 110(2):65-74) and Aurora-C (Kimura et al., 1999, Journal of Biological Chemistry, 274(11): 7334-40) in tumour cells when compared to normal cells.

Importantly, it has also been demonstrated that abrogation of Aurora-A expression and function by antisense oligonucleotide treatment of human tumour cell lines (WO 97/22702 and WO 99/37788) leads to cell cycle arrest and exerts an antiproliferative effect in these tumour cell lines. Additionally, small molecule inhibitors of Aurora-A and Aurora-B have been demonstrated to have an antiproliferative effect in human tumour cells (Keen et al. 2001, Poster #2455, American Association of Cancer research annual meeting), as has selective abrogation of Aurora-B expression alone by siRNA treatment (Ditchfield et al. 2003. Journal of Cell Biology, 161(2): 267-280). This indicates that inhibition of the function of Aurora-A and/or Aurora-B will have an antiproliferative effect that may be useful in the treatment of human tumours and other hyperproliferative disease. Further, inhibition of Aurora kinases as a therapeutic approach to these diseases may have significant advantages over targeting signalling pathways upstream of the cell cycle (e.g. those activated by growth factor receptor tyrosine kinases such as epidermal growth factor receptor (EGFR) or other receptors). Since the cell cycle is ultimately downstream of all of these diverse signalling events, cell cycle directed therapies such as inhibition of Aurora kinases would be predicted to be active across all proliferating tumour cells, whilst approaches directed at specific signalling molecules (e.g. EGFR) would be predicted to be active only in the subset of tumour cells which express those receptors. It is also believed that significant "cross talk" exists between these signalling pathways meaning that inhibition of one component may be compensated for by another.

A number of quinazoline derivatives have been proposed hitherto for use in the inhibition of various kinases. For example, WO 96/09294, WO 96/15118 and WO 99/06378 describe the use of certain quinazoline compounds as receptor tyrosine kinase inhibitors, which may be useful in the treatment of proliferative disease and WO 00/21955 discloses certain quinazoline derivatives as inhibitors of the effects of VEGF.

Quinazoline derivatives have also been disclosed for use in the inhibition of Aurora-A kinase. WO 01/21597 discloses quinazoline derivative bearing a 6-membered aromatic ring containing at least one nitrogen atom. However despite the compounds of WO 01/21597 there still exists the need for further compounds having Aurora kinase inhibitory properties.

The applicants have been successful in finding a novel series of compounds which inhibit the effects of the Aurora kinases and in particular Aurora-A and/or Aurora-B kinase and which have certain properties that make them particularly useful in formulating medicaments for the treatment of disease. In particular the compounds are of use in the treatment of proliferative disease such as cancer. The compound are useful in treating either solid or haematological tumours where Aurora kinases are known to be active, and especially in diseases such as colorectal, breast, lung, prostate, pancreatic or bladder and renal cancer as well as leukemias and lymophomas.

According to one aspect of the present invention there is provided a compound of formula (I): 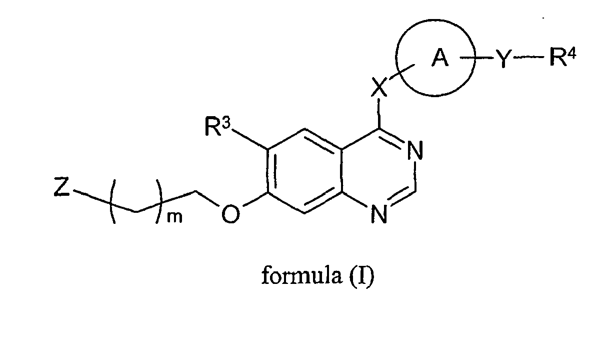
wherein **A** is 6-membered heteroaryl containing a nitrogen atom and optionally containing one or two further nitrogen atoms;
**X** is O, S, S(O), S(O)₂ or NR¹⁴;
**m** is 0, 1, 2, 3 or 4;
**Y** is a group selected from O, NR⁵CO, CONR⁵, CR⁶R⁷CONR⁵ and CR⁶R⁷NR⁵;
**Z** is a group selected from -NR¹R², phosphonooxy, C₃₋₆cycloalkyl which C₃₋₆cycloalkyl is substituted by phosphonooxy or C₁₋₄alkyl substituted by phosphonooxy, and a 4- to 7-membered ring linked via a carbon atom containing a nitrogen atom and optionally containing a further nitrogen atom, which ring may be saturated, unsaturated or partially saturated which ring is substituted on carbon or nitrogen by phosphonooxy or C₁₋₄alkyl (substituted by phosphonooxy) and which ring is optionally further substituted on carbon or nitrogen by 1, 2 or 3 halo or C₁₋₄alkyl groups;
**R¹** is a group selected from -COR⁸, -CONR⁸R⁹ and C₁₋₆alkyl which C₁₋₆alkyl is substituted by phosphonooxy and optionally further substituted by 1 or 2 halo or methoxy groups;
**R²** is a group selected from hydrogen, -COR¹⁰, -CONR¹⁰R¹¹ and C₁₋₆alkyl which C₁₋₆alkyl is optionally substituted by 1, 2 or 3 halo or C₁₋₄alkoxy groups, -S(O)ₚR¹¹ (where p is 0, 1 or 2) or phosphonooxy, or R² is a group selected from C₂₋₆alkenyl, C₂₋₆alkynyl, C₃₋₆cycloalkyl and C₃₋₆cycloalkylC₁₋₄alkyl;
or **R¹** and **R²** together with the nitrogen to which they are attached form a 4- to 7- membered ring optionally containing a further nitrogen atom which ring may be saturated, unsaturated or partially saturated which ring is substituted on carbon or nitrogen by a group selected from phosphonooxy and C₁₋₄alkyl substituted by phosphonooxy or -NR⁸R⁹, and which ring is optionally further substituted on carbon or nitrogen by 1, 2 or 3 halo or C₁₋₄alkyl groups;
**R³** is a group selected from hydrogen, halo, cyano, nitro, C₁₋₆alkoxy, C₁₋₆alkyl, -OR¹², - CHR¹²R¹³, -OC(O)R¹², -C(O)R¹², -NR¹²C(O)R¹³, -C(O)NR¹²R¹³, -NR¹²SO₂R¹³ and - NR¹²R¹³;
**R⁴** is hydrogen or a group selected from C₁₋₄alkyl, heteroaryl, heteroarylC₁₋₄alkyl, aryl and arylC₁₋₄alkyl which group is optionally substituted by 1, 2 or 3 substitutents selected from halo, methyl, ethyl, cyclopropyl and ethynyl;
**R⁵** is a group selected from hydrogen, C₁₋₄alkyl, C₂₋₄alkenyl, C₂₋₄alkynyl, C₃₋₆cycloalkyl and C₃₋₆cycloalkylC₁₋₄alkyl;
**R⁶** and **R⁷** are independently selected from hydrogen, halo, C₁₋₄alkyl, C₃₋₆cycloalkyl, hydroxy and C₁₋₄alkoxy;
**R⁸** is C₁₋₄alkyl substituted by phosphonooxy and optionally further substituted by 1 or 2 halo or methoxy groups;
**R⁹** is selected from hydrogen and C₁₋₄alkyl;
**R¹⁰** is selected from hydrogen and C₁₋₄alkyl which C₁₋₄alkyl is optionally substituted by halo, C₁₋₄alkoxy, S(O)_{q} (where q is 0, 1 or 2) or phosphonoxy;
**R¹¹, R¹², R¹³** and **R¹⁴** are independently selected from hydrogen, C₁₋₄alkyl and heterocyclyl;
or a pharmaceutically acceptable salt thereof.

Within the present invention, it is to be understood that, insofar as certain compounds of formula (I) herein defined may exist in optically active or racemic forms by virtue of one or more asymmetric carbon or sulphur atoms, the invention includes in its definition any such optically active or racemic form which possesses Aurora kinase inhibitory activity and in particular Aurora-A and/or Aurora-B kinase inhibitory activity. The synthesis of optically active forms may be carried out by standard techniques of organic chemistry well known in the art, for example by synthesis from optically active starting materials or by resolution of a racemic form. Similarly, the above-mentioned activity may be evaluated using the standard laboratory techniques referred to herein.

Within the present invention it is to be understood that a compound of formula (I) or a salt thereof may exhibit the phenomenon of tautomerism and that the formulae drawings within this specification can represent only one of the possible tautomeric forms. It is to be understood that the invention encompasses any tautomeric form which has Aurora kinase inhibitory activity and in particular Aurora-A and/or Aurora-B kinase inhibitory activity and is not to be limited merely to any one tautomeric form utilised within the formulae drawings.

It is also to be understood that certain compounds of formula (I) and salts thereof can exist in solvated as well as unsolvated forms such as, for example, hydrated forms. It is to be understood that the invention encompasses all such solvated forms which have Aurora kinase inhibitory activity and in particular Aurora-A and/or Aurora-B kinase inhibitory activity.

The present invention relates to the compounds of formula (I) as herein defined as well as to the salts thereof. Salts for use in pharmaceutical compositions will be pharmaceutically acceptable salts, but other salts may be useful in the production of the compounds of formula (I) and their pharmaceutically acceptable salts. Pharmaceutically acceptable salts of the invention may, for example, include acid addition salts of compounds of formula (I) as herein defined which are sufficiently basic to form such salts. Such acid addition salts include but are not limited to furmarate, methanesulphonate, hydrochloride, hydrobromide, citrate and maleate salts and salts formed with phosphoric and sulphuric acid. In addition where compounds of formula (I) are sufficiently acidic, salts are base salts and examples include but are not limited to, an alkali metal salt for example sodium or potassium, an alkaline earth metal salt for example calcium or magnesium, or organic amine salt for example triethylamine, ethanolamine, diethanolamine, triethanolamine, morpholine, N-methylpiperidine, *N*-ethylpiperidine, dibenzylamine or amino acids such as lysine.

The compounds of formula (I) may also be provided as *in vivo* hydrolysable esters. An *in vivo* hydrolysable ester of a compound of formula (I) containing carboxy or hydroxy group is, for example a pharmaceutically acceptable ester which is cleaved in the human or animal body to produce the parent acid or alcohol. Such esters can be identified by administering, for example, intravenously to a test animal, the compound under test and subsequently examining the test animal's body fluid.

Suitable pharmaceutically acceptable esters for carboxy include C₁₋₆alkoxymethyl esters for example methoxymethyl; C₁₋₆alkanoyloxymethyl esters for example pivaloyloxymethyl, phthalidyl esters; C₃₋₈cycloalkoxycarbonyloxyC₁₋₆alkyl esters for example 1-cyclohexylcarbonyloxyethyl; 1,3-dioxolen-2-onylmethyl esters for example 5-methyl-1,3-dioxolen-2-onylmethyl; and C₁₋₆alkoxycarbonyloxyethyl esters for example 1-methoxycarbonyloxyethyl and may be formed at any carboxy group in the compounds of this invention.

Suitable pharmaceutically acceptable esters for hydroxy include inorganic esters such as phosphate esters (including phosphoramidic cyclic esters) and α-acyloxyalkyl ethers and related compounds which as a result of the *in vivo* hydrolysis of the ester breakdown to give the parent hydroxy group/s. Examples of α-acyloxyalkyl ethers include acetoxymethoxy and 2,2-dimethylpropionyloxymethoxy. A selection of *in vivo* hydrolysable ester forming groups for hydroxy include C₁-₁₀alkanoyl, for example formyl, acetyl, benzoyl, phenylacetyl, substituted benzoyl and phenylacetyl; C₁-₁₀alkoxycarbonyl (to give alkyl carbonate esters), for example ethoxycarbonyl; di-C₁-₄alkylcarbamoyl and *N*-(di-C₁-₄alkylaminoethyl)-*N-*C₁-₄alkylcarbamoyl (to give carbamates); di-C₁-₄alkylaminoacetyl and carboxyacetyl. Examples of ring substituents on phenylacetyl and benzoyl include aminomethyl, C₁₋₄alkylaminomethyl and di-(C₁-₄alkyl)aminomethyl, and morpholino or piperazino linked from a ring nitrogen atom via a methylene linking group to the 3- or 4- position of the benzoyl ring. Other interesting *in vivo* hydrolysable esters include, for example, R^{A}C(O)OC₁₋₆alkyl-CO-, wherein R^{A} is for example, benzyloxy-C₁-₄alkyl, or phenyl. Suitable substituents on a phenyl group in such esters include, for example, 4-C₁-₄piperazino-C₁-₄alkyl, piperazino-C₁-₄alkyl and morpholino-C₁-₄alkyl.

In this specification the generic term "alkyl" includes both straight-chain and branched-chain alkyl groups. However references to individual alkyl groups such as "propyl" are specific for the straight chain version only and references to individual branched-chain alkyl groups such as "*tert*-butyl" are specific for the branched chain version only. An analogous convention applies to other generic terms, for example "alkenyl" and "alkynyl".

"Cycloalkyl" is a monocyclic, saturated alkyl ring and "aryl" is a monocyclic or bicyclic aromatic ring.

Unless otherwise specified "heteroaryl" is a monocyclic or bicyclic aromatic ring containing 5 to 10 ring atoms of which 1, 2, 3 or 4 ring atoms are chosen from nitrogen, sulphur or oxygen where a ring nitrogen or sulphur may be oxidised.

"Heterocyclyl" is a saturated, unsaturated or partially saturated, monocyclic or bicyclic ring containing 4 to 12 atoms of which 1, 2, 3 or 4 ring atoms are chosen from nitrogen, sulphur or oxygen, which ring may be carbon or nitrogen linked, wherein a -CH₂-group can optionally be replaced by a -C(O)-; wherein a ring nitrogen or sulphur atom is optionally oxidised to form the N-oxide or S-oxide(s); wherein a ring -NH is optionally substituted by acetyl, formyl, methyl or mesyl; and which a ring is optionally substituted by one or more halo.

"Phosphonooxy" is in one aspect a group of formula -OP(O)(OH)₂. However the term "phosphonooxy" also includes salts of this group such as those formed with alkali metal ions such as sodium or potassium ions or alkaline earth metal ions, for example calcium or magnesium ions.

Where optional substituents are chosen from "1 or 2", from "1, 2, or 3" or from "1, 2, 3 or 4" groups or substituents it is to be understood that this definition includes all substituents being chosen from one of the specified groups i.e. all substitutents being the same or the substituents being chosen from two or more of the specified groups i.e. the substitutents not being the same.

Compounds of the present invention have been named with the aid of computer software (ACD/Name version 6.6 or ACD Name Batch version 6.0).

Suitable values for any R group (R¹ to R¹⁴) or any part or substitutent for such groups include:
- for C₁₋₄alkyl:: methyl, ethyl, propyl, isopropyl, butyl, 2-methylpropyl and *tert*-butyl;
- for C₁₋₆alkyl:: C₁₋₄alkyl, pentyl, 2,2-dimethylpropyl, 3-methylbutyl and hexyl;
- for C₂₋₄alkenyl:: vinyl, allyl and 1-propenyl;
- for C₂₋₆alkenyl:: C₂₋₄alkenyl, 1-butenyl, 2-butenyl, 3-butenyl, 2-methylbut-2-enyl, 3-methylbut-1-enyl, 1-pentenyl, 3-pentenyl and 4-hexenyl;
- for C₂₋₄alkynyl:: ethynyl, 1-propynyl, 2-propynyl and 3-butynyl;
- for C₂₋₆alkynyl:: C₂₋₄alkynyl, 2-pentynyl, hexynyl and 1-methylpent-2-ynyl;
- for C₃₋₆cycloalkyl:: cyclopropyl, cyclobutyl, cyclopentyl and cyclohexyl;
- for C₃₋₆cycloalkylC₁₋₄alkyl:: cyclopropylmethyl, cyclopropylethyl, cyclobutylmethyl, cyclopentylmethyl and cyclohexylmethyl;
- for aryl:: phenyl and naphthyl;
- for arylC₁₋₄alkyl:: benzyl, phenethyl, naphthylmethyl and naphthylethyl;
- for halo:: fluoro, chloro, bromo and iodo;
- for C₁₋₄alkoxy:: methoxy, ethoxy, propoxy and isopropoxy;
- for C₁₋₆alkoxy:: C₁₋₄alkoxy, pentyloxy, 1-ethylpropoxy and hexyloxy;
- for heteroaryl:: pyridyl, imidazolyl, quinolinyl, cinnolyl, pyrimidinyl, thiophenyl, pyrrolyl, pyrazolyl, thiazolyl, triazolyl, oxazolyl, isoxazolyl and pyrazinyl; preferably thiazolyl, pyridyl, imidazolyl and pyrimidinyl; and more preferably pyridyl and pyrimidinyl;
- for heteroarylC₁₋₄alkyl:: pyridylmethyl, pyridylethyl, pyrimidinylethyl, pyrimidinylpropyl, pyrimidinylbutyl, imidazolylpropyl, imidazolylbutyl, quinolinylpropyl, 1,3,4-triazolylpropyl and oxazolylmethyl;
- for heterocyclyl:: furyl, thienyl, pyrrolyl, pyrrolidinyl, imidazolyl, triazolyl, thiazolyl, tetrazolyl, oxazolyl, isoxazolyl, pyrazolyl, pyridyl, pyrimidinyl, pyrazinyl, pyridazinyl, triazinyl, quinolinyl, isoquinolinyl, quinoxalinyl, benzothiazolyl, benzoxazolyl, benzothienyl, benzofuryl, piperidinyl, *N*-acetylpiperidinyl, *N*-methylpiperidinyl, *N-*formylpiperazinyl, *N*-mesylpiperazinyl, homopiperazinyl, piperazinyl, azetidinyl, oxetanyl, morpholinyl, tetrahydroisoquinolinyl, tetrahydroquinolinyl, indolinyl, pyranyl, dihydro-2*H*-pyranyl, tetrahydrofuranyl, 2,5-dioximidazolidinyl, 2,2-dimethyl-1,3-dioxolanyl and 3,4-dimethylenedioxybenzyl.

It should be noted that examples given for terms used in the description are not limiting.

Preferred values of A, X, m, Y, Z, R³ and R⁴ are as follows. Such values may be used where appropriate with any of the definitions, claims or embodiments defined herein.

In one aspect of the invention A is pyridyl or pyrimidinyl. In a further aspect A is a group of formula (a), (b), (c) or (d): 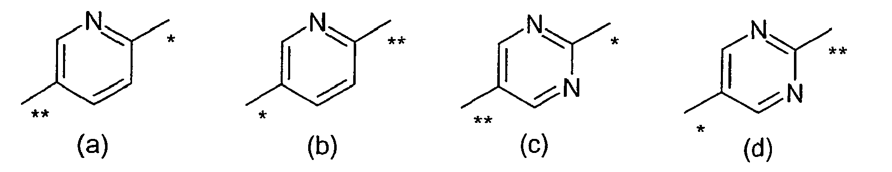 where * is the point of attachment to the X group of formula (I) and ** is the point of attachment to the Y group of formula (I). In a preferred aspect A is a group of formula (b) or (d).

In one aspect of the invention X is NR¹⁴, O or S. In another aspect X is NR¹⁴. In yet another aspect X is NH.

In one aspect of the invention m is 0, 2, 3 or 4. In another aspect m is 2.

In one aspect of the invention Y is O, NR⁵CO or CR⁶R⁷NR⁵. In another aspect Y is O, NHCO or CH₂NH. In a further aspect Y is NHCO

In one aspect of the invention Z is a group selected from -NR¹R², phosphonooxy, cyclopropyl which cyclopropyl is substituted by C₁₋₄alkyl substituted by phosphonooxy, and a piperidine or piperazine ring linked via carbon which ring is substituted on carbon or nitrogen by phosphonooxy or C₁₋₄alkyl substituted by phosphonooxy. In another aspect Z is a group selected from -NR¹R², phosphonooxy, 2-(phosphonooxymethyl)cyclopropyl and 1-(2-phosphonooxyethyl)piperidin-4-yl. In another aspect Z is -NR¹R₂.

In one aspect of the invention R¹ is C₁₋₅alkyl substituted by phosphonooxy. In another aspect R¹ is C₁₋₅alkyl substituted by phosphonooxy and further substituted by 1 or 2 halo. In a further aspect R¹ is 2-phosphonooxyethyl, 2-phosphonooxy-1,1-dimethylethyl, 2-phosphonooxy-2-methylethyl, 3-phosphonooxy-1,1-dimethylpropyl, 3-phosphonooxypropyl and 4-phosphonooxybutyl. In yet another aspect R¹ is 2-phosphonooxyethyl, 3-phosponooxy-1,1-dimethylpropyl or 4-phosphonooxybutyl. In a further aspect R¹ is 2-phosphonooxyethyl.

In one aspect of the invention R² is a group selected from hydrogen, C₁₋₆alkyl which C₁₋₆alkyl is optionally substituted by 1, 2 or 3 halo or C₁₋₄alkoxy groups, C₂₋₆alkenyl, C₂₋₆alkynyl, C₃₋₆cycloalkyl and C₃₋₆cycloalkylC₁₋₄alkyl. In another aspect R² is hydrogen, C₁₋₅alkyl, C₂₋₄alkynyl or C₃₋₆cycloalkyl. In another aspect R² is hydrogen, allyl, 2-propynyl, methyl, ethyl, propyl, isopropyl, 2-methylpropyl, butyl, 2,2-dimethylpropyl, cyclopropyl, cyclopropylmethyl, cyclobutyl, cyclobutylmethyl, cyclopentyl, cyclohexyl, cyclopentylmethyl, 3,3,3-trifluoropropyl and 2-methoxyethyl. In yet another aspect R² is hydrogen, methyl, ethyl, isopropyl, 2-methylpropyl, 2,2-dimethylpropyl, cyclopropyl, cyclobutyl, cyclohexyl or prop-2-ynyl. In a further aspect R² is hydrogen, methyl, ethyl isopropyl or cyclohexyl.

In one aspect of the invention R¹ and R² together with the nitrogen to which they are attached form a saturated 5- to 6-membered ring optionally containing a further nitrogen atom wherein the ring is substituted on carbon or nitrogen by a group selected from phosphonooxy and C₁₋₄alkyl (substituted by phosphonooxy or -NR⁸R⁹) and where the ring is optionally further substituted on carbon or nitrogen by 1 or 2 C₁₋₄alkyl groups. In another aspect of the invention R¹ and R² together with the nitrogen to which they are attached form a piperidine, pyrrolidine or piperazine ring which is substituted on carbon or nitrogen by a group selected from phosphonooxy, phosphonooxymethyl and 2-phosphonooxyethyl and where the ring is optionally further substituted on carbon or nitrogen by 1 or 2 methyl. In a further aspect of the invention R¹ and R² together with the nitrogen to which they are attached form 4-(phosphonooxymethyl)piperidinyl, 2-(phosphonooxymethyl)piperidinyl, 2-(phosphonooxymethyl)pyrrolidinyl, 4-(2-phosphonooxyethyl)piperazinyl, 3-(phosphonooxy)pyrrolidinyl, 3-(phosphonooxy)piperidinyl, 4-(phosphonooxy)piperidinyl, 4-(2-phosphonooxyethyl)piperidinyl, 2-(2-phosphonooxyethyl)pyrrolidinyl or 2-(2-phosphonooxyethyl)piperidinyl. In yet another aspect R¹ and R² together with the nitrogen to which they are attached form 2-(phosphonooxymethyl)piperidinyl, 4-(phosphonooxy)piperidinyl, 4-(2-phosphonooxyethyl)piperidinyl, 2-(2-phosphonooxyethyl)piperidinyl, 2-(phosphonooxymethyl)pyrrolidinyl, 4-(phosphonooxymethyl)piperidinyl or 4-(2-phosphonooxyethyl)piperazinyl.

In one aspect of the invention R³ is C₁₋₄alkoxy or hydrogen. In another aspect R³ is methoxy or hydrogen. In another aspect R³ is methoxy. In another aspect R³ is hydrogen.

In one aspect R⁴ is phenyl or benzyl optionally substituted by 1 or 2 of fluoro or chloro. In another aspect R⁴ is 3-fluorophenyl, 3-chlorophenyl, 3-chlorobenzyl, 3,5-difluorophenyl, 3,4-difluorophenyl, 2-fluorophenyl, 2,3-difluorophenyl, 2,4-difluorophenyl, 2,5-difluorophenyl, 3-chloro-4-fluorophenyl and 3-chloro-4-fluorobenzyl. In a further aspect R⁴ is 3-fluorophenyl, 3-chlorophenyl, 3-chlorobenzyl, 3,4-difluorophenyl, 3-chloro-4-fluorophenyl and 3-chloro-4-fluorobenzyl. In yet a further aspect R⁴ is 3-fluorophenyl. In another aspect R⁴ is 3-chlorophenyl. In a further aspect R⁴ is 3-chlorobenzyl. In yet another aspect R⁴ is 3,4-difluorophenyl. In another aspect R⁴ is 3-chloro-4-fluorophenyl. In a further aspect R⁴ is 3-chloro-4-fluorobenzyl.

In one aspect R⁵ is hydrogen or methyl. In another aspect R⁵ is hydrogen.

In one aspect of the invention R⁶ is hydrogen, fluoro, chloro or methyl. In another aspect R⁶ is hydrogen.

In one aspect of the invention R⁷ is hydrogen, fluoro, chloro or methyl. In another aspect R⁷ is hydrogen.

In one aspect R⁸ is 2-phosphonooxyethyl.

In one aspect of the invention R⁹ is hydrogen, methyl or ethyl.

In one aspect of the invention R¹⁰ is hydrogen, methyl or ethyl.

In one aspect of the invention R¹¹ is hydrogen, methyl or ethyl.

In one aspect of the invention R¹² is hydrogen or methyl.

In one aspect of the invention R¹³ is hydrogen or methyl.

In one aspect of the invention R¹⁴ is hydrogen or methyl.

A preferred class of compounds is of formula (I) wherein:
A is a group of formula (a), (b), (c) or (d) as defmed above;
X is NH;
m is 0, 1, 2, 3 or 4;
Y is O, NR⁵CO or CR⁶R⁷NR⁵
Z is -NR¹R², phoshonooxy, cyclopropyl which cyclopropyl is substituted by C₁₋₄alkyl substituted by phosphonooxy, and a piperidine or piperazine ring linked via a carbon atom which ring is substituted on carbon or nitrogen by phosphonooxy or C₁₋₄alkyl substituted by phosphonooxy;
R¹ is C₁₋₅alkyl substituted by phosphonooxy;
R² is a group selected from hydrogen, C₁₋₆alkyl which C₁₋₆alkyl is optionally substituted by 1, 2 or 3 halo or C₁₋₄alkoxy groups, C₂₋₆alkenyl, C₂₋₆alkynyl, C₃₋₆cycloalkyl and C₃₋₆cycloalkylC₁₋₄alkyl;
R³ is C₁₋₄alkoxy or hydrogen;
R⁴ is phenyl or benzyl optionally substituted by 1 or 2 of fluoro or chloro;
R⁵ is hydrogen or methyl; and
R⁶ and R⁷ are independently hydrogen, fluoro, chloro or methyl;
or a pharmaceutically acceptable salt thereof.

Another preferred class of compounds is of formula (I) wherein:
A is a group of formula (b) or (d) as defined above;
X is NH;
m is 0, 1, 2, 3 or 4;
Y is O, NR⁵CO or CR⁶R⁷NR⁵
Z is -NR¹R², phoshonooxy, cyclopropyl which cyclopropyl is substituted by C₁₋₄alkyl substituted by phosphonooxy, and a piperidine or piperazine ring linked via a carbon atom which ring is substituted on carbon or nitrogen by phosphonooxy or C₁₋₄alkyl substituted by phosphonooxy;
R¹ is C₁₋₅alkyl substituted by phosphonooxy;
R² is hydrogen, C₁₋₅alkyl, C₂₋₄alkynyl or C₃₋₆cycloalkyl;
R³ is methoxy;
R⁴ is phenyl or benzyl optionally substituted by 1 or 2 of fluoro or chloro;
R⁵ is hydrogen or methyl; and
R⁶ and R⁷ are independently hydrogen, fluoro, chloro or methyl;
or a pharmaceutically acceptable salt thereof.

Another preferred class of compounds is of formula (I) wherein:
A is a group of formula (a), (b), (c) or (d) as defined above;
X is NH;
m is 0, 1, 2, 3 or 4;
Y is O, NR⁵CO or CR⁶R⁷NR⁵
Z is -NR¹R², phoshonooxy, cyclopropyl which cyclopropyl is substituted by C₁₋₄alkyl substituted by phosphonooxy, and a piperidine or piperazine ring which the ring is substituted by phosphonooxy or C₁₋₄alkyl substituted by phosphonooxy;
R¹ and R² together with the nitrogen to which they are attached form a piperidine, pyrrolidine or piperazine ring which ring is substituted on carbon or nitrogen by a group selected from phosphonooxy, phosphonooxymethyl and 2-phosphonooxyethyl and which ring is optionally further substituted on carbon or nitrogen by 1 or 2 methyl.
R³ is C₁₋₄alkoxy or hydrogen;
R⁴ is phenyl or benzyl optionally substituted by 1 or 2 of fluoro or chloro;
R⁵ is hydrogen or methyl; and
R⁶ and R⁷ are independently hydrogen, fluoro, chloro or methyl;
or a pharmaceutically acceptable salt thereof.

A further preferred class of compounds is of formula (I) wherein:
A is a group of formula (b) or (d) as defined above;
X is NH;
m is 0, 1, 2, 3 or 4;
Y is O, NR⁵CO or CR⁶R⁷NR⁵
Z is -NR¹R², phoshonooxy, cyclopropyl (substituted by C₁₋₄alkyl (substituted by phosphonooxy)) and piperidine or piperazine ring where the ring is substituted by phosphonooxy or C₁₋₄alkyl (substituted by phosphonooxy);
R¹ and R² together with the nitrogen to which they are attached form a piperidine, pyrrolidine or piperazine ring which is substituted on carbon or nitrogen by a group selected from phosphonooxy, phosphonooxymethyl and 2-phosphonooxyethyl and where the ring is optionally further substituted on carbon or nitrogen by 1 or 2 methyl.
R³ is methoxy;
R⁴ is phenyl or benzyl optionally substituted by 1 or 2 of fluoro or chloro;
R⁵ is hydrogen or methyl; and
R⁶ and R⁷ are independently hydrogen, fluoro, chloro or methyl;
or a pharmaceutically acceptable salt thereof.

In another aspect of the invention, a preferred compound of the invention is any compound selected from :
3-[(3-{[4-({6-[(3-chlorobenzyl)oxy]pyridin-3-yl}amino)-6-methoxyquinazolin-7-yl]oxy}propyl)amino]-3-methylbutyl dihydrogen phosphate;
3-[(3-{[4-({6-[(3-chlorobenzoyl)amino]pyridin-3-yl}amino)-6-methoxyquinazolin-7 yl]oxy}propyl)amino]-3-methylbutyl dihydrogen phosphate;
2-[(3-{[4-({6-[(3 chlorobenzoyl)amino]pyridin-3-yl}amino)-6-methoxyquinazolin-7-yl]oxy}propyl)(ethyl)amino]ethyl dihydrogen phosphate;
2-[1-(3-{[4-({6-[(3-chlorobenzoyl)amino]pyridin-3-yl}amino)-6-methoxyquinazolin-7 yl]oxy}propyl)piperidin-2-yl]ethyl dihydrogen phosphate;
[(2R)-1-(3-{[4-({6-[(3-chlorobenzoyl)amino]pyridin-3-yl}amino)-6-methoxyquinazolin-7-yl]oxy}propyl)pyrrolidin-2-yl]methyl dihydrogen phosphate;
2-[1-(3-{[4-({6-[(3-chlorobenzoyl)amino]pyridin-3-yl}amino)-6-methoxyquinazolin-7-yl]oxy}propyl)piperidin-4-yl]ethyl dihydrogen phosphate;
2-[ethyl(3-{[4-({6-[(3-fluorobenzoyl)amino]pyridin-3-yl}amino)-6-methoxyquinazolin-7-yl]oxy}propyl)amino]ethyl dihydrogen phosphate;
2-[(3-{[4-({6-[(3,4-difluorobenzoyl)amino]pyridin-3-yl}amino)-6-methoxyquinazolin-7-yl]oxy}propyl)(isopropyl)amino]ethyl dihydrogen phosphate;
(3-{[4-({6-[(3-chlorobenzoyl)amino]pyridin-3-yl}amino)-6-methoxyquinazolin-7-yl]oxy}propyl)piperidin-4-yl dihydrogen phosphate;
4-{[4-({6-[(3-chlorobenzoyl)amino]pyridin-3-yl}amino)-6-methoxyquinazolin-7-yl]oxy}butyl dihydrogen phosphate;
2-[(3-{[4-({6-[(3-chlorobenzoyl)amino]pyridin-3-yl}amino)-6-methoxyquinazolin-7-yl]oxy}propyl)(methyl)amino]ethyl dihydrogen phosphate;
[1-(3-{[4-({6-[(3-chlorobenzoyl)amino]pyridin-3-yl}amino)-6-methoxyquinazolin-7-yl]oxy}propyl)piperidin-2-yl]methyl dihydrogen phosphate;
2-[(5-{[4-({6-[(3-chlorobenzoyl)amino]pyridin-3-yl}amino)-6-methoxyquinazolin-7-yl]oxy}pentyl)(ethyl)amino]ethyl dihydrogen phosphate;
4-[(3-{[4-({6-[(3-chlorobenzoyl)amino]pyridin-3-yl}amino)-6-methoxyquinazolin-7-yl]oxy}propyl)(ethyl)amino]butyl dihydrogen phosphate;
2-[(3-{[4-({6-[(3-fluorobenzoyl)amino]pyridin-3-yl}amino)-6-methoxyquinazolin-7-yl]oxy}propyl)(methyl)amino]ethyl dihydrogen phosphate;
2-[(3-{[4-({6-[(3-chlorobenzoyl)amino]pyridin-3-yl}amino)-6-methoxyquinazolin-7-yl]oxy}propyl)(isobutyl)amino]ethyl dihydrogen phosphate;
2-[(3-{[4-({6-[(3-chlorobenzoyl)amino]pyridin-3-yl}amino)-6-methoxyquinazolin-7-yl]oxy}propyl)(cyclopropyl)amino]ethyl dihydrogen phosphate;
[1-(3-{[4-({6-[(3-chlorobenzoyl)amino]pyridin-3-yl}amino)-6-methoxyquinazolin-7-yl]oxy}propyl)piperidin-4-yl]methyl dihydrogen phosphate;
2-[4-(3-{[4-({6-[(3-chlorobenzoyl)amino]pyridin-3-yl}amino)-6-methoxyquinazolin-7-yl]oxy}propyl)piperazin-1-yl]ethyl dihydrogen phosphate;
[(2*S*)-1-(3-{[4-({6-[(3-chlorobenzoyl)amino]pyridin-3-yl}amino)-6-methoxyquinazolin-7-yl]oxy}propyl)pyrrolidin-2-yl]methyl dihydrogen phosphate;
2-[(3-{[4-({6-[(3-chlorobenzoyl)amino]pyridin-3-yl}amino)-6-methoxyquinazolin-7-yl]oxy}propyl)(cyclobutyl)amino]ethyl dihydrogen phosphate;
2-[(3-{[4-({6-[(3-chlorobenzoyl)amino]pyridin-3-yl}amino)-6-methoxyquinazolin-7-yl]oxy}propyl)(prop-2-yn-1-yl)amino]ethyl dihydrogen phosphate;
2-[(3-{[4-({2-[(3-chloro-4-fluorobenzoyl)amino]pyrimidin-5-yl}amino)-6-methoxyquinazolin-7-yl]oxy}propyl)(cyclohexyl)amino]ethyl dihydrogen phosphate;
2-[(3-{[4-({2-[(3-chloro-4-fluorobenzoyl)amino]pyrimidin-5-yl}amino)-6-methoxyquinazolin-7-yl]oxy}propyl)(ethyl)amino]ethyl dihydrogen phosphate;
3-{[4-({2-[(3-chlorobenzoyl)amino]pyrimidin-5-yl}amino)-6-methoxyquinazolin-7-yl]oxy}propyl dihydrogen phosphate;
1-[3-({4-[(2-{[(3-chloro-4-fluorophenyl)amino]methyl}pyrimidin-5-yl)amino]-6-methoxyquinazolin-7-yl} oxy)propyl]piperidin-4-yl dihydrogen phosphate;
3-[(3-{[4-({2-[(3-chloro-4-fluorobenzyl)oxy]pyrimidin-5-yl}amino)-6-methoxyquinazolin-7-yl]oxy}propyl)amino]-3-methylbutyl dihydrogen phosphate;
2-[(3-{[4-({2-[(3-chlorobenzoyl)amino]pyrimidin-5-yl}amino)-6-methoxyquinazolin-7-yl]oxy}propyl)(2,2-dimethylpropyl)amino]ethyl dihydrogen phosphate;
[2-({[4-({2-[(3-chloro-4-fluorobenzoyl)amino]pyrimidin-5-yl}amino)-6-methoxyquinazolin-7-yl]oxy}methyl)cyclopropyl]methyl dihydrogen phosphate; and
2-[4-({[4-({2-[(3-chloro-4-fluorobenzoyl)amino]pyrimidin-5-yl}amino)-6-methoxyqumazolin-7-yl]oxy}methyl)piperidin-1-yl]ethyl dihydrogen phosphate;
or a pharmaceutically acceptable salt thereof.

In another aspect, a more preferred compound is any one selected from:
3-[(3-{[4-({6-[(3-chlorobenzyl)oxy]pyridin-3-yl}amino)-6-methoxyquinazolin-7-yl]oxy}propyl)amino]-3-methylbutyl dihydrogen phosphate;
3-[(3-{[4-({6-[(3chlorobenzoyl)amino]pyridin-3-yl}amino)-6-methoxyquinazolin-7 yl]oxy}propyl)amino]-3-methylbutyl dihydrogen phosphate;
2-[(3-{[4-({6-[(3 chlorobenzoyl)amino]pyridin-3-yl}amino)-6-methoxyquinazolin-7-yl]oxy}propyl)(ethyl)amino]ethyl dihydrogen phosphate;
2-[ethyl(3-{[4-({6-[(3-fluorobenzoyl)amino]pyridin-3-yl}amino)-6-methoxyquinazolin-7-yl]oxy}propyl)amino]ethyl dihydrogen phosphate;
2-[(3-{[4-({6-[(3,4-difluorobenzoyl)amino]pyridin-3-yl}amino)-6-methoxyquinazolin-7-yl]oxy}propyl)(isopropyl)amino]ethyl dihydrogen phosphate;
2-[(3-{[4-({6-[(3-chlorobenzoyl)amino]pyridin-3-yl}amino)-6-methoxyquinazolin-7-yl]oxy}propyl)(methyl)amino]ethyl dihydrogen phosphate;
2-[(5-{[4-({6-[(3-chlorobenzoyl)amino]pyridin-3-yl}amino)-6-methoxyquinazolin-7-yl]oxy}pentyl)(ethyl)amino] ethyl dihydrogen phosphate;
4-[(3-{[4-({6-[(3-chlorobenzoyl)amino]pyridin-3-yl}amino)-6-methoxyquinazolin-7-yl]oxy}propyl)(ethyl)amino]butyl dihydrogen phosphate;
2-[(3-{[4-({6-[(3-fluorobenzoyl)amino]pyridin-3-yl}amino)-6-methoxyquinazolin-7-yl]oxy} propyl)(methyl)amino]ethyl dihydrogen phosphate;
2-[(3-{[4-({6-[(3-chlorobenzoyl)amino]pyridin-3-yl}amino)-6-methoxyquinazolin-7-yl]oxy}propyl)(isobutyl)amino]ethyl dihydrogen phosphate;
2-[(3-{[4-({6-[(3-chlorobenzoyl)amino]pyridin-3-yl}amino)-6-methoxyquinazolin-7-yl]oxy}propyl)(cyclopropyl)amino]ethyl dihydrogen phosphate;
2-[(3-{[4-({6-[(3-chlorobenzoyl)amino]pyridin-3-yl}amino)-6-methoxyquinazolin-7-yl]oxy}propyl)(cyclobutyl)amino]ethyl dihydrogen phosphate;
2-[(3-{[4-({6-[(3-chlorobenzoyl)amino]pyridin-3-yl}amino)-6-methoxyquinazolin-7-yl]oxy}propyl)(prop-2-yn-1-yl)amino]ethyl dihydrogen phosphate;
2-[(3-{[4-({2-[(3-chloro-4-fluorobenzoyl)amino]pyrimidin-5-yl}amino)-6-methoxyquinazolin-7-yl]oxy}propyl)(cyclohexyl)amino]ethyl dihydrogen phosphate;
2-[(3-{[4-({2-[(3-chloro-4-fluorobenzoyl)amino]pyrimidin-5-yl}amino)-6-methoxyquinazolin-7-yl]oxy}propyl)(ethyl)amino]ethyl dihydrogen phosphate;
3-[(3-{[4-({2-[(3-chloro-4-fluorobenzyl)oxy]pyrimidin-5-yl}amino)-6-methoxyquinazolin-7-yl]oxy}propyl)amino]-3-methylbutyl dihydrogen phosphate; and
2-[(3-{[4-({2-[(3-chlorobenzoyl)amino]pyrimidin-5-yl} amino)-6-methoxyquinazolin-7-yl]oxy}propyl)(2,2-dimethylpropyl)amino]ethyl dihydrogen phosphate;
or a pharmaceutically acceptable salt thereof.

In a further aspect, a more preferred compound is any one selected from:
2-[1-(3-{[4-({6-[(3-chlorobenzoyl)amino]pyridin-3-yl}amino)-6-methoxyquinazolin-7 yl]oxy}propyl)piperidin-2-yl]ethyl dihydrogen phosphate;
[(2R)-1-(3-{[4-({6-[(3-chlorobenzoyl)amino]pyridin-3-yl}amino)-6-methoxyquinazolin-7-yl]oxy}propyl)pyrrolidin-2-yl]methyl dihydrogen phosphate;
2-[1-(3-{[4-({6-[(3-chlorobenzoyl)amino]pyridin-3-yl}amino)-6-methoxyquinazolin-7-yl]oxy}propyl)piperidin-4-yl]ethyl dihydrogen phosphate;
(3-{[4-({6-[(3-chlorobenzoyl)amino]pyridin-3-yl}amino)-6-methoxyquinazolin-7-yl]oxy}propyl)piperidin-4-yl dihydrogen phosphate;
[1-(3-{[4-({6-[(3-chlorobenzoyl)amino]pyridin-3-yl}amino)-6-methoxyquinazolin-7-yl]oxy}propyl)piperidin-2-yl]methyl dihydrogen phosphate;
[1-(3-{[4-({6-[(3-chlorobenzoyl)amino]pyridin-3-yl}amino)-6-methoxyquinazolin-7-yl]oxy}propyl)piperidin-4-yl]methyl dihydrogen phosphate;
2-[4-(3-{[4-({6-[(3-chlorobenzoyl)amino]pyridin-3-yl}amino)-6-methoxyquinazolin-7-yl]oxy}propyl)piperazin-1-yl]ethyl dihydrogen phosphate;
[(2*S*)-1-(3-{[4-({6-[(3-chlorobenzoyl)amino]pyridin-3-yl}amino)-6-methoxyquinazolin-7-yl]oxy}propyl)pyrrolidin-2-yl]methyl dihydrogen phosphate; and
1-[3-({4-[(2-{[(3-chloro-4-fluorophenyl)amino]methyl}pyrimidin-5-yl)amino]-6-methoxyquinazolin-7-yl}oxy)propyl]piperidin-4-yl dihydrogen phosphate;
or a pharmaceutically acceptable salt thereof.

Another more preferred compound is any one selected from:
4-{[4-({6-[(3-chlorobenzoyl)amino]pyridin-3-yl}amino)-6-methoxyquinazolin-7-yl]oxy}butyl dihydrogen phosphate;
3-{[4-({2-[(3-chlorobenzoyl)amino]pyrimidin-5-yl}amino)-6-methoxyquinazolin-7-yl]oxy}propyl dihydrogen phosphate;
[2-({[4-({2-[(3-chloro-4-fluorobenzoyl)amino]pyrimidin-5-yl}amino)-6-methoxyquinazolin-7-yl]oxy}methyl)cyclopropyl]methyl dihydrogen phosphate; and
2-[4-({[4-({2-[(3-chloro-4-fluorobenzoyl)amino]pyrimidin-5-yl}amino)-6-methoxyquinazolin-7-yl]oxy}methyl)piperidin-1-yl]ethyl dihydrogen phosphate;
or a pharmaceutically acceptable salt thereof

A particularly preferred compound is any one selected from:
2-[(3-{[4-({6-[(3 chlorobenzoyl)amino]pyridin-3-yl}amino)-6-methoxyquinazolin-7-yl]oxy}propyl)(ethyl)amino]ethyl dihydrogen phosphate;
2-[(3-{[4-({6-[(3-chlorobenzoyl)amino]pyridin-3-yl}amino)-6-methoxyquinazolin-7-yl]oxy}propyl)(isobutyl)amino]ethyl dihydrogen phosphate
2-[(3-{[4-({6-[(3-chlorobenzoyl)amino]pyridin-3-yl}amino)-6-methoxyquinazolin-7-yl]oxy}propyl)(cyclopropyl)amino]ethyl dihydrogen phosphate
2-[(3-{[4-({6-[(3-chlorobenzoyl)amino]pyridin-3-yl}amino)-6-methoxyquinazolin-7-yl]oxy}propyl)(cyclobutyl)amino]ethyl dihydrogen phosphate
2-[(3-{[4-({6-[(3-chlorobenzoyl)amino]pyridin-3-yl}amino)-6-methoxyquinazolin-7-yl]oxy}propyl)(prop-2-yn-1-yl)amino]ethyl dihydrogen phosphate
2-[(3-{[4-({2-[(3-chlorobenzoyl)amino]pyrimidin-5-yl}amino)-6-methoxyquinazolin-7-yl]oxy}propyl)(2,2-dimethylpropyl)amino]ethyl dihydrogen phosphate;
or a pharmaceutically acceptable salt thereof.

In another aspect the present invention provides a process for the preparation of a compound of formula (I) or a pharmaceutically acceptable salt thereof, which process comprises converting a compound of formula (II) into a compound of formula (I) by phosphorylation of an appropriate hydroxy group: 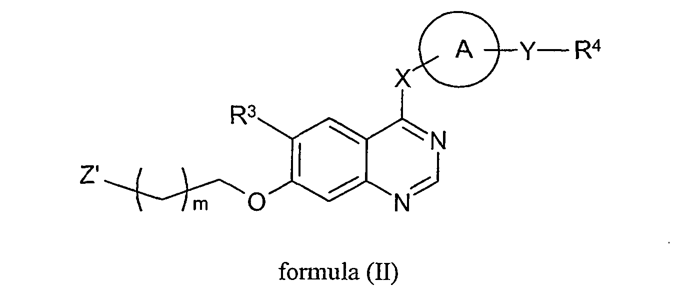 where A, X, m, Y, R³ and R⁴ are as defined for formula (I); and **Z'** is a group selected from - NR^{1'}R^{2'}, hydroxy, C₃₋₆cycloalkyl which C₃₋₆cycloalkyl is substituted by hydroxy or C₁₋₄alkyl substituted by hydroxy, and a 4- to 7-membered ring linked via a carbon atom, containing a nitrogen atom and optionally containing a further nitrogen atom, which ring may be saturated, unsaturated or partially saturated and which ring is substituted on carbon or nitrogen by hydroxy or C₁₋₄alkyl substituted by hydroxy and which ring is optionally further substituted on carbon or nitrogen by 1, 2 or 3 halo or C₁₋₄alkyl groups; **R^{1'}** is a group selected from-COR^{8'}, -CONR^{8'}R⁹ and C₁₋₆alkyl which C₁₋₆alkyl is substituted by hydroxy and optionally further substituted by 1 or 2 halo or methoxy groups; **R^{2'}** is a group selected from hydrogen, - COR¹⁰, -CONR¹⁰R¹¹ and C₁₋₆alkyl which C₁₋₆alkyl is optionally substituted by 1, 2 or 3 halo or C₁₋₄alkoxy groups, -S(O)ₚR¹¹ (where p is 0, 1 or 2) or hydroxy, or R^{2'} is a group selected from C₂₋₆alkenyl, C₂₋₆alkynyl, C₃₋₆cycloalkyl and C₃₋₆cycloalkylC₁₋₄alkyl; or **R^{1'}** and **R^{2'}** together with the nitrogen to which they are attached form a 4- to 7- membered ring optionally containing a further nitrogen atom which ring may be saturated, unsaturated or partially saturated and which ring is substituted on carbon or nitrogen by a group selected from hydroxy and C₁₋₄alkyl which C₁₋₄alkyl is substituted by hydroxy or -NR^{8'}R⁹ and which ring is optionally further substituted on carbon or nitrogen by 1, 2 or 3 halo or C₁₋₄alkyl groups; and where **R^{8'}** is C₁₋₄alkyl substituted by hydroxy and optionally further substituted by 1 or 2 halo or methoxy groups:
and thereafter if necessary:
   i) converting a compound of the formula (I) into another compound of the formula (I); and/or
   ii) removing any protecting groups; and/or
   iii) forming a pharmaceutically acceptable salt thereof.

Phosphorylation may be suitably performed by treatment with 1-H tetrazole (or a suitable replacement such as S-ethyl tetrazole or pyridinium hydrochloride) and di-*tert-*butyldiethylphosphoramidite or dibenzyldiethylphosphoramidite at 5 to 35 °C under an inert atmosphere for 30 minutes to 4 hours followed by treatment with an oxidizing agent such as meta-chloroperbenzoic acid (mCPBA) or 30% aqueous hydrogen peroxide at -10 to 25 °C for 2 to 18 hour. Deprotection of the *tert*-butyl groups to yield the phosphate group is required as a final step with these reagents and may be readily achieved by treatment with 4.0 N hydrochloric acid in 1,4-dioxane at 10 to 35 °C for 12 to 18 hours.

This process may further comprise a method for the preparation of a compound of formula (II) where Z' is -NR^{1'}R^{2'} which method comprises the reaction of a compound of formula (III) where L is a leaving group such as halo (e.g.chloro): 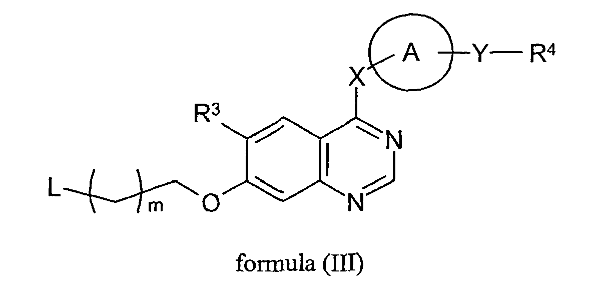 with an amine of formula (IV): 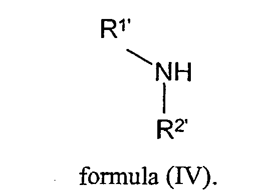 Suitable reaction conditions for this method include heating a compound of formula (III) with an excess of amine of formula (IV) in an inert solvent such as dimethylacetamide, with or without the addition of a suitable catalyst (such as *tetra*-n-butylammoniuim iodide or potassium iodide) at a temperature of 50 to 100 °C for 12 to 72 hours.

The amines of formula (IV) are known in the art or may be prepared by the skilled person using methods known in the art.

The process may further comprise a method for the preparation of a compound of formula (III) which method comprises the reaction of a compound of formula (V) where P is a suitable protecting group for a hydroxy group (such as benzyl): 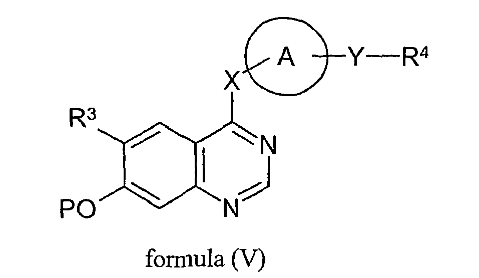 by reaction with a compound of formula (VI) where L' is a leaving group such as halo (e.g. bromo): 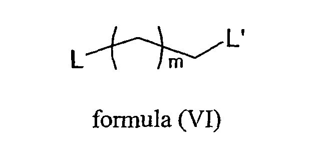 Such a reaction can be achieved (after removal of the protecting group using a method selected from those already described in the literature) under a range of conditions described in the literature such as heating a compound of formula (V) with a compound of formula (VI) in the presence of a catalyst such as caesium carbonate in a solvent such as acetonitrile at a temperature of 80 to 100 °C for 1 to 4 hours.

The process may further comprise a method for the preparation of a compound of formula (V) where X is NR¹⁴, O or S which method comprises the reaction of a compound of formula (VII) where L is a leaving group such as halo (e.g. chloro) and where P is a protecting group for a hydroxy group (such as benzyl): 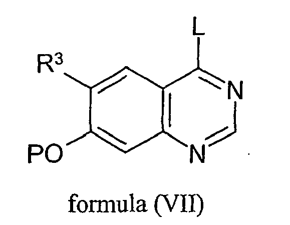 with a compound of formula (VIII) 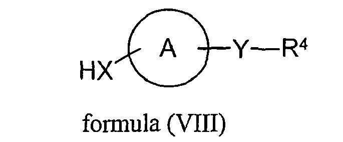 Such a reaction can be achieved under a range of conditions described in the literature such as heating a compound of formula (VII) with a compound of formula (VIII) in a solvent such as isopropanol or dimethylacetamide (optionally in the presence of an acid catalyst such as hydrochloric acid) at a temperature of 80 to 100 °C for 2 to 6 hours. Alternatively the reaction may be effected using a base such as sodium hydride and carrying out the reaction in an inert solvent such as dimethylformamide at a temperature of 50 to 80 °C for 2 to 6 hours.

The process may further comprise a method for the preparation of a compound of formula (VII) which method comprises the reaction of a compound of formula (IX) where P is a protecting group for a hydroxy group (such as benzyl) with a chlorinating agent such as thionyl chloride, phosphorus oxychloride or phosphorus pentachloride. 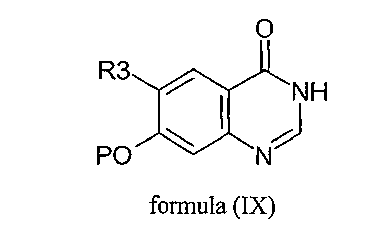 Suitable reaction conditions are illustrated herein.

A method for the preparation of a compound of formula (IX) comprises the reaction of a compound of formula (X) where R' may be either hydrogen or an alkyl, aryl or benzyl group and where P is a protecting group: 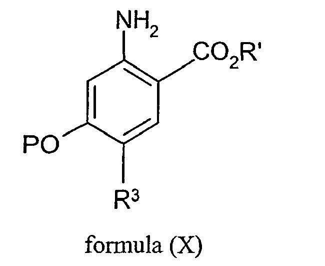 with formamide or a suitable equivalent (such as formamidine acetate). The reaction is suitably effected either by heating a compound of formula (X) in neat formamide or by heating in a suitable solvent such as 2-methoxyethanol at elevated temperature, conveniently at the reflux temperature of the solvent.

Compounds of formula (X) are either known compounds or they can be prepared by the skilled person using conventional methods. In particular, compounds of formula (X) may be prepared by reduction of the corresponding nitro compound of formula (XI) where R' may be either hydrogen or an alkyl, aryl or benzyl group: 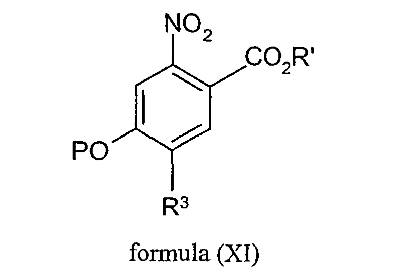 Suitable reaction conditions are illustrated herein.

Compounds of formula (XI) may be obtained by nitration of a compound of formula (XII)) where R' may be either hydrogen or an alkyl, aryl or benzyl group: 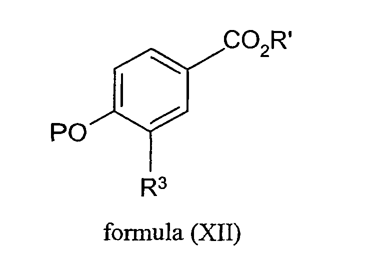 for example, using nitric acid as the nitrating agent. Again, suitable reaction conditions are illustrated herein.

The compounds of formula (VIII) are known in the art or may be prepared by the skilled person using methods known in the art. However the process may further comprise a method for the preparation of a compound according to formula (VIII) where Y is NR⁵CO and X is NH which method comprises the reduction of a compound of formula (XIII) 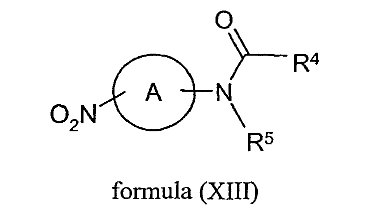 for example, using hydrogen (in the presence of a platinum or palladium catalyst) as a reducing agent. Again, suitable reaction conditions are illustrated herein.

The process may further comprise a method for the preparation of a compound according to formula (XIII) which method comprises the reaction of a compound of formula (XIV) 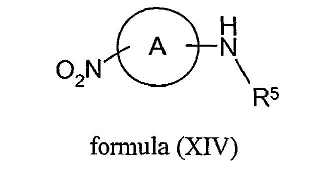 with a compound of formula (XV) where L is an appropriate leaving group (such as carboxylate, (C₁₋₁₀alkyl)COO or halo, such as chloro): 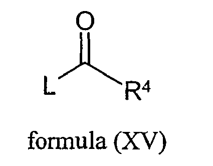 Again, suitable reaction conditions are illustrated herein.

It will be appreciated that certain of the various ring substituents in the compounds of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogen group. Particular examples of modifications include the reduction of a nitro group to an amino group by for example, catalytic hydrogenation with a nickel catalyst or treatment with iron in the presence of hydrochloric acid with heating; oxidation of alkylthio to alkylsulphinyl or alkylsulphonyl.

It will also be appreciated that in some of the reactions mentioned herein it may be necessary/desirable to protect any sensitive groups in the compounds. The instances where protection is necessary or desirable and suitable methods for protection are known to those skilled in the art. Conventional protecting groups may be used in accordance with standard practice (for illustration see T.W. Green, Protective Groups in Organic Synthesis, John Wiley and Sons, 1991). Thus, if reactants include groups such as amino, carboxy or hydroxy it may be desirable to protect the group in some of the reactions mentioned herein.

A suitable protecting group for an amino or alkylamino group is, for example, an acyl group, for example an alkanoyl group such as acetyl, an alkoxycarbonyl group, for example a methoxycarbonyl, ethoxycarbonyl or *tert*-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed for example, by hydrolysis with a suitable base such as an alkali metal hydroxide, for example lithium or sodium hydroxide. Alternatively an acyl group such as a *tert*-butoxycarbonyl group may be removed, for example, by treatment with a suitable acid as hydrochloric, sulphuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively an arylmethyl group such as a benzyl group may be removed, for example, by hydrogenation over a catalyst such as palladium-on-carbon.

A suitable protecting group for a carboxy group is, for example, an esterifying group, for example a methyl or an ethyl group which may be removed, for example, by hydrolysis with a base such as sodium hydroxide, or for example a *tert*-butyl group which may be removed, for example, by treatment with an acid, for example an organic acid such as trifluoroacetic acid, or for example a benzyl group which may be removed, for example, by hydrogenation over a catalyst such as palladium-on-carbon.

The protecting groups may be removed at any convenient stage in the synthesis using conventional techniques well known in the chemical art.

According to a further aspect of the invention there is provided a pharmaceutical composition which comprises a compound formula (I), or a pharmaceutically acceptable salt thereof, as defined herein in association with a pharmaceutically acceptable diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

Suitable pharmaceutically acceptable excipients for a tablet formulation include, for example, inert diluents such as lactose, sodium carbonate, calcium phosphate or calcium carbonate, granulating and disintegrating agents such as corn starch or algenic acid; binding agents such as starch; lubricating agents such as magnesium stearate, stearic acid or talc; preservative agents such as ethyl or propyl p-hydroxybenzoate, and anti-oxidants, such as ascorbic acid. Tablet formulations may be uncoated or coated either to modify their disintegration and the subsequent absorption of the active ingredient within the gastrointestinal track, or to improve their stability and/or appearance, in either case, using conventional coating agents and procedures well known in the art.

Compositions for oral use may be in the form of hard gelatin capsules in which the active ingredient is mixed with an inert solid diluent, for example, calcium carbonate, calcium phosphate or kaolin, or as soft gelatin capsules in which the active ingredient is mixed with water or an oil such as peanut oil, liquid paraffm, soya bean oil, coconut oil, or preferably olive oil, or any other acceptable vehicle

Aqueous suspensions generally contain the active ingredient in finely powdered form together with one or more suspending agents, such as sodium carboxymethylcellulose, methylcellulose, hydroxypropylmethylcellulose, sodium alginate, polyvinyl-pyrrolidone, gum tragacanth and gum acacia; dispersing or wetting agents such as lecithin or condensation products of an alkylene oxide with fatty acids (for example polyoxyethylene stearate), or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives (such as ethyl or propyl p-hydroxybenzoate, anti-oxidants (such as ascorbic acid), colouring agents, flavouring agents, and/or sweetening agents (such as sucrose, saccharine or aspartame).

Oily suspensions may be formulated by suspending the active ingredient in a vegetable oil (such as arachis oil, olive oil, sesame oil or coconut oil) or in a mineral oil (such as liquid paraffm). The oily suspensions may also contain a thickening agent such as beeswax, hard paraffin or cetyl alcohol. Sweetening agents such as those set out above, and flavouring agents may be added to provide a palatable oral preparation. These compositions may be preserved by the addition of an anti-oxidant such as ascorbic acid.

Dispersible or lyophilised powders and granules suitable for preparation of an aqueous suspension or solution by the addition of water generally contain the active ingredient together with a dispersing or wetting agent, suspending agent and one or more preservatives. Suitable dispersing or wetting agents and suspending agents are exemplified by those already mentioned above. Additional excipients such as sweetening, flavouring and colouring agents, may also be present.

The pharmaceutical compositions of the invention may also be in the form of oil-in-water emulsions. The oily phase may be a vegetable oil, such as olive oil or arachis oil, or a mineral oil, such as for example liquid paraffin or a mixture of any of these. Suitable emulsifying agents may be, for example, naturally-occurring gums such as gum acacia or gum tragacanth, naturally-occurring phosphatides such as soya bean, lecithin, an esters or partial esters derived from fatty acids and hexitol anhydrides (for example sorbitan monooleate) and condensation products of the said partial esters with ethylene oxide such as polyoxyethylene sorbitan monooleate. The emulsions may also contain sweetening, flavouring and preservative agents.

Syrups and elixirs may be formulated with sweetening agents such as glycerol, propylene glycol, sorbitol, aspartame or sucrose, and may also contain a demulcent, preservative, flavouring and/or colouring agent.

The pharmaceutical compositions may also be in the form of a sterile injectable aqueous or oily suspension, solutions, emulsions or particular systems, which may be formulated according to known procedures using one or more of the appropriate dispersing or wetting agents and suspending agents, which have been mentioned above. A sterile injectable preparation may also be a sterile injectable solution or suspension in a non-toxic parenterally-acceptable diluent or solvent, for example a solution in polyethylene glycol.

Suppository formulations may be prepared by mixing the active ingredient with a suitable non-irritating excipient which is solid at ordinary temperatures but liquid at the rectal temperature and will therefore melt in the rectum to release the drug. Suitable excipients include, for example, cocoa butter and polyethylene glycols.

Topical formulations, such as creams, ointments, gels and aqueous or oily solutions or suspensions, may generally be obtained by formulating an active ingredient with a conventional, topically acceptable, vehicle or diluent using conventional procedure well known in the art.

Compositions for administration by insufflation may be in the form of a fmely divided powder containing particles of average diameter of, for example, 30µm or much less preferably 5µm or less and more preferably between 5µm and 1µm, the powder itself comprising either active ingredient alone or diluted with one or more physiologically acceptable carriers such as lactose. The powder for insufflation is then conveniently retained in a capsule containing, for example, 1 to 50mg of active ingredient for use with a turbo-inhaler device, such as is used for insufflation of the known agent sodium cromoglycate.

Compositions for administration by inhalation may be in the form of a conventional pressurised aerosol arranged to dispense the active ingredient either as an aerosol containing finely divided solid or liquid droplets. Conventional aerosol propellants such as volatile fluorinated hydrocarbons or hydrocarbons may be used and the aerosol device is conveniently arranged to dispense a metered quantity of active ingredient.

For further information on formulation the reader is referred to Chapter 25.2 in Volume 5 of Comprehensive Medicinal Chemistry (Corwin Hansch; Chairman of Editorial Board), Pergamon Press 1990.

Therefore in a further aspect of the invention there is provided a compound of formula (I), or a pharmaceutically acceptable salt thereof, for use in therapy. Further provided is a compound of formula (I), or a pharmaceutically acceptable salt thereof, for use as a medicament. A compound of formula (I), or a pharmaceutically acceptable salt thereof, is also provided for use in the treatment of a disease where the inhibition of one or more Aurora kinase is beneficial. In particular it is envisaged that inhibition of Aurora-A kinase and/or Aurora-B kinase may be beneficial. Preferably inhibition of Aurora-B kinase is beneficial. A compound of formula (I), or a pharmaceutically acceptable salt thereof, has further use in the treatment of hyperproliferative diseases such as cancer and in particular colorectal, breast, lung, prostate, pancreatic or bladder and renal cancer or leukemias or lymphomas.

Additionally a compound of formula (I), or a pharmaceutically acceptable salt thereof is provided for use in a method of treatment of a wam-blooded animal such as man by therapy. According to this aspect, there is provided a compound of formula (I) or a pharmaceutically acceptable salt thereof for use in the method of treating a human suffering from a disease in which the inhibition of one or more Aurora kinases is beneficial, comprising the steps of administering to a person in need thereof a therapeutically effective amount of a compound of formula (I) or a pharmaceutically acceptable salt thereof In particular it is envisaged that inhibition of Aurora-A kinase and/or Aurora-B kinase may be beneficial. Preferably inhibition of Aurora-B kinase is beneficial. Further provided is a compound of formula (I) or a pharmaceutically acceptable salt thereof for use in the method of treating a human suffering from a hyperproliferative disease such as cancer and in particular particular colorectal, breast, lung, prostate, pancreatic or bladder and renal cancer or leukemias or lymphomas, comprising the steps of administering to a person in need thereof a therapeutically effective amount of a compound of formula (I) or a pharmaceutically acceptable salt thereof.

In another aspect of the invention, there is provided the use of a compound of formula (I) or a pharmaceutically acceptable salt thereof, in the preparation of a medicament for the treatment of a disease where the inhibition of one or more Aurora kinase is beneficial. In particular it is envisaged that inhibition of Aurora-A kinase and/or Aurora-B kinase may be beneficial. Preferably inhibition of Aurora-B kinase is beneficial. In another aspect of the invention, there is provided the use of a compound of formula (I) or a pharmaceutically acceptable salt thereof, in the preparation of a medicament for the treatment of hyperproliferative diseases such as cancer and in particular colorectal, breast, lung, prostate, pancreatic or bladder and renal cancer or leukemias or lymphomas.

For the above mentioned therapeutic uses the dose administered will vary with the compound employed, the mode of administration, the treatment desired, the disorder indicated and the age and sex of the animal or patient. The size of the dose would thus be calculated according to well known principles of medicine.

In using a compound of formula (I) for therapeutic or prophylactic purposes it will generally be administered so that a daily dose in the range, for example, 0.05 mg/kg to 50 mg/kg body weight is received, given if required in divided doses. In general lower doses will be administered when a parenteral route is employed. Thus, for example, for intravenous administration, a dose in the range, for example, 0.05 mg/kg to 25 mg/kg body weight will generally be used. Similarly, for administration by inhalation, a dose in the range, for example, 0.05 mg/kg to 25 mg/kg body weight will be used.

The treatment defined herein may be applied as a sole therapy or may involve, in addition to the compound of the invention, conventional surgery or radiotherapy or chemotherapy. Such chemotherapy may include one or more of the following categories of anti-tumour agents :-
(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside and hydroxyurea; antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin);
(ii) cytostatic agents such as antioestrogens (for example tamoxifen, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) Agents which inhibit cancer cell invasion (for example metalloproteinase inhibitors like marimastat and inhibitors of urokinase plasminogen activator receptor function);
(iv) inhibitors of growth factor function, for example such inhibitors include growth factor antibodies, growth factor receptor antibodies (for example the anti-erbb2 antibody trastuzumab [Herceptin^{™}] and the anti-erbb1 antibody cetuximab [C225]), farnesyl transferase inhibitors, tyrosine kinase inhibitors and serine-threonine kinase inhibitors, for example inhibitors of the epidermal growth factor family (for example EGFR family tyrosine kinase inhibitors such as *N*-(3-chloro-4-fluorophenyl)-7-methoxy-6-(3-morpholinopropoxy)quinazolin-4-amine (gefitinib, AZD1839), *N*-(3-ethynylphenyl)-6,7-bis(2-methoxyethoxy)quinazolin-4-amine (erlotinib, OSI-774) and 6-acrylamido-*N*-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti-vascular endothelial cell growth factor antibody bevacizumab [Avastin™], compounds such as those disclosed in International Patent Applications WO 97/22596, WO 97/30035, WO 97/32856 and WO 98/13354) and compounds that work by other mechanisms (for example linomide, inhibitors of integrin αvβ3 function and angiostatin);
(vi) vascular damaging agents such as Combretastatin A4 and compounds disclosed in International Patent Applications WO 99/02166, WO00/40529, WO 00/41669, WO01/92224, WO02/04434 and WO02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example *ex vivo* and *in vivo* approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.
In addition a compound of the invention may be used in combination with one or more cell cycle inhibitors. In particular with cell cycle inhibitors which inhibit bub1, bubR1 or CDK. Such conjoint treatment may be achieved by way of the simultaneous, sequential or separate dosing of the individual components of the treatment. Such combination products employ the compounds of this invention within the dosage range described herein and the other pharmaceutically-active agent within its approved dosage range.

In addition to their use in therapeutic medicine, the compounds of formula (I) and their pharmaceutically acceptable salts are also useful as pharmacological tools in the development and standardisation of *in vitro* and *in vivo* test systems for the evaluation of the effects of inhibitors of cell cycle activity in laboratory animals such as cats, dogs, rabbits, monkeys, rats and mice, as part of the search for new therapeutic agents.

In the above other pharmaceutical composition, process, method, use and medicament manufacture features, the alternative and preferred embodiments of the compounds of the invention described herein also apply.

The compounds of the invention inhibit the serine-threonine kinase activity of the Aurora kinases, in particular Aurora-A and/or Aurora-B and thus inhibit the cell cycle and cell proliferation. These properties may be assessed for example, using one or more of the procedures set out below. Whilst not wishing to be bound by theoretical constraints, it is believed that the compounds of formula (I) described herein may act as prodrugs. In procedures (c) and (d) set out below it is believed that a phosphonooxy group present in the compound of formula (I) is cleaved *in situ* to yield a hydroxy group and that such cleavage is necessary for activity is these assays.

### (a) In Vitro Aurora-A kinase inhibition test

This assay determines the ability of a test compound to inhibit serine-threonine kinase activity. DNA encoding Aurora-A may be obtained by total gene synthesis or by cloning. This DNA may then be expressed in a suitable expression system to obtain polypeptide with serine-threonine kinase activity. In the case of Aurora-A, the coding sequence was isolated from cDNA by polymerase chain reaction (PCR) and cloned into the BamH1 and Not1 restriction endonuclease sites of the baculovirus expression vector pFastBac HTc (GibcoBRL/Life technologies). The 5' PCR primer contained a recognition sequence for the restriction endonuclease BamH1 5' to the Aurora-A coding sequence. This allowed the insertion of the Aurora-A gene in frame with the 6 histidine residues, spacer region and rTEV protease cleavage site encoded by the pFastBac HTc vector. The 3' PCR primer replaced the Aurora-A stop codon with additional coding sequence followed by a stop codon and a recognition sequence for the restriction endonuclease Not1. This additional coding sequence (5' TAC CCA TAC GAT GTT CCA GAT TAC GCT TCT TAA 3') encoded for the polypeptide sequence YPYDVPDYAS. This sequence, derived from the influenza hemagglutin protein, is frequently used as a tag epitope sequence that can be identified using specific monoclonal antibodies. The recombinant pFastBac vector therefore encoded for an N-terminally 6 his tagged, C terminally influenza hemagglutin epitope tagged Aurora-A protein. Details of the methods for the assembly of recombinant DNA molecules can be found in standard texts, for example Sambrook et al. 1989, Molecular Cloning - A Laboratory Manual, 2nd Edition, Cold Spring Harbor Laboratory press and Ausubel et al. 1999, Current Protocols in Molecular Biology, John Wiley and Sons Inc.

Production of recombinant virus can be performed following manufacturer's protocol from GibcoBRL. Briefly, the pFastBac-1 vector carrying the Aurora-A gene was transformed into E. coli DH10Bac cells containing the baculovirus genome (bacmid DNA) and via a transposition event in the cells, a region of the pFastBac vector containing gentamycin resistance gene and the Aurora-A gene including the baculovirus polyhedrin promoter was transposed directly into the bacmid DNA. By selection on gentamycin, kanamycin, tetracycline and X-gal, resultant white colonies should contain recombinant bacmid DNA encoding Aurora-A. Bacmid DNA was extracted from a small scale culture of several BH10Bac white colonies and transfected into Spodoptera frugiperda Sf21 cells grown in TC100 medium (GibcoBRL) containing 10% serum using CellFECTIN reagent (GibcoBRL) following manufacturer's instructions. Virus particles were harvested by collecting cell culture medium 72 hrs post transfection. 0.5 mls of medium was used to infect 100 ml suspension culture of Sf21s containing 1 x 10⁷ cells/ml. Cell culture medium was harvested 48 hrs post infection and virus titre determined using a standard plaque assay procedure. Virus stocks were used to infect Sf9 and "High 5" cells at a multiplicity of infection (MOI) of 3 to ascertain expression of recombinant Aurora-A protein.

For the large scale expression of Aurora-A kinase activity, Sf21 insect cells were grown at 28°C in TC100 medium supplemented with 10% foetal calf serum (Viralex) and 0.2% F68 Pluronic (Sigma) on a Wheaton roller rig at 3 r.p.m. When the cell density reached 1.2x10⁶ cells ml⁻¹ they were infected with plaque-pure Aurora-A recombinant virus at a multiplicity of infection of 1 and harvested 48 hours later. All subsequent purification steps were performed at 4°C. Frozen insect cell pellets containing a total of 2.0 x 10⁸ cells were thawed and diluted with lysis buffer (25 mM HEPES (N-[2-hydroxyethyl]piperazine-N'-[2-ethanesulphonic acid]) pH7.4 at 4°C, 100 mM KCl, 25 mM NaF, 1 mM Na₃VO₄, 1 mM PMSF (phenylmethylsulphonyl fluoride), 2 mM 2-mercaptoethanol, 2 mM imidazole, 1 µg/ml aprotinin, 1 µg/ml pepstatin, 1 µg/ml leupeptin), using 1.0 ml per 3 x 10⁷ cells. Lysis was achieved using a dounce homogeniser, following which the lysate was centrifuged at 41,000g for 35 minutes. Aspirated supernatant was pumped onto a 5 mm diameter chromatography column containing 500 µl Ni NTA (nitrilo-tri-acetic acid) agarose (Qiagen, product no. 30250) which had been equilibrated in lysis buffer. A baseline level of UV absorbance for the eluent was reached after washing the column with 12 ml of lysis buffer followed by 7 ml of wash buffer (25 mM HEPES pH7.4 at 4°C, 100 mM KCl, 20 mM imidazole, 2 mM 2-mercaptoethanol). Bound Aurora-A protein was eluted from the column using elution buffer (25 mM HEPES pH7.4 at 4°C, 100 mM KCl, 400 mM imidazole, 2 mM 2-mercaptoethanol). An elution fraction (2.5 ml) corresponding to the peak in UV absorbance was collected. The elution fraction, containing active Aurora-A kinase, was dialysed exhaustively against dialysis buffer (25 mM HEPES pH7.4 at 4°C, 45% glycerol (v/v), 100 mM KCl, 0.25% Nonidet P40 (v/v), 1 mM dithiothreitol).

Each new batch of Aurora-A enzyme was titrated in the assay by dilution with enzyme diluent (25 mM Tris-HCl pH7.5, 12.5 mM KCl, 0.6 mM DTT). For a typical batch, stock enzyme is diluted 1 in 666 with enzyme diluent and 20µl of dilute enzyme is used for each assay well. Test compounds (at 10 mM in dimethylsulphoxide (DMSO) were diluted with water and 10 µl of diluted compound was transferred to wells in the assay plates. "Total" and "blank" control wells contained 2.5% DMSO instead of compound. Twenty microlitres of freshly diluted enzyme was added to all wells, apart from "blank" wells. Twenty microlitres of enzyme diluent was added to "blank" wells. Twenty microlitres of reaction mix (25 mM Tris-HCl, 78.4 mM KCl, 2.5 mM NaF, 0.6mM dithiothreitol, 6.25 mM MnCl₂, 6.25 mM ATP, 7.5 µM peptide substrate [biotin-LRRWSLGLRRWSLGLRRWSLGLRRWSLG]) containing 0.2 µCi [γ³³P]ATP (Amersham Pharmacia, specific activity ≥2500Ci/mmol) was then added to all test wells to start the reaction. The plates were incubated at room temperature for 60 minutes. To stop the reaction 100 µl 20% v/v orthophosphoric acid was added to all wells. The peptide substrate was captured on positively-charged nitrocellulose P30 filtermat (Whatman) using a 96-well plate harvester (TomTek) and then assayed for incorporation of ³³P with a Beta plate counter. "Blank" (no enzyme) and "total" (no compound) control values were used to determine the dilution range of test compound which gave 50% inhibition of enzyme activity.

In this test, the compounds of the invention give 50% inhibition of enzyme activity at concentrations of 0.3 nM to 1000 nM and in particular compound 24 in Table 2 gave 50% inhibition of enzyme activity at a concentration of 0.3 nM.

### (b) In Vitro Aurora-B kinase inhibition test

This assay determines the ability of a test compound to inhibit serine-threonine kinase activity. DNA encoding Aurora-B may be obtained by total gene synthesis or by cloning. This DNA may then be expressed in a suitable expression system to obtain polypeptide with serine-threonine kinase activity. In the case of Aurora-B, the coding sequence was isolated from cDNA by polymerase chain reaction (PCR) and cloned into the pFastBac system in a manner similar to that described above for Aurora-A (i.e. to direct expression of a 6-histidine tagged Aurora-B protein).

For the large scale expression of Aurora-B kinase activity, Sf21 insect cells were grown at 28°C in TC100 medium supplemented with 10% foetal calf serum (Viralex) and 0.2% F68 Pluronic (Sigma) on a Wheaton roller rig at 3 r.p.m. When the cell density reached 1.2x10⁶ cells ml⁻¹ they were infected with plaque-pure Aurora-B recombinant virus at a multiplicity of infection of 1 and harvested 48 hours later. All subsequent purification steps were performed at 4°C. Frozen insect cell pellets containing a total of 2.0 x 10⁸ cells were thawed and diluted with lysis buffer (50 mM HEPES (N-[2-hydroxyethyl]piperazine-N'-[2-ethanesulphonic acid]) pH7.5 at 4°C , 1 mM Na₃VO₄, 1 mM PMSF (phenylmethylsulphonyl fluoride), 1 mM dithiothreitol, 1 µg/ml aprotinin, 1 µg/ml pepstatin, 1 µg/ml leupeptin), using 1.0 ml per 2 x 10⁷ cells. Lysis was achieved using a sonication homogeniser, following which the lysate was centrifuged at 41,000g for 35 minutes. Aspirated supernatant was pumped onto a 5 mm diameter chromatography column containing 1.0 ml CM sepharose Fast Flow (Amersham Pharmacia Biotech) which had been equilibrated in lysis buffer. A baseline level of UV absorbance for the eluent was reached after washing the column with 12 ml of lysis buffer followed by 7 ml of wash buffer (50 mM HEPES pH7.4 at 4°C , 1 mM dithiothreitol). Bound Aurora-B B protein was eluted from the column using a gradient of elution buffer (50 mM HEPES pH7.4 at 4°C, 0.6 M NaCl, 1 mM dithiothreitol, running from 0% elution buffer to 100% elution buffer over 15 minutes at a flowrate of 0.5 ml/min). Elution fractions (1.0 ml) corresponding to the peak in UV absorbance was collected. Elution fractions were dialysed exhaustively against dialysis buffer (25 mM HEPES pH7.4 at 4°C, 45% glycerol (v/v), 100 mM KCl, 0.05% (v/v) IGEPAL CA630 (Sigma Aldrich), 1 mM dithiothreitol). Dialysed fractions were assayed for Aurora-B kinase activity.

Each new batch of Aurora-B enzyme was titrated in the assay by dilution with enzyme diluent (25mM Tris-HCl pH7.5, 12.5mM KCl, 0.6mM DTT). For a typical batch, stock enzyme is diluted 1 in 40 with enzyme diluent and 20µl of dilute enzyme is used for each assay well. Test compounds (at 10 mM in dimethylsulphoxide (DMSO) were diluted with water and 10 µl of diluted compound was transferred to wells in the assay plates. "Total" and "blank" control wells contained 2.5% DMSO instead of compound. Twenty microlitres of freshly diluted enzyme was added to all wells, apart from "blank" wells. Twenty microlitres of enzyme diluent was added to "blank" wells. Twenty microlitres of reaction mix (25 mM Tris-HCl, 78.4 mM KCl, 2.5 mM NaF, 0.6mM dithiothreitol, 6.25 mM MnCl₂, 37.5 mM ATP, 25 µM peptide substrate [biotin-LRRWSLGLRRWSLGLRRWSLGLRRWSLG]) containing 0.2 µCi [γ³³P]ATP (Amersham Pharmacia, specific activity ≥2500 Ci/mmol) was then added to all test wells to start the reaction. The plates were incubated at room temperature for 60 minutes. To stop the reaction 100 µl 20% v/v orthophosphoric acid was added to all wells. The peptide substrate was captured on positively-charged nitrocellulose P30 filtermat (Whatman) using a 96-well plate harvester (TomTek) and then assayed for incorporation of ³³P with a Beta plate counter. "Blank" (no enzyme) and "total" (no compound) control values were used to determine the dilution range of test compound which gave 50% inhibition of enzyme activity.

In this test, the compounds of the invention give 50% inhibition of enzyme activity at concentrations of 0.3 nM to 1000 nM and in particular compound 24 in Table 2 gave 50% inhibition of enzyme activity at a concentration of 12.3 nM.

### (c) In Vitro cell proliferation assay

This and other assays can be used to determine the ability of a test compound to inhibit the growth of adherent mammalian cell lines, for example the human tumour cell line SW620 (ATCC CCL-227). This assay determines the ability of at test compound to inhibit the incorporation of the thymidine analogue, 5'-bromo-2'-deoxy-uridine (BrdU) into cellular DNA. SW620 or other adherent cells were typically seeded at 1x10⁵ cells per well in L-15 media (GIBCO) plus 5% foetal calf serum, 1% L-glutamine (100 µl/well) in 96 well tissue culture treated 96 well plates (Costar) and allowed to adhere overnight. The following day the cells were dosed with compound (diluted from 10 mM stock in DMSO using L-15 (with 5% FCS, 1% L-glutamine). Untreated control wells and wells containing a compound known to give 100% inhibition of BrdU incorporation were included on each plate. After 48 hours in the presence / absence of test compound the ability of the cells to incorporate BrdU over a 2 hour labelling period was determined using a Boehringer (Roche) Cell Proliferation BrdU ELISA kit (cat. No. 1 647 229) according to manufacturers directions. Briefly, 15 µl of BrdU labelling reagent (diluted 1:100 in media - L-15, 5% FCS, 1% L-glutamine) was added to each well and the plate returned to a humidified (+5% CO₂) 37°C incubator for 2 hours. After 2 hours the labelling reagent was removed by decanting and tapping the plate on a paper towel. FixDenat solution (50 µl per well) was added and the plates incubated at room temperature for 45 minutes with shaking. The FixDenat solution was removed by decanting and tapping the inverted plate on a paper towel. The plate was then washed once with phosphate buffered saline (PBS) and 100 µl /well of Anti-BrdU-POD antibody solution (diluted 1:100 in antibody dilution buffer) added. The plate was then incubated at room temperature with shaking for 90 minutes. Unbound Anti-BrdU-POD antibody was removed by decanting and washing the plate 4 times with PBS before being blotted dry. TMB substrate solution was added (100µl/well) and incubated for approximately 10 minutes at room temperature with shaking until a colour change was apparent. The optical density of the wells was then determined at 690 nm wavelength using a Titertek Multiscan plate reader. The values from compound treated, untreated and 100% inhibition controls were used to determine the dilution range of a test compound that gave 50% inhibition of BrdU incorporation. The compounds of the invention are active at 0.3 nM to 10000 nM in this test and in particular compound 24 in table 2 was active at 300 nM.

### (d) In Vitro cell cycle analysis assay

This assay determines the ability of a test compound to arrest cells in specific phases of the cell cycle. Many different mammalian cell lines could be used in this assay and SW620 cells are included here as an example. SW620 cells were seeded at 7 x 10⁵ cells per T25 flask (Costar) in 5 ml L-15 (5% FCS, 1% L-glutamine). Flasks were then incubated overnight in a humidified 37°C incubator with 5% CO₂. The following day, 5 µl of L-15 (5% FCS, 1% L-glutamine) carrying the appropriate concentration of test compound solubilised in DMSO was added to the flask . A no compound control treatments was also included (0.5% DMSO). The cells were then incubated for a defined time (24 hours) with compound. After this time the media was aspirated from the cells and they were washed with 5 ml of prewarmed (37°C) sterile PBSA, then detached from the flask by brief incubation with trypsin and followed by resuspension in 5 ml of 1% Bovine Serum Albumin (BSA, Sigma-Aldrich Co.) in sterile PBSA. The samples were then centrifuged at 2200 rpm for 10 minutes. The supernatant was aspirated to leave 200 µl of the PBS/BSA solution. The pellet was resuspended in this 200 µl of solution by pipetting 10 times to create a single cell suspension. One ml of ice-cold 80% ethanol was slowly added to each cell suspension and the samples stored at -20°C overnight or until required for staining. Cells were pelleted by centrifugation, ethanol aspirated off and pellets resuspended in 200µl PBS containing 100 µg/ml RNAse (Sigma Aldrich) and 10 µg/ml Propidium Iodide (Sigma Aldrich). Cell suspensions were incubated at 37°C for 30minutes, a further 200 µl PBS added and samples stored in the dark at 4°C overnight.

Each sample was then syringed 10 times using 21-guage needle. The samples were then transferred to LPS tubes and DNA content per cell analysed by Fluorescence activated cell sorting (FACS) using a FACScan flow cytometer (Becton Dickinson). Typically 30,000 events were counted and recorded using CellQuest v1.1 software (Verity Software). Cell cycle distribution of the population was calculated using Modfit software (Verity Software) and expressed as percentage of cells with 2N (G0/G1), 2N-4N (S phase) and with 4N (G2/M) DNA content.

The compounds of the invention are active in this test at 0.3 nM to 10000 nM and in particular compounds 24 in table 2 was active at 1.5 µM

The invention will now be illustrated in the following non limiting examples, in which standard techniques known to the skilled chemist and techniques analogous to those described in these Examples may be used where appropriate, and in which, unless otherwise stated:
(i) evaporations were carried out by rotary evaporation *in vacuo* and work up procedures were carried out after removal of residual solids such as drying agents by filtration;
(ii) operations were carried out at ambient temperature, typically in the range 18-25°C and in air unless stated, or unless the skilled person would otherwise operate under an atmosphere of an inert gas such as argon;
(iii) column chromatography (by the flash procedure) and medium pressure liquid chromatography (MPLC) were performed on Merck Kieselgel silica (Art. 9385);
(iv) yields are given for illustration only and are not necessarily the maximum attainable;
(v) the structures of the end products of the formula (I) were generally confirmed by nuclear (generally proton) magnetic resonance (NMR) and mass spectral techniques; proton magnetic resonance chemical shift values were measured in deuterated dimethyl sulphoxide (DMSO d₆) (unless otherwise stated) on the delta scale (ppm downfield from tetramethylsilane) using one of the following four instruments
- Varian Gemini 2000 spectrometer operating at a field strength of 300 MHz
- Bruker DPX300 spectrometer operating at a field strength of 300MHz
- JEOL EX 400 spectrometer operating at a field strength of 400 MHz
- Bruker Avance 500 spectrometer operating at a field strength of 500MHz
Peak multiplicities are shown as follows: s, singlet; d, doublet; dd, double doublet; t, triplet; q, quartet; qu, quintet; m, multiplet; br s, broad singlet.
(vi) robotic synthesis was carried out using a Zymate XP robot, with solution additions via a Zymate Master Laboratory Station and stirred via a Stem RS5000 Reacto-Station at 25°C;
(vii) work up and purification of reaction mixtures from robotic synthesis was carried out as follows: evaporations were carried out *in vacuo* using a Genevac HT 4; column chromatography was performed using either an Anachem Sympur MPLC system on silica using 27 mm diameter columns filled with Merck silica (60 µm, 25 g); the structures of the final products were confirmed by LCMS on a Waters 2890 / ZMD micromass system using the following and are quoted as retention time (RT) in minutes:

| | |
|---|---|
| Column: | waters symmetry C18 3.5 µm 4.6x50 mm |
| Solvent A: | H₂O |
| Solvent B: | CH₃CN |
| Solvent C : | methanol + 5% HCOOH |
| Flow rate: | 2.5 ml / min |
| Run time: | 5 minutes with a 4.5 minute gradient from 0-100% C |
| Wavelength: | 254 nm, bandwidth 10 nm |
| Mass detector: | ZMD micromass |
| Injection volume | 0.005 ml |

(viii) Analytical LCMS for compounds which had not been prepared by robotic synthesis was performed on a Waters Alliance HT system using the following and are quoted as retention time (RT) in minutes:

| | |
|---|---|
| Column: | 2.0 mm x 5 cm Phenomenex Max-RP 80A |
| Solvent A: | Water |
| Solvent B: | Acetonitrile |
| Solvent C: | Methanol / 1 % formic acid or Water / 1% formic acid |
| Flow rate: | 1.1 ml / min |
| Run time: | 5 minutes with a 4.5 minute gradient from 0-95% B + constant 5% solvent C |
| Wavelength: | 254 nm, bandwidth 10 nm |
| Injection volume | 0.005 ml |
| Mass detector: | Micromass ZMD |

(ix) Preparative high performance liquid chromatography (HPLC) was performed on either

| - Waters preparative LCMS instrument, with retention time (RT) measured in minutes: | |
|---|---|
| Column: | β-basic Hypercil (21x100 mm) 5µm |
| Solvent A: | Water / 0.1% Ammonium carbonate |
| Solvent B: | Acetonitrile |
| Flow rate: | 25 ml / min |
| Run time: | 10 minutes with a 7.5 minute gradient from 0-100% B |
| Wavelength: | 254 nm, bandwidth 10 nm |
| Injection volume | 1 - 1.5 ml |
| Mass detector : | Micromass ZMD |

| - Gilson preparative HPLC instrument, with retention time (RT) measured in minutes: | |
|---|---|
| Column: | 21 mm x 15 cm Phenomenex Luna2 C18 |
| Solvent A: | Water + 0.1% trifluoracetic acid, |
| Solvent B: | Acetonitrile + 0.1% trifluoracetic acid |
| Flow rate: | 21 ml / min |
| Run time: | 20 minutes with various 10 minute gradients from 5-100% B |
| Wavelength: | 254 nm, bandwidth 10 nm |
| Injection volume | 0.1-4.0 ml |

(x) intermediates were not generally fully characterised and purity was assessed by thin layer chromatography (TLC), HPLC, infra-red (IR), MS or NMR analysis.

Particular examples of compounds of formula (I) are set out in Tables 1, 2 and 3:

**TABLE 1**

| | | |
|---|---|---|
| 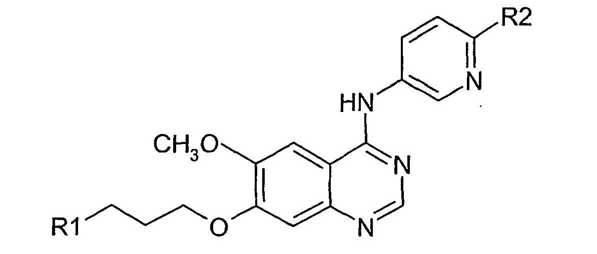 | | |

| Compound | R1 | R2 |
|---|---|---|
| 1 | 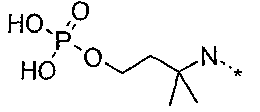 | OCH₂-(3-chlorophenyl) |
| 2 | 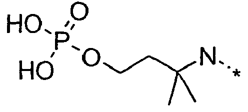 | NH-CO-(3-chlorophenyl) |
| 3 | 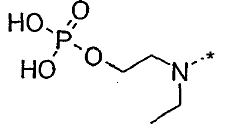 | NH-CO-(3-chlorophenyl) |
| 4 | 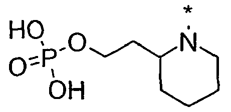 | NH-CO-(3-chlorophenyl) |
| 5 | 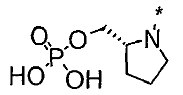 | NH-CO-(3-chlorophenyl) |
| 6 | 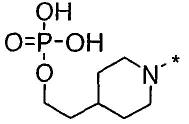 | NH-CO-(3-chlorophenyl) |
| 7 | 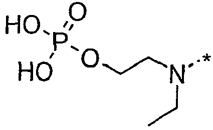 | NH-CO-(3-fluorophenyl) |
| 8 | 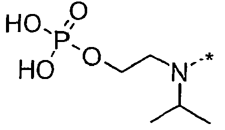 | NH-CO-(3,4-difluorophenyl) |
| 9 | 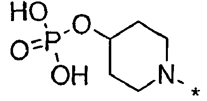 | NH-CO-(3-chlorophenyl) |
| 10 | 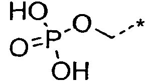 | NH-CO-(3-chlorophenyl) |
| 11 | 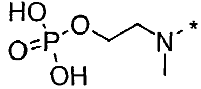 | NH-CO-(3-chlorophenyl) |
| 12 | 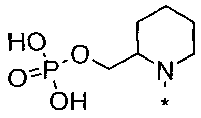 | NH-CO-(3-chlorophenyl) |
| 13 | 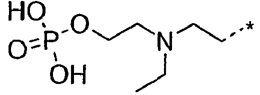 | NH-CO-(3-chlorophenyl) |
| 14 | 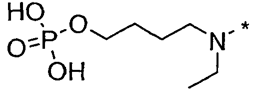 | NH-CO-(3-chlorophenyl) |
| 15 | 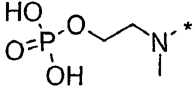 | NH-CO-(3-fluorophenyl) |
| 16 | 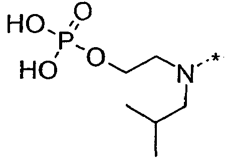 | NH-CO-(3-chlorophenyl) |
| 17 | 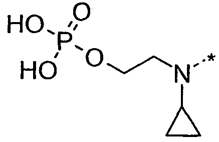 | NH-CO-(3-chlorophenyl) |
| 18 | 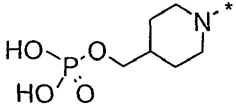 | NH-CO-(3-chlorophenyl) |
| 19 | 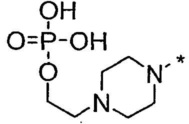 | NH-CO-(3-chlorophenyl) |
| 20 | 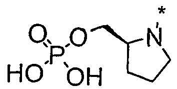 | NH-CO-(3-chlorophenyl) |
| 21 | 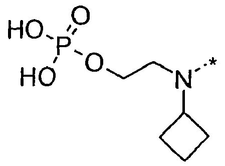 | NH-CO-(3-chlorophenyl) |
| 22 | 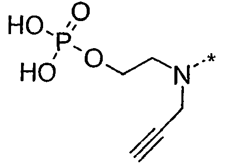 | NH-CO-(3-chlorophenyl) |

**TABLE 2**

| | | |
|---|---|---|
| 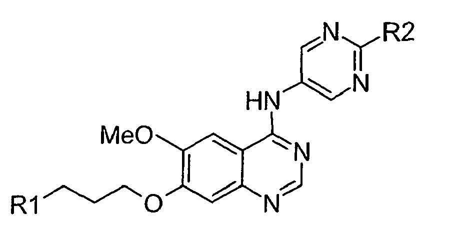 | | |

| | R1 | R2 |
|---|---|---|
| 23 | 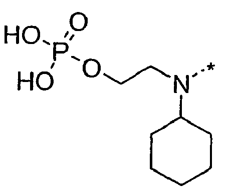 | NH-CO-(3-chloro,4-fluorophenyl) |
| 24 | 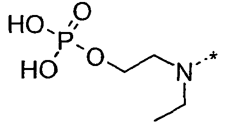 | NH-CO-(3-chloro-4-fluorophenyl) |
| 25 | 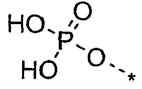 | NH-CO-(3-chlorophenyl) |
| 26 | 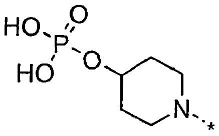 | CH₂NH-(3-chloro-4-fluorophenyl) |
| 27 | 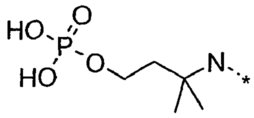 | OCH₂-(3-chloro-4-fluorophenyl) |
| 28 | 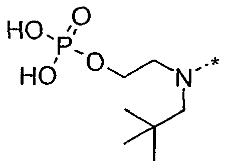 | NH-CO-(3-chlorophenyl) |

**TABLE 3**

| | | |
|---|---|---|
| 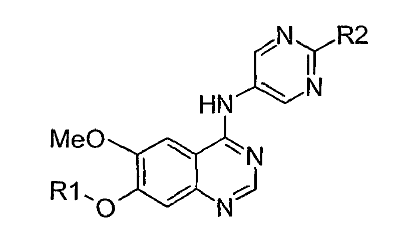 | | |

| Compound | R1 | R2 |
|---|---|---|
| 29 | 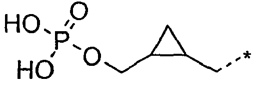 | NH-CO-(3-chloro-4-fluorophenyl) |
| 30 | 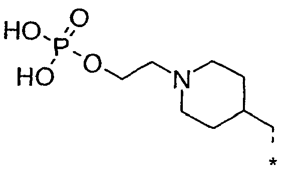 | NH-CO-(3-chloro-4-fluorophenyl) |

### Example 1 - Preparation of Compound 1 in Table 1 - 3-[(3-{[4-({6-[(3-chlorobenzyl)oxy]pyridin-3-yl}amino)-6-methoxyquinazolin-7-yl]oxy}propyl)amino]-3-methylbutyl dihydrogen phosphate

3-[(3-{[4-({6-[(3-chlorobenzyl)oxy]pyridin-3-yl}amino)-6-methoxyquinazolin-7-yl]oxy}propyl)amino]-3-methylbutan-1-ol (1.7 g, 3.1 mmol) and dry 1*H*-tetrazole (647 mg, 9.23 mmol) were dissolved with heating (50°C) in dry dimethylacetamide (8 ml) under a nitrogen atmosphere. Di-*tert*-butyl-*N*,*N*-diethylphosphoramidite (1.2 ml, 4.3 mmol) was added dropwise to the reaction mixture and left to stir at ambient temperature for 20 hours. The reaction mixture was diluted with dichloromethane (160 ml) and washed with aqueous sodium bicarbonate solution (50 ml of a saturated solution). The aqueous layer was further extracted with dichloromethane (150 ml) and the combined organics were dried (sodium sulphate), filtered and concentrated under reduced pressure to yield di-*tert*-butyl 3-[(3-{[4-({6-[(3-chlorobenzyl)oxy]pyridin-3-yl}amino)-6-methoxyquinazolin-7-yl]oxy}propyl)amino]-3-methylbutyl phosphite as a viscous, yellow oil. The oil was dissolved in tetrahydrofuran (10 ml) and hydrogen peroxide (680 µl of a 30 % w/w aqueous solution, - 8.8 N, 6.00 mmol) was added slowly at 0 °C. The reaction was warmed to ambient temperature over 10 minutes. The resulting homogeneous solution was stirred for 2 hours then cooled to 0 °C and a solution of sodium metabisulphite (11.5 ml of a 0.53 N aqueous solution) was introduced dropwise. The reaction was warmed to ambient temperature over 15 minutes then diluted with ethyl acetate (100 ml). Aqueous sodium bicarbonate solution (100 ml of a saturated aqueous solution) was added, the phases separated and the aqueous layer further extracted with ethyl acetate (3 x 100 ml). The combined organics were dried (sodium sulphate), filtered and concentrated under reduced pressure to yield di-*tert*-butyl 3-[(3-{[4-({6-[(3-chlorobenzyl)oxy]pyridin-3-yl}amino)-6-methoxyquinazolin-7-yl]oxy}propyl)amino]-3-methylbutyl phosphate as a viscous, yellow oil (1.97 g), which was used in the next step of the reaction sequence without further purification.
³¹p-NMR {¹H}(CDCl₃): -8.73 (s, 1P):
MS (+ve ESI): 744 (M+H)⁺.

Hydrogen chloride (4.30 ml of a 4.0 N solution in 1,4-dioxane, 17.2 mmol) was added, dropwise to a solution of the crude phosphate ester (1.97 g) in 1,4-dioxane (86 ml) upon which a white solid precipitated from the reaction mixture. The resulting heterogeneous reaction mixture was stirred for a further 19 hours and diethyl ether was added (100 ml). The precipitate was filtered and washed with diethyl ether (3 x 30 ml) then dried under high vacuum for 48 hours to yield the title compound, 3-[(3-{[4-({6-[(3-chlorobenzyl)oxy]pyridin-3-yl}amino)-6-methoxyquinazolin-7-yl]oxy}propyl)amino]-3-methylbutyl dihydrogen phosphate (di-hydrochloride salt) as an off-white solid (1.7 g, 91 % yield) :
¹H-NMR (DMSO-d₆): 11.86 (s, 1H), 9.12 (br s, 2H), 8.81 (s, 1H), 8.53 (s, 1H), 8.50 (d, 1H), 8.08 (dd, 1H), 7.54 (br s, 1H), 7.46-7.39 (m, 4H), 7.05 (d, 1H), 5.41 (s, 2H), 4.34 (t, 2H), 4.05 (s, 3H), 3.98 (dd, 2H), 3.11 (br s, 2H), 2.30 (m, 2H), 2.05 (t, 2H), 1.36 (s, 6H) :
³¹p-NMR {¹H}(DMSO-d₆): -0.05 (s, 1P) :
MS (-ve ESI): 630 (M-H)⁻,
MS (+ve ESI): 632 (M+H)⁺.
3-[(3-{[4-({6-[(3-chlorobenzyl)oxy]pyridin-3-yl}amino)-6-methoxyquinazolin-7-yl]oxy}propyl)amino]-3-methylbutan-1-ol, used as the starting material, was obtained as follows:
a) A mixture of 2-amino-4-benzyloxy-5-methoxybenzamide (10 g, 0.04 mol), (prepared according *to* J. Med. Chew. 1977, 20, 146-149), and Gold's reagent (7.4 g, 0.05 mol) in dioxane (100 ml) was stirred and heated at reflux for 24 hours. Sodium acetate (3.02 g, 0.04 mol) and acetic acid (1.65 ml, 0.03 mol) were added to the reaction mixture and it was heated for a further 3 hours. The volatiles were removed by evaporation and water was added to the residue. The solid was collected by filtration, washed with water and dried. Recrystallisation from acetic acid yielded 7-(benzyloxy)-6-methoxyquinazolin-4(3*H*)-one (8.7 g, 84 % yield) as a white solid.
b) Chloromethyl pivalate (225ml), 1.56 mol) was added dropwise to a stirred mixture of 7-(benzyloxy)-6-methoxyquinazolin-4(3*H*)-one (400 g, 1.42 mol) and potassium carbonate (783 g, 5.67 mol) in dimethylacetamide (5500 ml). The reaction was heated to 90 °C for 4 hours. The reaction was cooled and filtered to remove inorganic salts. The filtrate was concentrated in *vacuo* to yield, crude [7-(benzyloxy)-6-methoxy-4-oxoquinazolin-3(4*H*)-yl]methyl pivalate (562 g, 100 % yield) :
   ¹H-NMR (DMSO-d₆) : 8.33 (s, 1H), 7.30-7.50 (m, 6H), 7.25 (s, 1H), 5.90 (s, 2H), 5.25 (s, 2H), 3.88 (s, 3H), 1.10 (s, 9H) :
   MS (+ve ESI): 397 (M+H)⁺.
c) 10 % palladium on carbon (56 g, 53 mmol) was added to a solution of [7-(benzyloxy)-6-methoxy-4-oxoquinazolin-3(4*H*)-yl]methyl pivalate (562 g, 1.42 mmol) in dimethylacetamide (3500 ml) at ambient temperature and stirred for 3 hours under an atmosphere of hydrogen (1 bar). The reaction was filtered through a pad of celite and the solvent evaporated *in vacuo*. The residual solid was dissolved in 20 % methanol in dichloromethane and passed through a pad of silica gel. Evaporation of the solvent *in vacuo* followed by trituration with methanol yielded, (7-hydroxy-6-methoxy-4-oxoquinazolin-3(4*H*)-yl)methyl pivalate (188 g, 43 % yield) :
   ¹H-NMR (DMSO-d₆) : 8.25 (s, 1H), 7.45 (s, 1H), 6.97 (s, 1H), 5.85 (s, 2H), 4.04 (s, 1H), 3.87 (s, 3H), 1.10 (s, 9H) :
   MS (+ve ESI): 307 (M+H)⁺.
d) A mixture of (7-hydroxy-6-methoxy-4-oxoquinazolin-3(4*H*)-yl)methyl pivalate (100 g, 0.33 mol), 3-bromopropanol (49.3 g, 0.35 mol) and potassium carbonate (133 g, 0.96 mol) in dimethylformamide (500 ml) was stirred at 80 °C for 20 hours. The reaction was cooled and concentrated to quarter volume *in vacuo*. The residue was poured into ice/water (1500 ml) and the resulting solid collected by suction filtration. Purification by crystallisation from ethanol, yielded 7-(3-hydroxypropoxy)-6-methoxy-4-oxoquinazolin-3(4*H*)-yl methyl pivalate (33.8 g, 41 % yield) as a beige solid :
   ¹H-NMR (DMSO-d₆) : 7.95 (s, 1H), 7.43 (s, 1H), 7.10 (s, 1H), 4.16 (t, 2H), 3.86 (m, 5H), 2.08 (t, 2H), 1.12 (s, 9H) :
   MS (+ve ESI): 365 (M+H)⁺.
e) An aqueous sodium hydroxide solution (100 ml, 0.20 mol) was added to a solution of yielded 7-(3-hydroxypropoxy)-6-methoxy-4-oxoquinazolin-3(4*H*)-yl methyl pivalate (33.8 g, 93.0 mmol) in methanol (300 ml) and the solution heated to reflux for 1 hour. The methanol was evaporated *in vacuo*, the residue was acidified with aqueous hydrochloric acid, sodium bicarbonate was added and the solid was collected by suction filtration. Washing with water and drying yielded 7-(3-hydroxypropoxy)-6-methoxyquinazolin-4(3*H*)-one (26 g, 95 % yield):
   ¹H-NMR (DMSO-d₆) : 7.96 (s, 1H), 7.41 (s, 1H), 7.07 (s, 1H), 4.14 (t, 2H), 3.84 (s, 3H), 3.55 (t, 2H), 1.90 (t, 2H) :
   MS (+ve ESI): 251 (M+H)⁺.
f) 7-(3-hydroxypropoxy)-6-methoxyquinazolin-4(3*H*)-one (25.0 g, 100 mmol) was added slowly to a solution of dimethylformamide (1 ml) in thionyl chloride (250 ml). The mixture was heated to reflux for 4 hours then cooled and the solvents evaporated *in vacuo*. The residue was dissolved in dichloromethane and washed with aqueous sodium bicarbonate, brine, dried over magnesium sulphate and evaporated. Trituration and collection of the solid by suction filtration yielded 4-chloro-7-(3-chloropropoxy)-6-methoxyquinazoline (19.5 g, 68 % yield) as a yellow solid :
   ¹H-NMR (CDCl₃) : 8.85 (s, 1H), 7.40 (s, 1H), 7.38 (s, 1H), 4.38 (t, 2H), 4.03 (s, 3H), 3.8 (t, 2H), 2.40 (m, 2H) :
   MS (+ve ESI): 287 (M+H)⁺.
g) 4-chloro-7-(3-chloropropoxy)-6-methoxyquinazoline (574 mg, 2.00 mmol) and 5-amino-2-(3-chlorobenzyloxy)pyridine (468 mg, 2.0 mmol, see WO 0121597) were heated in dimethylacetamide (10 ml) at 100 °C for 4 hours. The reaction was cooled to ambient temperature and the solid collected by suction filtration and washed with diethyl ether (25 ml) and acetone (5 ml). Drying *in vacuo* yielded *N*-{6-[(3-chlorobenzyl)oxy]pyridin-3-yl}-7-(3-chloropropoxy)-6-methoxyquinazolin-4-amine (640 mg, 66 % yield) as a white solid :
   ¹H-NMR (DMSO-d₆) : 11.60 (s, 1H), 8.78 (s, 1H), 8.46 (s, 1H), 8.37 (s, 1H), 8.04 (dd, 1H), 7.52 (s, 1H), 7.39 (s, 1H), 7.03 (d, 1H), 5.39 (s, 2H), 4.29 (t, 2H), 4.01 (s, 3H), 3.82 (t, 2H), 2.29 (m, 2H) :
   MS (+ve ESI): 485.5 (M+H)⁺.
h) *N-*{6-[(3-chlorobenzyl)oxy]pyridin-3-yl}-7-(3-chloropropoxy)-6-methoxyquinazolin-4-amine (1.83 g, 3.77 mmol), 3-amino-3-methylbutanol (1.94 g, 18.9 mmol) and potassium iodide (62 mg, 0.38 mmol) in dimethylacetamide (10 ml) was heated at 90 °C for 16 hours. The mixture was cooled, poured into 2.0 N aqueous ammonia (150 ml) and the supernatant decanted off and extracted with ethyl acetate (2 x 50ml). The insoluble gum was dissolved in dichloromethane/methanol mixture, combined with the ethyl acetate fraction and evaporated *in vacuo*. Purification by flash chromatography on silica gel, eluting with dichloromethane :
   methanol : aqueous ammonia (100:5:0.3 to 100:25:2) yielded 3-[(3-{[4-({6-[(3-chlorobenzyl)oxy]pyridin-3-yl}amino)-6-methoxyquinazolin-7-yl]oxy}propyl)amino]-3-methylbutan-1-ol (1.56 g, 75 % yield) as an off-white solid :
   ¹H-NMR (DMSO-d₆): 9.52 (s, 1H), 8.40 (m, 2H), 8.09 (dd, 1H), 7.80 (s, 1H), 7.53 (s, 1H), 7.41 (m, 3H), 7.18 (s, 1H), 6.97 (d, 1H), 5.38 (s, 2H), 4.19 (t, 1H), 3.96 (s, 3H), 3.52 (t, 2H), 2.64 (t, 2H), 1.88 (t, 2H), 1.51 (t, 2H), 1.03 (s, 6H) :
   MS (-ve ESI): 550.7 (M-H)⁻,
   MS (+ve ESI): 552.7 (M+H)⁺.

### Example 2 - Preparation of Compound 2 in Table 1 - 3-[(3-{[4-({6-[(3 chlorobenzoyl)amino]pyridin-3-yl}amino)-6-methoxyquinazolin-7 yl]oxy}propyl)amino]-3-methylbutyl dihydrogen phosphate

Di-*tert*-butyl-*N*,*N*-diethylphosphoramidite (660 mg, 2.65 mmol) was added slowly to a mixture of 3-chloro-*N*-{5-[(7-{3-[(3-hydroxy-1,1-dimethylpropyl)amino]propoxy}-6-methoxyquinaxolin-4-yl)amino]pyridin-2-yl}benzamide (742 mg, 1.31 mmol) and 1*H-*tetrazole (273 mg, 3.9 mmol) in dimethylacetamide (1.7 ml). The reaction was stirred under an inert atmosphere at ambient temperature for 24 hours during which a further portion of di-*tert*-butyl-*N*,*N*-diethylphosphoramidite (330 mg, 1.32 mmol) was added. Dichloromethane (34 ml), methanol (1.7 ml) and 20 % aqueous potassium hydrogen carbonate solution (5 ml) were added and the reaction mixture stirred briefly. The aqueous layer was removed by filtration through a Varian CE1020 Chem Elut Hydromatrix cartridge. Solvent evaporation *in vacuo* gave a yellow oil which was taken up in tetrahydrofuran (2 ml), cooled to 0 °C and treated with 30 % aqueous hydrogen peroxide (0.4 ml, 3.9 mmol). The mixture was allowed to warm to ambient temperature and stirred for 2 hours. The reaction was cooled to 0 °C, quenched with aqueous sodium metabisulphite, treated with 20 % aqueous potassium hydrogen carbonate and extracted into 10:1 dichloromethane : methanol (4 x 10 ml). Solvent evaporation *in vacuo* gave a yellow oil, which was taken up in dioxane (40 ml) and treated dropwise with a 4.0 N solution of hydrogen chloride in dioxane (1.96 ml, 7.86 mmol). The white slurry was stirred at ambient temperature for 18 hours, diluted with an equal volume of dichloromethane and the solution absorbed onto flash silica. Purification by flash chromatography, eluting with dichloromethane : methanol: formic acid : water (100:20:3:3 to 100:40:10:10), yielded the title compound (as the trihydrochloride salt, 380 mg, 38 % yield) as a pale yellow solid :
¹H-NMR (DMSO-d₆ + CD₃COOD): 8.80 (s, 1H), 8.73 (d, 1H), 8.30 (m, 2H), 8.19 (dd, 1H), 8.04 (m, 1H), 7.96 (d, 1H), 7.60 (m, 1H), 7.50 (t, 1H), 7.39 (s, 1H), 4.31 (t, 2H), 4.02 (m, 5H), 3.11 (t, 2H), 2.27 (m, 2H), 2.04 (m, 2H), 1.35 (s, 6H) :
³¹P-NMR (DMSO-d₆ + CD₃COOD): 0.08 (s, 1P):
MS (+ve ESI): 645.7, 647.7 (M+H)⁺.
3-chloro-*N*-{5-[(7-{3-[(3-hydroxy-1,1-dimethylpropyl)amino]propoxy}-6-methoxyquinazolin-4-yl)amino]pyridin-2-yl}benzamide, used as the starting material, was obtained as follows:
a) 3-chlorobenzoyl chloride (22.1 g, 127 mmol) was added dropwise to a stirred solution of 2-amino-5-ntropyridine (16.0 g, 115 mmol) in pyridine (240 ml) and heated to 100 °C for 16 hours. The solution was evaporated *in vacuo*, the resulting solid was taken-up in dichloromethane (200 ml) and eluted through silica gel (800 g), with dichloromethane. The relevant fractions were combined and concentrated under reduced pressure to yield 3-chloro-*N*-(5-nitropyridin-2-yl)benzamide (31.6 g, 99 % yield) as an off-white solid :
   ¹H-NMR (DMSO-d₆): 11.64 (s, 1H), 9.20 (d, 1H), 8.63 (dd, 1H), 8.40 (d, 1H), 8.06 (s, 1H), 7.98 (d, 1H), 7.69 (d, 1H), 7.55 (dd, 1H) :
   MS (-ve ESI) 276 (M-H)⁻,
   MS (+ve ESI): 278 (M+H)⁺,
b) 3-Chloro-*N*-(5-nitropyridin-2-yl)benzamide (31.5 g, 113 mmol) and 10 % platinum on carbon (3.6 g) in dichloromethane was stirred under a hydrogen atmosphere (1 bar pressure) for 18 hours. The suspension was filtered through Celite®, the filter cake was washed with ethyl acetate : ethanol (10:1, 1400 ml) and the filtrate concentrated under reduced pressure to yield *N*-(5-aminopyridin-2-yl)-3-chlorobenzamide (26.3 g, 94 % yield) as an off-white powdery solid :
   ¹H-NMR (DMSO-d₆): 10.50 (s, 1H), 8.03 (s, 1H), 7.96 (d, 1H), 7.80 (d, 1H), 7.76 (d, 1H), 7.61 (d, 1H), 7.52 (dd, 1H), 7.04 (dd, 1H), 5.22 (s, 2H) :
   MS (-ve ESI): 246 (M-H)⁻,
   MS (+ve ESI): 248 (M+H)⁺,
c) 4-chloro-7-(3-chloropropoxy)-6-methoxyquinazoline (2.87 g, 10.0 mmol) and *N*-(5-aminopyridin-2-yl)-3-chlorobenzamide (2.54 g, 10.0 mmol) were suspended in dry dimethylacetamide (100 ml) under a nitrogen atmosphere and warmed to 50 °C. Hydrogen chloride (2.5 ml of a 4.0 N solution in 1,4-dioxane, 10 mmol) was added dropwise, causing precipitation of a yellow solid from the reaction mixture. The resulting heterogeneous reaction mixture was stirred for 4.5 hours at 80 °C then cooled to ambient temperature. Diethyl ether (200 ml) was added and the solid collected by suction filtration to yield 3-chloro-*N*-(5-{[7-(3-chloropropoxy)-6-methoxyquinazolin-4-yl]amino}pyridin-2-yl)benzamide hydrochloride as a pale yellow solid (4.83 g, 90 % yield) :
   ¹H-NMR (DMSO-d₆): 11.64 (br s, 1H), 11.04 (br s, 1H), 8.83 (s, 1H), 8.75 (d, 1H), 8.39 (s, 1H), 8.25 (d, 1H), 8.18 (dd, 1H), 8.08 (s, 1H), 7.97 (d, 1H), 7.65 (d, 1H), 7.55 (t, 1H), 7.39 (s, 1H), 4.30 (t, 2H), 4.03 (s, 3H), 3.82 (t, 2H), 2.29 (m, 2H) :
   MS (-ve ESI): 496.3 (M-H)⁻,
   MS (+ve ESI): 498.3 (M+H)⁺,
d) 3-chloro-*N*-(5-{[7-(3-chloropropoxy)-6-methoxyquinazolin-4-yl]amino}pyridin-2-yl)benzamide hydrochloride (1.07 g, 2.00 mmol), 3-amino-3-methylbutanol (1.03 g, 10.0 mmol) and potassium iodide (32 mg, 0.2 mmol) in dimethylacetamide (2.5 ml) was heated at 80 °C for 4 days. The mixture was cooled, diluted with dichloromethane (25 ml) and the solution absorbed onto silica gel. Purification by flash chromatography, eluting with dichloromethane : methanol: aqueous ammonia (100:5:0.5 to 100:25:2), yielded 3-chloro-*N-*{5-[(7-{3-[(3-hydroxy-1,1-dimethylpropyl)amino]propoxy}-6-methoxyquinazolin-4-yl)amino]pyridin-2-yl}benzamide as a pale yellow solid (804 mg, 71 % yield) :
   ¹H-NMR (DMSO-d₆): 10.92 (br s, 1H), 9.68 (s, 1H), 8.80 (s, 1H), 8.48 (s, 1H), 8.29 (d, 1H), 8.19 (d, 1H), 8.08 (s, 1H), 8.00 (d, 1H), 7.88 (s, 1H), 7.66 (d, 1H), 7.55 (dd, 1H), 7.20 (s, 1H), 4.20 (t, 2H), 3.97 (s, 3H), 3.50 (m, 2H), 2.74 (t, 2H), 1.94 (m, 2H), 1.57 (m, 2H), 1.07 (s, 6H):
   MS (-ve ESI): 563 (M-H)⁻,
   MS (+ve ESI): 565 (M+H)⁺.

### Example 3 - Preparation of Compound 3 in Table 1 - 2-[(3-{[4-({6-[(3 chlorobenzoyl)amino]pyridin-3-yl}amino)-6-methoxyquinaxolin-7-yl]oxy}propyl)(ethyl)amino]ethyl dihydrogen phosphate

An analogous reaction to that described in example 2, but starting with 3-chloro-*N*-{5-[(7-{3-[ethyl(2-hydroxyethyl)amino]propoxy}-6-methoxyquinazolin-4-yl)amino]pyridin-2-yl}benzamide (244 mg, 0.443 mmol), yielded the title compound (as the trihydrochloride salt, 174 mg, 53 % yield) as a pale yellow solid :
¹H-NMR (DMSO-d₆ + CD₃COOD): 8.79 (s, 1H), 8.71 (d, 1H), 8.31 (d, 1H), 8.16 (dd, 1H), 8.08 (s, 2H), 8.00 (d, 1H), 7.63 (m, 1H), 7.53 (m, 2H), 4.41 (t, 2H), 4.20 (m, 2H), 4.02 (s, 3H), 3.41 (m, 2H), 3.32 (t, 2H), 3.26 (q, 2H), 2.24 (m, 2H), 1.28 (t, 3H) :
MS (+ve ESI): 631.6, 633.6 (M+H)⁺.
3-chloro-*N*-{5-[(7-{3-[ethyl(2-hydroxyethyl)amino]propoxy}-6-methoxyquinazolin-4-yl)amino]pyridin-2-yl}benzamide, used as the starting material, was obtained as described in example 2d but starting with 2-(ethylamino)ethanol (334 mg, 3.75 mmol). The reaction yielded 3-chloro-*N*-{5-[(7-{3-[ethyl(2-hydroxyethyl)amino]propoxy}-6-methoxyquinazolin-4-yl)amino]pyridin-2-yl}benzamide (304 mg, 74 % yield) as a pale yellow solid :
¹H-NMR (DMSO-d₆): 9.59 (s, 1H), 8.77 (d, 1H), 8.45 (s, 1H), 8.26 (dd, 1H), 8.18 (d, 1H), 8.07 (t, 1H), 7.98 (d, 1H), 7.81 (s, 1H), 7.65 (m, 1H), 7.54 (t, 1H), 7.18 (s, 1H), 4.26 (t, 1H), 4.17 (t, 2H), 3.96 (s, 3H), 3.42 (q, 2H), 2.58 (t, 2H), 2.50 (m, 4H), 1.98 (m, 2H), 0.95 (t, 3H) :
MS (-ve ESI): 549.6 (M-H)⁻,
MS (+ve ESI): 551.5 (M+H)⁺.

### Example 4 - Preparation of Compound 4 in Table 1 - 2-[1-(3-{[4-({6-[(3-chlorobenzoyl)amino]pyridin-3-yl}amino)-6-methoxyquinazolin-7 yl]oxy}propyl)piperidin-2-yl]ethyl dihydrogen phosphate

An analogous reaction to that described in example 2, but starting with 3-chloro-*N*-{5-[(7-{3-[2-(2-hydroxyethyl)piperidin-1-yl]propoxy}-6-methoxyquinazolin-4-yl)amino]pyridin-2-yl}benzamide (286 mg, 0.48 mmol), yielded the title compound (as the trihydrochloride salt, 114 mg, 31 % yield) as a pale yellow solid :
¹H-NMR (DMSO-d6 + CD₃COOD): 8.92 (s, 1H), 8.71 (d, 1H), 8.31 (d, 1H), 8.17 (m, 1H), 8.09 (s, 1H), 7.98 (d, 1H), 7.63 (m, 1H), 7.52 (m, 1H), 7.36 (s, 1H), 4.32 (t, 2H), 4.03 (s, 3H), 3.96 (m, 2H), 3.30 (m, 5H), 2.30 (m, 2H), 1.95 (m, 4H), 1.74 (m, 4H), 1.52 (m, 2H) :
MS (+ve ESI): 671.7, 673.7 (M+H)⁺.
3-chloro-*N*-{5-[(7-{3-[2-(2-hydroxyethyl)piperidin-1-yl]propoxy}-6-methoxyquinazolin-4-yl)amino]pyridin-2-yl}benzamide, used as the starting material, was obtained as described in example 2d but starting with 2-(hydroxyethyl)piperidine (1.29 g, 10.0 mmol). The reaction yielded 3-chloro-*N*-{5-[(7-{3-[2-(2-hydroxyethyl)piperidin-1-yl]propoxy}-6-methoxyquinazolin-4-yl)amino]pyridin-2-yl}benzamide (913 mg, 77 % yield) as a pale yellow solid:
¹H-NMR (DMSO-d₆): 10.91 (s, 1H), 9.72 (br s, 1H), 8.81 (d, 1H), 8.46 (s, 1H), 8.29 (dd, 1H), 8.20 (d, 1H), 8.10 (t, 1H), 8.00 (d, 1H), 7.91 (s, 1H), 7.65 (m, 1H), 7.55 (t, 1H), 7.20 (s, 1H), 4.20 (t, 2H), 3.99 (s, 3H), 3.50 (m, 2H), 3.00 (m, 2H), 2.80 (m, 2H), 2.60 (m, 1H), 2.03 (m, 2H), 1.93 (m, 1H), 1.72 (m, 1H), 1.57 (m, 4H), 1.39 (m, 2H) :
MS (-ve ESI): 589.6 (M-H)⁻,
MS (+ve ESI): 591.5 (M+H)⁺.

### Example 5 - Preparation of Compound 5 in Table 1 - [(2R)-1-(3-{[4-({6-[(3-chlorobenzoyl)amino]pyridin-3-yl}amino)-6-methoxyquinazolin-7-yl]oxy}propyl)pyrrolidin-2-yl]methyl dihydrogen phosphate

An analogous reaction to that described in example 2, but starting with 3-chloro-*N*-{5-[(7-{3-[(2*R*)-2-(hydroxymethyl)pyrrolidin-1-yl]propoxy}-6-methoxyquinazolin-4-yl)amino]pyridin-2-yl}benzamide (78 mg, 0.139 mmol), yielded the title compound (as the trihydrochloride salt, 49 mg, 47 % yield) as a pale yellow solid :
¹H-NMR (DMSO-d₆ + CD₃COOD): 8.85 (s, 1H), 8.74 (m, 1H), 8.32 (d, 1H), 8.21 (m, 2H), 8.09 (s, 2H), 7.99 (d, 1H), 7.66 (m, 1H), 7.55 (t, 1H), 7.44 (s, 1H), 4.35 (m, 2H), 4.21 (m, 1H), 4.12 (m, 1H), 4.04 (s, 3H), 3.80 (m, 1H), 3.65 (m, 1H), 3.50 (m, 1H), 3.28 (m, 2H), 2.32 (m, 2H), 2.19 (m, 1H), 1.95 (m, 3H) :
MS (+ve ESI): 643.6, 645.6 (M+H)⁺.
3-chloro-*N*-{5-[(7-{3-[(2*R*)-2-(hydroxymethyl)pyrrolidin-1-yl]propoxy}-6-methoxyquinazolin-4-yl)amino]pyridin-2-yl}benzamide used as the starting material was obtained as follows:
A mixture of 3-chloro-*N*-(5-{[7-(3-chloropropoxy)-6-methoxyquinazolin-4-yl]amino}pyridin-2-yl)benzamide (100 mg, 0.20 mmol), (2*R*)-pyrrolidin-2-ylmethanol (102 mg, 1.00 mmol) and tetra-*n*-butylammonium iodide (7.5 mg, 0.02 mmol) in dimethylacetamide (1 ml) was heated at 60 °C for 17 hours. The mixture was cooled and diluted with dichloromethane (10 ml) and purified by flash chromatography on silica gel, eluting with dichloromethane : methanol: 7.0 N ammonia in methanol (9:1:0 to 9:1:0.8) yielded 3-chloro-*N*-{5-[(7-{3-[(2*R*)-2-(hydroxymethyl)pyrrolidin-1-yl]propoxy}-6-methoxyquinazolin-4-yl)amino]pyridin-2-yl}benzamide (107 mg, 95 % yield) as a yellow solid:
   ¹H-NMR (DMSO-d₆): 10.96 (s, 1H), 9.76 (s, 1H), 8.82 (d, 1H), 8.48 (s, 1H), 8.30 (dd, 1H), 8.21 (d, 1H), 8.10 (s, 1H), 8.00 (d, 1H), 7.94 (s, 1H), 7.67 (d, 1H), 7.55 (dd, 1H), 7.21 (s, 1H), 4.20 (t, 2H), 3.98 (s, 3H), 3.48 (m, 2H), 2.73 (m, 2H), 1.55-2.15 (m, 7H) :
   MS (-ve ESI): 561.5 (M-H)⁻,
   MS (+ve ESI): 563.6 (M+H)⁺,

### Example 6 - Preparation of Compound 6 in Table 1 - 2-[1-(3-{[4-({6-[(3-chlorobenzoyl)amino]pyridin-3-yl}amino)-6-methoxyquinazolin-7-yl]oxy}propyl)piperidin-4-yl]ethyl dihydrogen phosphate

An analogous reaction to that described in example 2, but starting with 3-chloro-*N*-{5-[(7-{3-[4-(2-hydroxyethyl)piperidin-1-yl]propoxy}-6-methoxyquinazolin-4-yl)amino]pyridin-2-yl}benzamide (200 mg, 0.34 mmol), yielded the title compound (as the trihydrochloride salt, 137 mg, 45 % yield) as a pale yellow solid :
¹H-NMR (DMSO-d₆ + CD₃COOD): 8.82 (s, 1H), 8.75 (d, 1H), 8.35 (s, 1H), 8.27 (m, 2H), 8.06 (s, 1H), 7.98 (d, 1H), 7.62 (m, 1H), 7.50 (m, 1H), 7.37 (s, 1H), 4.29 (m, 2H), 4.02 (s, 3H), 3.90 (q, 2H), 3.53 (d, 2H), 3.22 (m, 3H), 2.93 (t, 2H), 2.31 (m, 2H), 1.90 (m, 2H), 1.70 (m, 1H), 1.55 (m, 3H) :
MS (+ve ESI): 671.7, 673.7 (M+H)⁺.
3-chloro-*N*-{5-[(7-{3-[4-(2-hydroxyethyl)piperidin-1-yl]propoxy}-6-methoxyquinazolin-4-yl)amino]pyridin-2-yl}benzamide used as the starting material, was obtained as described in example 2d but starting with 2-(piperidin-4-yl)ethanol (1.73 ml, 20.0 mmol). The reaction yielded 3,chloro-*N*-{5-[(7-{3-[4-(2-hydroxyethyl)piperidin-1-yl]propoxy}-6-methoxyquinazolin-4-yl)amino]pyridin-2-yl}benzamide (1.4 g, 59 % yield) as a yellow solid :
¹H-NMR (DMSO-d₆): 10.96 (br s, 1H), 9.61 (s, 1H), 8.79 (br s, 1H), 8.48 (s, 1H), 8.30 (m, 1H), 8.21 (d, 1H), 8.10 (s, 1H), 8.00 (d, 1H), 7.84 (s, 1H), 7.64 (d, 1H), 7.58 (m, 1H), 7.21 (s, 1H), 4.30 (m, 2H), 4.19 (m, 2H), 3.98 (s, 3H), 3.48 (m, 2H), 2.73 (m, 2H), 2.41 (m, 2H), 1.98-1.80 (m, 3H), 1.60 (d, 2H), 1.45 (br s, 2H), 1.05-1.20 (m, 2H):
MS (-ve ESI): 589.5 (M-H)⁻,
MS (+ve ESI): 591.5 (M+H)⁺.

### Example 7 - Preparation of Compound 7 in Table 1 - 2-[ethyl(3-{[4-({6-[(3-fluorobenzoyl)amino]pyridin-3-yl}amino)-6-methoxyquinazolin-7-yl]oxy}propyl)amino]ethyl dihydrogen phosphate

Di-*tert*-butyl-*N,N*-diethylphosphoramidite (630 mg, 2.52 mmol) was added to a mixture of 3-fluoro-*N*-{5-[(7-{3-[ethyl(2-hydroxyethyl)amino]propoxy}-6-methoxyquinazolin-4-yl)amino]pyridin-2-yl}benzamide (673 mg, 1.26 mmol) and dry 1 *H-*tetrazole (176 mg, 2.52 mmol) in dimethylacetamide (10 ml). The reaction was stirred under an inert atmosphere at ambient temperature for 160 minutes. Dichloromethane (100 ml) and 20 % aqueous potassium hydrogen carbonate solution (5 ml) were added and the reaction mixture stirred briefly. The aqueous layer was removed by filtration through a Varian CE1020 Chem Elut Hydromatrix cartridge and the organic fraction was evaporated in *vacuo* to give a yellow oil which was taken up in tetrahydrofuran (5 ml). The solution was cooled to -5 °C, 30 % aqueous hydrogen peroxide (0.21 ml, 1.89 mmol) was added slowly and the reaction was stirred at 0 °C for 1 hour. The reaction was washed with aqueous sodium metabisulphite and 20 % aqueous potassium hydrogen carbonate solution and extracted into dichloromethane. Solvent evaporation *in vacuo* gave a yellow oil which was purified by chromatography on silica gel, eluting with dichloromethane : methanol : concentrated aqueous ammonia (200:16:1) to yield a pale yellow oil. This was taken up in dioxane (20 ml) and treated dropwise with a 4.0 N solution of hydrogen chloride in dioxane (1.89 ml, 7.56 mmol). The white slurry was stirred at ambient temperature for 18 hours, diluted with methanol (40 ml) and dichloromethane (40 ml) and the solution absorbed onto flash silica. Purification by flash chromatography, eluting with dichloromethane : methanol : formic acid : water (100:20:3:3 to 100:40:10:10), yielded the title compound (as the trihydrochloride salt, 324 mg, 37 % yield) as a pale yellow solid :
¹H-NMR (DMSO-d₆ + CD₃COOD): 8.82 (d, 1H), 8.58 (s, 1H), 8.30 (dd, 2H), 8.20 (d, 1H), 7.92 (s, 1H), 7.88 (d, 1H), 7.83 (d, 1H), 7.53 (dd, 1H), 7.39 (m, 1H), 4.24 (t, 2H), 4.08 (m, 2H), 3.99 (s, 3H), 3.26 (m, 6H), 2.24 (m, 2H), 1.25 (t, 3H) :
³¹P-NMR (DMSO-d₆ + CD₃COOD): 1.68 (s, 1P) :
MS (+ve ESI): 615.5 (M+H)⁺.
3-fluoro-*N*-{5-[(7-{3-[ethyl(2-hydroxyethyl)amino]propoxy}-6-methoxyquinazolin-4-yl)amino]pyridin-2-yl}benzamide, used as the starting material, was obtained as follows:
a) An analogous reaction to that described in example 2a but starting with 3-fluorobenzoyl chloride (4.95g, 31.2mmol) yielded *N*-(5-nitropyridin-2-yl)-3-fluorobenzamide as a light yellow solid (6.44 g, 88 % yield) :
   ¹H-NMR (DMSO-d₆): 11.58 (br s, 1H), 9.21 (s, 1H), 8.64 (dd, 1H), 8.42 (d, 1H), 7.87 (m, 2H), 7.58 (m, 1H), 7.46 (m, 2H) :
   MS (-ve ESI): 260 (M-H)⁻,
   MS (+ve ESI): 262 (M+H)⁺.
b) An analogous reaction to that described in example 2b but starting with *N*-(5-nitropyridin-2-yl)-3-fluorobenzamide (5.8g, 22.2mmol) yielded *N*-(5-aminopyridin-2-yl)-3-fluorobenzamide as a cream solid (5.0 g, 96 % yield):
   ¹H-NMR (DMSO-d₆): 10.38 (br s, 1H), 7.79 (m, 4H), 7.55 (m, 1H), 7.38 (t, 1H), 7.01 (dd, 1H), 5.18 (br s, 2H) :
   MS (-ve ESI): 230 (M-H)⁻,
   MS (+ve ESI): 232 (M+H)⁺.
c) An analogous reaction to that described in example 2c but starting with *N*-(5-aminopyridin-2-yl)-3-fluorobenzamide (0.69 g, 3.0 mmol) yielded 3-fluoro-*N*-(5-{[7-(3-chloropropoxy)-6-methoxyquinazolin-4-yl]amino}pyridin-2-yl)benzamide hydrochloride as a pale yellow solid (1.52 g, 98 % yield) :
   ¹H-NMR (DMSO-d₆): 11.78 (br s, 1H), 11.02 (br s, 1H), 8.84 (s, 1H), 8.76 (d, 1H), 8.45 (s, 1H), 8.21 (m, 2H), 7.86 (m, 2H), 7.55 (m, 1H), 7.43 (m, 2H), 4.28 (t, 2H), 4.03 (s, 3H), 3.81 (t, 2H), 2.28 (m, 2H) :
   MS (-ve ESI): 480.0 (M-H)⁻,
   MS (+ve ESI): 482.0 (M+H)⁺.
d) An anlogous reaction to that described in example 2d but starting with 3-fluoro-*N*-(5-{[7-(3-chloropropoxy)-6-methoxyquinazolin-4-yl]amino}pyridin-2-yl)benzamide hydrochloride (1.03 g, 2.00 mmol) and 2-(ethylamino)ethanol (0.89 g, 10.0 mmol) yielded 3-fluoro-*N*-{5-[(7-{3-[ethyl(2-hydroxyethyl)amino]propoxy}-6-methoxyquinazolin-4-yl)amino]pyridin-2-yl}-benzamide (667 mg, 62 % yield) as a pale yellow solid :
   ¹H-NMR (DMSO-d₆): 9.59 (s, 1H), 8.77 (d, 1H), 8.44 (s, 1H), 8.26 (dd, 1H), 8.19 (d, 1H), 7.85 (m, 3H), 7.56 (m, 1H), 7.42 (m, 1H), 7.17 (s, 1H), 4.35 (br s, 1H), 4.18 (t, 2H), 3.96 (s, 3H), 3.44 (q, 2H), 2.44 (m, 6H), 1.91 (m, 2H), 0.97 (t, 3H) :
   MS (-ve ESI): 533.6 (M-H)⁻,
   MS (+ve ESI): 535.6 (M+H)⁺.

### Example 8 - Preparation of Compound 8 in Table 1 - 2-[(3-{[4-({6-[(3,4-difluorobenzoyl)amino]pyridin-3-yl}amino)-6-methoxyquinazolin-7-yl]oxy}propyl)(isopropyl)amino]ethyl dihydrogen phosphate

An analogous reaction to that described in example 7, but starting with 3,4-difluoro-*N-*{5-[(7-{3-[(2-hydroxyethyl)(isopropyl)amino]propoxy}-6-methoxyquinazolin-4-yl)amino]pyridin-2-yl}benzamide (652 mg, 1.15 mmol), yielded the title compound (as a dihydrochloride salt, 398 mg, 45 % yield) as a pale yellow solid :
¹H-NMR (DMSO-d₆ + CD₃COOD): 8.76 (m, 2H), 8.29 (s, 1H), 8.22 (m, 2H), 8.10 (m, 1H), 7.92 (m, 1H), 7.66 (s, 1H), 7.54 (dd, 1H), 7.37 (s, 1H), 4.41 (t, 2H), 4.18 (m, 2H), 4.04 (s, 3H), 3.74 (m, 1H), 3.37 (m, 2H), 3.23 (m, 2H), 2.26 (m, 2H), 1.28 (d, 6H) :
MS (+ve ESI): 647.5 (M+H)⁺.
3,4-difluoro-*N*-{5-[(7-{3-[(2-hydroxyethyl)(isopropyl)amino]propoxy}-6-methoxyquinazolin-4-yl)amino]pyridin-2-yl}benzamide, used as the starting material, was obtained as follows :
a) An analogous reaction to that described in example 2a but starting with 3,4-difluorobenzoyl chloride (19.4 g, 110 mmol) yielded *N*-(5-nitropyridin-2-yl)-3,4-difluorobenzamide (24.9 g, 89 % yield) :
   ¹H-NMR (DMSO-d₆): 11.60 (br s, 1H), 9.20 (d, 1H), 8.63 (dd, 1H), 8.38 (d, 1H), 8.11 (m, 1H), 7.92 (m, 1H), 7.59 (d, 1H) :
   MS (+ve ESI): 280.3 (M+H)⁺.
b) An analogous reaction to that described in example 2b but starting with *N*-(5-nitropyridin-2-yl)-3,4-difluorobenzamide (24.7 g, 88.4 mmol) yielded *N*-(5-aminopyridin-2-yl)-3,4-difluorobenzamide (21 g, 95 % yield) as an off-white powder :
   ¹H-NMR (DMSO-d₆): 10.44 (br s, 1H), 8.06 (m, 1H), 7.94 (m, 1H), 7.73 (m, 1H), 7.00 (m, 1H), 5.18 (br s, 2H) :
   MS (+ve ESI): 250.3 (M+H)⁺.
c) An analogous reaction to that described in example 2c but starting with *N*-(5-aminopyridin-2-yl)-3,4-difluorobenzamide (1.24 g, 5.0 mmol) yielded 3,4-difluoro-*N*-(5-{[7-(3-chloropropoxy)-6-methoxyquinazolin-4-yl]amino}pyridin-2-yl)benzamide hydrochloride as a pale yellow solid (2.56 g, 96 % yield) :
   ¹H-NMR (DMSO-d₆): 8.84 (s, 1H), 8.75 (d, 1H), 8.43 (s, 1H), 8.20 (m, 2H), 8.10 (m, 1H), 7.93 (m, 1H), 7.59 (m, 1H), 7.41 (m, 1H), 4.28 (t, 2H), 4.03 (s, 3H), 3.81 (t, 2H), 2.27 (m, 2H):
   MS (-ve ESI): 498.5 (M-H)⁻,
   MS (+ve ESI): 500.5 (M+H)⁺.
d) An analogous reaction to that described in example 2d but starting with 3,4-difluoro-*N-*(5-{[7-(3-chloropropoxy)-6-methoxyquinazolin-4-yl]amino}pyridin-2-yl)benzamide hydrochloride (0.40 g, 0.75 mmol) and 2-(isopropylamino)ethanol (0.39 g, 3.75 mmol) yielded 3,4-difluoro-*N*-{5-[(7-{3-[(2-hydroxyethyl)(isopropyl)amino]propoxy}-6-methoxyquinazolin-4-yl)amino]pyridin-2-yl}benzamide (193 mg, 45 % yield) as a pale yellow solid:
   ¹H-NMR (DMSO-d₆): 10.92 (s, 1H), 9.60 (s, 1H), 8.77 (d, 1H), 8.46 (s, 1H), 8.26 (dd, 1H), 8.19 (d, 1H), 8.13 (m, 1H), 7.95 (m, 1H), 7.84 (s, 1H), 7.57 (m, 1H), 7.17 (s, 1H), 4.21 (m, 3H), 3.96 (s, 3H), 3.36 (m, 2H), 2.88 (m, 1H), 2.56 (m, 2H), 2.45 (m, 2H), 1.85 (m, 2H), 0.91 (d, 6H) :
   MS (-ve ESI): 565.4 (M-H)⁻,
   MS (+ve ESI): 567.3 (M+H)⁺.

### Example 9 - Preparation of Compound 9 in Table 1 1-(3-{[4-({6-[(3-chlorobenzoyl)amino]pyridin-3-yl}amino)-6-methoxyquinazolin-7-yl]oxy}propyl)piperidin-4-yl dihydrogen phosphate

3-chloro-*N*-[5-({7-[3-(4-hydroxypiperidin-1-yl)propoxy]-6-methoxyquinazolin-4-yl}amino)pyridin-2-yl]benzamide (281 mg, 0.50 mmol) and dry 1 *H*-tetrazole (157 mg, 2.30 mmol) were dissolved in dry dimethylacetamide (10.6 ml). Di-*tert*-butyl-*N,N* diethylphosphoramidite (210 ul, 0.74 mmol) was added and the reaction stirred at ambient temperature for 2 hours. Further portions of 1 *H-*tetrazole (157 mg, 2.3 mmol) and di-*tert-*butyl-*N,N*-diethylphosphoramidite (210 µl, 0.74 mmol) were added and the reaction mixture stirred for 18 hours. The reaction was cooled to - 40 °C and 3-chloroperoxybenzoic acid (366 mg, 1.49 mmol) added. The reaction was warmed to ambient temperature, concentrated and suspended in a solution of sodium metabisulphite (10 ml of a 0.26 M solution). This was extracted with ethyl acetate and the organics washed with sodium bicarbonate (saturated solution), dried with magnesium sulphate and concentrated. Purification by flash chromatography on alumina, eluting with dichloromethane : methanol : saturated ammonia (97:2:1) yielded di-*tert*-butyl 1-(3-{[4-({6-[(3-chlorobenzoyl)amino]pyridin-3-yl}amino)-6-methoxyquinazolin-7-yl]oxy}propyl)piperidin-4-yl phosphate as a yellow solid (210 mg, 56 % yield):
¹H-NMR (DMSO-d₆): 10.91 (s, 1H), 9.59 (s, 1H), 8.77 (d, 1H), 8.45 (s, 1H), 8.26 (dd, 1H), 8.18 (d, 1H), 8.09 (s, 1H), 7.98 (d, 1H), 7.82 (s, 1H), 7.65 (d, 1H), 7.54 (t, 1H), 7.18 (s, 1H), 4.17 (m, 3H), 3.96 (s, 3H), 3.36 (m, 2H), 2.62 (m, 2H), 2.44 (t, 2H), 2.21 (t, 2H), 1.87 (m, 4H), 1.63, (m, 2H), 1.39 (s, 18H).

Hydrogen chloride (150 µl of a 4.0 N solution in 1,4-dioxane, 0.6 mmol) was added, dropwise to a solution of di-*tert*-butyl 1-(3-{[4-({6-[(3-chlorobenzoyl)amino]pyridin-3-yl}amino)-6-methoxyquinazolin-7-yl]oxy}propyl)piperidin-4-yl phosphate (70 mg, 0.1 mmol) in 1,4-dioxane (3 ml). The resulting heterogeneous reaction mixture was stirred for a further hour and diethyl ether was added (10 ml). The precipitate was filtered and washed with diethyl ether (3 x 10 ml) then dried under high vacuum for 48 hours to yield the title compound 1-(3-{[4-({6-[(3-chlorobenzoyl)amino]pyridin-3-yl}amino)-6 methoxyquinazolin-7-yl]oxy}propyl)piperidin-4-yl dihydrogen phosphate (di-hydrochloride) as an off-white solid (34 mg, 53 % yield) :
¹H-NMR (DMSO-d₆): 11.98 (s, 1H), 11.11 (s, 1H), 8.87 (s, 1H), 8.79 (s, 1H), 8.58 (s, 1H), 8.24 (s, 2H), 8.09 (s, 1H), 7.99 (d, 1H), 7.68 (dd, 1H), 7.55 (t, 1H), 7.44 (s, 1H), 4.52 (m, 1H), 4.31 (t, 2H), 4.05 (s, 3H), 3.49 (m, 2H), 3.24 (m, 2H), 3.08 (m, 2H), 2.35 (m, 2H), 2.14 (m, 2H), 2.05 (t, 2H), 2.00 (m, 2H) : MS (+ve ESI): 643.2 (M+H)⁺.
3-chloro-*N-*[5-({7-[3-(4-hydroxypiperidin-1-yl)propoxy]-6-methoxyquinazolin-4-yl}amino)pyridin-2-yl]benzamide, used as the starting material was obtained as described in example 2d but starting with 4-hydroxypiperidine (1.83 g, 18 mmol). The reaction yielded the desired compound as an off-white solid (860 mg, 85 % yield) :
¹H-NMR (DMSO-d₆): 10.48 (s, 1H), 9.35 (br s, 1H), 8.80 (s, 1H), 7.99 (d, 1H), 7.84 (s, 1H), 7.61 (d, 1H), 7.53 (t, 1H), 7.21 (s, 1H), 4.22 (t, 2H), 4.06 (s, 1H), 3.99 (s, 3H), 3.49 (m, 1H), 2.72 (m, 2H), 2.48 (m, 2H), 2.11 (d, 2H), 1.95 (m, 2H), 1.72 (m, 2H), 1.43 (m, 2H) : MS (+ve ESI): 563 (M+H)⁺.

### Example 10 - Preparation of Compound 10 in Table 1 - 4-{[4-({6-[(3-chlorobenzoyl)amino]pyridin-3-yl}amino)-6-methoxyquinazolin-7-yl]oxy}butyl dihydrogen phosphate

An analogous reaction to that described in example 9, but starting with 3-chloro-*N*-(5-{[7-(4-hydroxybutoxy)-6-methoxyquinazolin-4-yl]amino}pyridin-2-yl)benzamide (493 mg, 1.0 mmol) yielded the title compound as a pale yellow solid (250 mg, 88 % yield) : ¹H-NMR (DMSO-d₆): 11.81 (s, 1H), 11.08 (s, 1H), 8.85 (s, 1H), 8.76 (d, 1H), 8.46 (s, 1H), 8.23 (m, 2H), 8.09 (s, 1H), 7.98 (d, 1H), 7.67 (d, 1H), 7.55 (t, 1H), 7.41 (s, 1H), 4.22 (t, 2H), 4.04 (s, 3H), 3.91 (q, 2H), 1.91 (m, 2H), 1.77 (m, 2H):
³¹P-NMR (DMSO-d₆): 0.00 (t, 1P):
MS (+ve ESI): 574 (M+H)⁺.
3-chloro-*N*-(5-{[7-(4-hydroxybutoxy)-6-methoxyquinazolin-4-yl]amino}pyridin-2-yl)benzamide used as the starting material was obtained as follows:
a) An analogous reaction to that described in example 2c, but starting with 7-(benzyloxy)-4-chloro-6-methoxyquinazoline (615 mg, 1.12 mmol-see WO 9722596) and N-(5-aminopyridin-2-yl)-3-chlorobenzamide ( 362 mg, 1.46 mmol) afforded *N-*(5-{[7-(benzyloxy)-6-methoxyquinazolin-4-yl]amino}pyridin-2-yl)-3-chlorobenzamide (625 mg, 78 % yield) :
   ¹H-NMR (DMSO-d₆): 11.23 (s, 1H), 10.65 (br s, 1H), 8.82 (s, 1H), 8.73 (s, 1H), 8.40 (s, 1H), 8.25 (m, 2H), 8.10 (s, 1H), 8.02 (d, 1H), 7.65 (d, 1H), 7.53 (m, 4H), 7.38 (m, 3H), 5.36 (s, 2H), 4.08 (s, 3H).
b) *N*-(5-{[7-(benzyloxy)-6-methoxyquinazolin-4-yl]amino}pyridin-2-yl)-3-chlorobenzamide (615 mg, 1.12 mmol) was dissolved in trifluoroacetic acid (6 ml) and heated to 90 °C for 2 hours. The excess trifluoroacetic acid was evaporated and water was added cautiously to the slurry. This was added to a solution of ammonia (10 ml) and stirred rapidly. The resulting precipitate was isolated, washed with water and dried under high vacumm. The solid was triturated with acetone to afford 3-chloro-*N-*{5-[(7-hydroxy-6-methoxyquinazolin-4-yl)amino]pyridin-2-yl}benzamide (435 mg, 92 % yield) :
   ¹H-NMR (DMSO-d₆): 10.90 (s, 1H), 10.32 (s, 1H), 9.53 (s, 1H), 8.77 (d, 1H), 8.39 (s, 1H), 8.25 (m, 1H), 8.18 (d, 1H), 8.09 (s, 1H), 7.98 (d, 1H), 7.81 (s, 1H), 7.65 (d, 1H), 7.53 (t, 1H), 7.06 (s, 1H), 3.97 (s, 3H) :
   MS (+ve ESI): 422 (M+H)⁺.
c) 3-chloro-*N-*{5-[(7-hydroxy-6-methoxyquinazolin-4-yl)amino]pyridin-2-yl}benzamide (431 mg, 1.01 mmol), 4-bromobutyloxyacetate (294 mg, 1.5 mmol) and potassium carbonate (414 mg, 3.00 mmol) were dissolved in dimethylacetamide (15 ml) and stirred at ambient temperature for 18 hours. The reaction mixture was concentrated and water (10 ml) added. The resultant precipitate was removed by filtration, washed with water and dried under high vacuum. The solid was recrystallised from acetonitrile to afford 4-{[4-({6-[(3-chlorobenzoyl)amino]pyridin-3-yl}amino)-6-methoxyquinazolin-7-yl]oxy}butyl acetate (337 mg, 63 % yield) :
   ¹H-NMR (DMSO-d₆): 8.76 (s, 1H), 8.45 (s, 1H), 8.26 (m, 1H), 8.20 (d, 1H), 8.09 (s, 1H), 7.99 (d, 1H), 7.83 (s, 1H), 7.66 (d, 1H), 7.54 (t, 1H), 7.19 (s, 1H), 4.18 (t, 2H), 4.08 (t, 2H), 3.95 (s, 3H), 2.00 (s, 3H), 1.84 (m, 2H), 1.75 (m, 2H) :
   MS (+ve ESI): 536 (M+H)⁺.
d) 4-{[4-({6-[(3-chlorobenzoyl)amino]pyridin-3-yl}amino)-6-methoxyquinazolin-7-yl]oxy}butyl acetate (100 mg, 0.18 mmol) was dissolved in methanol (2.5 ml) and water (2.5 ml). Sodium hydroxide (15 mg, 0.36 mmol) was added and the reaction heated to 90 °C for 1 hour. The reaction was cooled and the precipitate was isolated by filtration, washed with water and dried under high vacuum to yield 3-chloro-*N*-(5-{[7-(4-hydroxybutoxy)-6-methoxyquinazolin-4-yl]amino}pyridin-2-yl)benzamide (64 mg, 72 % yield) :
   ¹H-NMR (DMSO-d₆): 10.91 (s, 1H), 9.59 (s, 1H), 8.77 (d, 1H), 8.47 (s, 1H), 8.26 (m, 1H), 8.19 (d, 1H), 8.09 (s, 1H), 7.99 (d, 1H), 7.83 (s, 1H), 7.66 (d, 1H), 7.54 (t, 1H), 7.18 (s, 1H), 4.45 (t, 1H), 4.14 (t, 2H), 3.97 (s, 3H), 3.47 (q, 2H), 1.84 (m, 2H), 1.61 (m, 2H): MS (+ve ESI): 494 (M+H)⁺.

### Example 11 - Preparation of Compound 11 in Table 1 - 2-[(3-{[4-({6-[(3-chlorobenzoyl)amino]pyridin-3-yl}amino)-6-methoxyquinazolin-7-yl]oxy}propyl)(methyl)amino]ethyl dihydrogen phosphate

Di-*tert*-butyl-*N,N*-diethylphosphoramidite (730 µl, 2.60 mmol) was added to a mixture of 3-chloro-*N*-{5-[(7-{3-[(2-hydroxyethyl)(methyl)amino]propoxy}-6-methoxyquinazolin-4-yl)amino]pyridin-2-yl}benzamide (1.00 g, 1.86 mmol) and dry 1 *H*-tetrazole (400 mg, 5.59 mmol) in dimethylacetamide (25 ml). The reaction was stirred under an inert atmosphere at ambient temperature for 18 hours. Dichloromethane (150 ml) and aqueous sodium hydrogen carbonate solution (50 ml) were added and the reaction mixture stirred briefly. The aqueous layer was extracted with dichloromethane and the combined organics dried (sodium sulphate) and concentrated to give a yellow oil which was taken up in tetrahydrofuran (15 ml). The mixture was cooled to 0 °C and 30 % aqueous hydrogen peroxide (0.50 ml, 3.72 mmol) was added slowly and the reaction was stirred at 0 °C for 1 hour. A further equivalent of 30 % aqueous hydrogen peroxide was added and the reaction allowed to warm to ambient temperature. The reaction was cooled to 0 °C and a solution of sodium metabisulphite (30 ml, 0.52 M solution) was added. This mixture was warmed to ambient temperature and extracted with ethyl acetate (4 x 100 ml). The combined organics were dried (sodium sulphite) and concentrated to afford the crude phosphate ester as a yellow oil:
MS (+ve ESI) : 729 (M+H)⁺.
This was taken up in dioxane (70 ml) and treated dropwise with a 4.0 N solution of hydrogen chloride in dioxane (5 ml, 20 mmol). The white slurry was stirred at ambient temperature for 18 hours and diethyl ether was added (200 ml). The precipitate was filtered and washed with diethyl ether (3 x 30 ml) then dried under high vacuum to yield the title compound 2-[(3-{[4-({6-[(3-chlorobenzoyl)amino]pyridin-3-yl}amino)-6-methoxyquinazolin-7-yl]oxy}propyl)(methyl)amino]ethyl dihydrogen phosphate (di-hydrochloride) as an off-white solid (1.2 g, 93 % yield) :
¹H-NMR (DMSO-d₆): 11.95 (m, 1H), 11.11 (s, 1H), 8.88 (s, 1H), 8.81 (s, 1H), 8.59 (m, 1H), 8.25 (m, 2H), 8.11 (m, 1H), 8.03 (d, 1H), 7.68 (m, 1H), 7.56 (t, 1H), 7.50 (m, 1H), 4.26 (m, 4H), 4.06 (s, 3H), 3.44 (m, 2H), 3.35 (m, 2H), 2.88 (s, 3H), 2.34 (m, 2H):
MS (+ve ESI) : 617 (M+H)⁺.

3-chloro-*N*-{5-[(7-{3-[(2-hydroxyethyl)(methyl)amino]propoxy}-6-methoxyquinazolin-4-yl)amino]pyridin-2-yl}benzamide used as the starting material was obtained as described in example 2d but starting with 2-(methylamino)ethanol (210 mg, 2.80 mmol). The reaction yielded the desired compound as an off-white solid (51 mg, 34 % yield) : ¹H-NMR (DMSO-d₆): 10.50 (s, 1H), 9.41 (s, 1H), 8.81 (s, 1H), 8.46 (s, 1H), 8.27 (dd, 1H), 8.15 (d, 1H), 8.08 (s, 1H), 7.99 (d, 1H), 7.88 (s, 1H), 7.62 (d, 1H), 7.53 (t, 1H), 7.22 (s, 1H), 4.24 (t, 2H), 4.02 (s, 3H), 3.57 (t, 2H), 2.75 (t, 2H), 2.65 (t, 2H), 2.38 (s, 3H), 2.02 (m, 2H) : MS (+ve ESI) : 537 (M+H)⁺.

### Example 12 - Preparation of Compound 12 in Table 1-[1-(3-{(4-({6-[(3-chlorobenzoyl)amino]pyridin-3-yl}amino)-6-methoxyquinazolin-7-yl]oxy}propyl)piperidin-2-yl]methyl dihydrogen phosphate

Di-*tert*-butyl-*N,N*-diethylphosphoramidite (136 µl, 0.49 mmol) was added to a mixture of 3-chloro-*N*-{5-[(7-{3-[2-(hydroxymethyl)piperidin-1-yl]propoxy}-6-methoxyquinazolin-4-yl)amino]pyridin-2-yl}benzamide (202 mg, 0.35 mmol) and dry 1 *H*-tetrazole (61 mg, 0.87 mmol) in dimethylacetamide (0.5 ml). The reaction was stirred under an inert atmosphere at ambient temperature for 18 hours before addition of another equivalent of each reagent and further stirring for 6 hours. Dichloromethane (10 ml) and methanol (0.5 ml) were added and the mixture washed with aqueous potassium carbonate solution (20 %, 10 ml). The organics were dried (magnesium sulphate) and concentrated to give a yellow oil which was taken up in tetrahydrofuran (2 ml). The mixture was cooled to 0 °C and 30 % aqueous hydrogen peroxide (72 µl, 0.7 mmol) was added slowly and the reaction was stirred at 0 °C for 1 hour. A further equivalent of 30 % aqueous hydrogen peroxide was added and the reaction allowed to warm to ambient temperature. The reaction was cooled to 0 °C and a solution of sodium metabisulphite (0.5 ml, 0.52 M solution) was added. This mixture was warmed to ambient temperature and extracted with dichloromethane : methanol (10:1). The combined organics were dried (magnesium sulphate) and concentrated to afford the crude phosphate ester as a yellow oil. This was taken up in dioxane (11 ml) and treated dropwise with a 4.0 N solution of hydrogen chloride in dioxane (0.55 ml, 2.20 mmol). The white slurry was stirred at ambient temperature for 18 hours and diethyl ether was added (200 ml). The precipitate was filtered and washed with acetonitrile then purified according to the method described in example 2 to yield the title compound 1-(3-{[4-({6-[(3-chlorobenzoyl)amino]pyrridin-3-yl}amino)-6-methoxyquinazolin-7-yl]oxy}propyl)piperidin-2-yl]methyl dihydrogen phosphate (dihydrochloride) as an off-white solid (140 mg, 64 % yield) :
¹H-NMR (DMSO-d₆+ CD₃COOD): 8.75 (s, 1H), 8.60 (br s, 1H), 8.22 (m, 2H), 8.06 (s, 1H), 7.98 (m, 2H), 7.61 (m, 1H), 7.50 (m, 1H), 7.42 (s, 1H), 4.29 (t, 2H), 4.11 (m, 1H), 3.99 (s, 3H), 3.85 (m, 1H), 3.23 (m, 1H), 3.09 (m, 1H), 2.52 (m, 2H), 2.25 (m, 2H), 1.73 (m, 5H), 1.50 (m, 2H) :
MS (+ve ESI): 657 (M+H)⁺.
3-chloro-*N*-{5-[(7-{3-[2-(hydroxymethyl)piperidin-1-yl]propoxy}-6-methoxyquinazolin-4-yl)amino]pyridin-2-yl}benzamide used as the starting material was obtained as described in example 2d but starting with 2-(hydroxymethyl)piperidine (323 mg, 2.8 mmol). The reaction yielded the desired compound as an off-white solid (63 mg, 39 % yield) :
¹H-NMR (DMSO-d₆): 10.50 (s, 1H), 9.37 (s, 1H), 8.82 (s, 1H), 8.49 (s, 1H), 8.27 (dd, 1H), 8.16 (d, 1H), 8.10 (s, 1H), 8.01 (d, 1H), 7.86 (s, 1H), 7.63 (d, 1H), 7.56 (t, 1H), 7.23 (s, 1H), 4.24 (t, 2H), 4.02 (s, 3H), 3.62 (m, 1H), 3.44 (m, 1H), 2.89 (m, 2H), 2.60 (m, 1H), 2.39 (m, 1H), 2.27 (m, 1H), 1.97 (t, 2H), 1.68-1.13 (m, 6H) :
MS (+ve ESI): 577 (M+H)⁺.

### Example 13 - Preparation of Compound 13 in Table 1 - 2-[(5-{[(4-({6-[(3-chlorobenzoyl)amino]pyridin-3-yl}amino)-6-methoxyquinazolin-7-yl]oxy}pentyl)(ethyl)amino]ethyl dihydrogen phosphate

An analogous reaction to that described in example 12, but starting with 3-chloro-*N-*(5-{[7-({5-[ethyl(2-hydroxyethyl)amino]pentyl}oxy)-6-methoxyquinazolin-4-yl]amino}pyridin-2-yl)benzamide (350 mg, 0.60 mmol), yielded the title compound (as the dihydrochloride salt, 340 mg, 77 % yield) as a pale yellow solid :
¹H-NMR (DMSO-d₆) : 11.88 (br s, 1H), 11.05 (br s, 1H), 8.81 (m, 2H), 8.53 (s, 1H), 8.25 (m, 2H), 8.09 (s, 1H), 7.98 (d, 1H), 7.67 (m, 1H), 7.55 (t, 1H), 7.44 (s, 1H), 7.51 (m, 3H), 4.26 (m, 4H), 4.06 (s, 3H), 3.38 (m, 2H), 3.15 (m, 4H), 1.82 (m, 4H), 1.49 (m, 2H), 1.23 (m, 3H) :
MS (+ve ESI) : 659 (M+H)⁺.
3-chloro-*N-*(5-{[7-({5-[ethyl(2-hydroxyethyl)amino]pentyl}oxy)-6-methoxyquinazolin-4-yl]amino}pyridin-2-yl)benzamide, used as the starting material, was obtained as follows:
a) An analogous reaction to that described in example 10c but starting with bromo-5-chloropentane (280 mg, 1.50 mmol) yielded 3-chloro-*N-*[5-({7-[(5-chloropentyl)oxy]-6-methoxyquinazolin-4-yl}amino)pyridin-2-yl]benzamide (162 mg, 20 % yield) :
   ¹H-NMR (DMSO-d₆) : 10.90 (s, 1H), 9.61 (s, 1H), 8.77 (s, 1H), 8.48 (s, 1H), 8.23 (m, 2H), 8.09 (s, 1H), 7.83 (s, 1H), 7.66 (d, 1H), 7.55 (t, 1H), 7.18 (s, 1H), 4.15 (t, 2H), 3.96 (s, 3H), 3.67 (t, 2H), 1.84 (m, 4H), 1.57 (m, 2H).
b) An analogous reaction to that described in example 2d but starting with 2-(ethylamino)ethanol (93 mg, 0.95 mmol) and 3-chloro-*N-*[5-({7-[(5-chloropentyl)oxy]-6-methoxyquinazolin-4-yl}amino)pyridin-2-yl]benzamide (100 mg, 0.19 mmol) yielded 3-chloro-*N-*(5-{[7-({5-[ethyl(2-hydroxyethyl)amino]pentyl}oxy)-6-methoxyquinazolin-4-yl]amino}pyridin-2-yl)benzamide (50 mg, 45 % yield) :
   ¹H-NMR (DMSO-d₆) : 11.22 (m, 1H), 11.05 (br s, 1H), 8.82 (s, 1H), 8.70 (d, 1H), 8.27 (m, 1H), 8.14 (m, 2H), 8.09 (m, 1H), 7.98 (m, 1H), 7.66 (m, 1H), 7.54 (m, 1H), 7.40 (s, 1H), 4.20 (t, 2H), 3.99 (s, 3H), 3.71 (t, 2H), 3.17 (m, 6H), 1.84 (m, 2H), 1.72 (m, 2H), 1.45 (m, 2H), 1.20 (t, 3H) :
   MS (-ve ESI) : 577 (M-H)⁻,
   MS (+ve ESI) : 579 (M+H)⁺.

### Example 14 - Preparation of Compound 14 in Table 1 - 4-[(3-{[4-({6-[(3-chlorobenzoyl)amino]pyridin-3-yl}amino)-6-methoxyquinazolin-7-yl]oxy}propyl)(ethyl)amino]butyl dihydrogen phosphate

An analogous reaction to that described in example 12, but starting with 3-chloro-*N-*{5-[(7-{3-[ethyl(4-hydroxybutyl)amino]propoxy}-6-methoxyquinazolin-4-yl)amino]pyridin-2-yl}benzamide (1.02 g, 1.76 mmol) yielded the title compound (as the dihydrochloride salt, 600 mg, 47 % yield) as a pale yellow solid :
¹H-NMR (DMSO-d₆) : 11.90 (br s, 1H), 11.61 (br s, 1H), 8.81 (m, 2H), 8.55 (s, 1H), 8.25 (m, 2H), 8.09 (s, 1H), 7.98 (d, 1H), 7.67 (m, 1H), 7.55 (t, 1H), 7.44 (s, 1H), 4.30 (m, 2H), 4.03 (s, 3H), 3.19 (m, 6H), 2.88 (m, 2H), 2.30 (m, 2H), 1.78 (m, 2H), 1.66 (m, 2H), 1.25 (m, 3H) : MS (+ve ESI) : 659 (M+H)⁺.

3-chloro-*N-*{5-[(7-{3-[ethyl(4-hydroxybutyl)amino]propoxy}-6-methoxyquinazolin-4-yl)amino]pyridin-2-yl}benzamide, used as the starting material, was obtained as described in example 2d, but starting with 4-(ethylamino)butanol (226 ml, 1.93 mmol). The reaction yielded the desired compound as the di-trifluoroacetic acid salt (113 mg, 51 % yield) :
¹H-NMR (DMSO-d₆): 11.04 (s, 1H), 10.99 (br s, 1H), 8.81 (s, 1H), 8.70 (m, 1H), 8.28 (m, 1H), 8.16 (m, 1H), 8.08 (m, 2H), 7.98 (m, 1H), 7.67 (m, 1H), 7.55 (t, 1H), 7.38 (s, 1H), 4.30 (t, 2H), 3.99 (s, 3H), 3.42 (t, 2H), 3.20 (m, 6H), 2.22 (m, 2H), 1.70 (m, 2H), 1.48 (m, 2H), 1.22 (t, 3H) :
MS (-ve ESI) : 577 (M-H)⁻,
MS (+ve ESI): 579 (M+H)⁺.

### Example 15 - Preparation of Compound 15 in Table 1 - 2-[(3-{[4-({6-[(3-fluorobenzoyl)amino]pyridin-3-yl}amino)-6-methoxyquinazolin-7-yl]oxy}propyl(methyl)amino]ethyl dihydrogen phosphate

An analogous reaction to that described in example 7, but starting with 3-fluoro-*N-*{5-[(7-{3-[(2-hydroxyethyl)(methyl)amino]propoxy}-6-methoxyquinazolin-4-yl)amino]pyridin-2-yl}benzamide (1.10 g, 2.11 mmol) yielded the title compound (as the formate salt, 180 mg, 13 % yield) as a pale yellow solid :
¹H-NMR (DMSO-d₆) : 12.15 (br s, 1H), 10.99 (br s, 1H), 8.81 (m, 2H), 8.64 (s, 1H), 8.25 (m, 1H), 7.88 (m, 2H), 7.51 (m, 3H), 4.26 (m, 4H), 4.06 (s, 3H), 3.43 (m, 2H), 3.34 (m, 2H), 2.86 (s, 3H), 2.34 (m, 2H) :
MS (+ve ESI) : 601 (M+H)⁺.
3-fluoro-*N-*{5-[(7-{3-[(2-hydroxyethyl)(methyl)amino]propoxy}-6-methoxyquinazolin-4-yl)amino]pyridin-2-yl}benzamide, used as the starting material, was obtained as follows:
a) An analogous reaction to that described in example 7d, but starting with 2-(methylamino)ethanol (780 µl, 9.67 mmol) yielded 3-fluoro-*N-*{5-[(7-{3-[(2-hydroxyethyl)(methyl)amino]propoxy}-6-methoxyquinazolin-4-yl)amino]pyridin-2-yl}benzamide (1.2 g, 72 % yield) :
   ¹H-NMR (DMSO-d₆): 10.85 (m, 1H), 9.61 (s, 1H), 8.78 (m, 1H), 8.45 (s, 1H), 8.28 (m, 1H), 8.19 (m, 1H), 7.87 (m, 3H), 7.55 (m, 1H), 7.43 (m, 1H), 7.18 (s, 1H), 4.43 (m, 1H), 4.16 (t, 2H), 3.98 (s, 3H), 3.44 (m, 2H), 2.50 (m, 2H), 2.41 (t, 2H), 2.20 (s, 3H), 1.89 (m, 2H) :
   MS (-ve ESI) : 519 (M-H)⁻,
   MS (+ve ESI): 521 (M+H)⁺.

### Example 16 - Preparation of Compound 16 in Table 1- 2-[(3-{[4-({6-[(3-chlorobenzoyl)amino]pyridin-3-yl}amino)-6-methoxyquinazolin-7-yl]oxy}propyl)(isobutyl)amino]ethyl dihydrogen phosphate

An analogous reaction to that described in example 12, but starting with 3-chloro-N-{5-[(7-{3-[(2-hydroxyethyl)(isobutyl)amino]propoxy}-6-methoxyquinazolin-4-yl)amino]pyridin-2-yl}benzamide (60 mg, 0.10 mmol), initially yielded di-*tert*-butyl 2-[(3-{[4-({6-[(3-chlorobenzoyl)amino]pyridin-3-yl}amino)-6-methoxyquinazolin-7-yl]oxy}propyl)(isobutyl)amino]ethyl phosphate (34 mg, 42% yield) :
¹H-NMR (DMSO-d₆) : 10.92 (s, 1H), 9.60 (s, 1H), 8.80 (d, 1H), 8.45 (s, 1H), 8.25 (dd, 1H), 8.20 (d, 1H), 8.05-8.02 (m, 1H), 8.0 (d, 1H), 7.80 (s, 1H), 7.65-7.60 (m, 1H), 7.55-7.50 (m, 1H), 7.20 (s, 1H), 4.20 (t, 2H), 4.0 (s, 3H), 3.85-3.80 (m, 2H), 2.65-2.60 (m, 4H), 2.20 (d, 2H), 1.95-1.80 (m, 2H), 1.40 (s, 18H), 0.85-0.80 (m, 7H) :
MS (-ve ESI) : 769 (M-H)⁻,
MS (+ve ESI) : 771 (M+H)⁺.
di-*tert*-butyl 2-[(3-{[4-({6-[(3-chlorobenzoyl)amino]pyridin-3-yl}amino)-6-methoxyquinazolin-7-yl]oxy}propyl)(isobutyl)amino]ethyl phosphate was subjected to deprotection with hydrochloric acid (according to the method described in example 12 to yield the title compound (as the di-hydrochloride salt, 30 mg, 93 % yield) as a pale yellow solid :
¹H-NMR (DMSO-d₆): 12.00 (s, 1H), 11.00 (s, 1H), 8.90 (s, 1H), 8.80 (s, 1H), 8.60 (s, 1H), 8.30-8.40 (m, 2H), 8.10 (s, 1H), 8.01 (d, 1H), 7.65 (d, 1H), 7.47 (m, 1H), 7.40 (s, 1H), 4.30-4.40 (m, 2H), 4.00 (s, 3H), 3.50-3.60 (m, 2H), 3.30-3.40 (m, 2H), 3.10 (d, 2H), 2.30-2.40 (m, 2H), 2.10-2.20 (m, 1H), 1.10 (s, 3H), 1.00 (s, 3H) :
MS (-ve ESI) : 657 (M-H)⁻,
MS (+ve ESI) : 659 (M+H)⁺.
3-chloro-*N-*{5-[(7-{3-[(2-hydroxyethyl)(isobutyl)amino]propoxy}-6-methoxyquinazolin-4-yl)amino]pyridin-2-yl}benzamide, used as the starting material, was obtained as follows:
a) A cooled (-60 °C) solution of ethylene oxide (5.28 g, 120 mmol) in methanol (14 ml), was added slowly to a solution of isobutylamine (30.7 g, 420 mmol) in methanol (100 ml) at -65 °C under argon. The mixture was allowed to warm to ambient temperature over 14 hours, concentrated *in vacuo* and the residual oil was purified by distillation (b.p. 130 °C / 0.5 mmHg) to yield 2-(isobutylamino)ethanol (11 g, 78 % yield) :
   ¹H-NMR (DMSO d₆) : 4.40 (m, 1H), 3.42 (m, 2H), 2.50 (m, 2H), 2.30 (d, 2H), 1.63 (m, 1H), 0.85 (d, 6H).
b) An analogous reaction to that described in example 2d, but starting with 2-(isobutylamino)ethanol (263 mg, 2.25 mmol) yielded 3-chloro-*N*-{5-[(7-{3-[(2-hydroxyethyl)(isobutyl)amino]propoxy}-6-methoxyquinazolin-4-yl)amino]pyridin-2-yl}benzamide (80 mg, 18 % yield) :
   ¹H-NMR (DMSO-d₆): 10.91 (s, 1H), 9.60 (s, 1H), 8.80 (d, 1H), 8.45 (s, 1H), 8.25 (dd, 1H), 8.20 (d, 1H), 8.04 (m, 1H), 8.00 (d, 1H), 7.80 (s, 1H), 7.62 (m, 1H), 7.53 (m, 1H), 7.20 (s, 1H), 4.25 (br s, 1H), 4.20 (t, 2H), 4.00 (s, 3H), 3.40 (t, 2H), 2.60 (t, 2H), 2.20 (d, 2H), 1.80-1.95 (m, 2H), 1.60-1.70 (m, 1H), 0.80 (s, 3H), 0.78 (s, 3H) :
   MS (-ve ESI) : 577, 579 (M-H)⁻,
   MS (+ve ESI) : 579, 581 (M+H)⁺.

### Example 17 - Preparation of Compound 17 in Table 1 - 2-[(3-{[4-({6-[(3-chlorobenzoyl)amino]pyridin-3-yl}amino)-6-methoxyquinazolin-7-yl]oxy}propyl)(cyclpropyl)amino]ethyl dihydrogen phosphate

An analogous reaction to that described in example 12, but starting with 3-chloro-N-{5-[(7-{3-[cyclopropyl(2-hydroxyethyl)amino]propoxy}-6-methoxyquinazolin-4-yl)amino]pyridin-2-yl}benzamide (130 mg, 0.23 mmol), initially yielded di-*tert*-butyl 2-[(3-{[4-({6-[(3-chlorobenzoyl)amino]pyridin-3-yl}amino)-6-methoxyquinazolin-7-yl]oxy}propyl)(cyclopropyl)amino]ethyl phosphate (170 mg, quantitative yield) :
¹H-NMR (DMSO-d₆): 11.00 (s, 1H), 10.60 (s, 1H), 8.80 (d, 1H), 8.45 (s, 1H), 8.25 (dd, 1H), 8.10 (d, 1H), 8.00 (d, 1H), 7.80 (s, 1H), 7.62 (m, 1H), 7.50-7.60 (m, 1H), 7.20 (s, 1H), 4.20 (t, 2H), 4.00 (s, 3H), 3.88 (m, 2H), 2.83 (m, 4H), 1.90-2.00 (m, 2H), 1.82 (m, 1H), 1.40 (s, 18H), 0.42 (m, 2H), 0.27 (m, 2H) :
MS (-ve ESI) : 753 (M-H)⁻,
MS (+ve ESI) : 755 (M+H)⁺.
di-*tert*-butyl 2-[(3-{[4-({6-[(3-chlorobenzoyl)amino]pyridin-3-yl}amino)-6-methoxyquinazolin-7-yl]oxy}propyl)(cyclopropyl)amino]ethyl phosphate was subjected to deprotection with hydrochloric acid (according to the method described in example 12 to yield the title compound (as the di-hydrochloride salt, 30 mg, 93 % yield) as a pale yellow solid:
¹H-NMR (DMSO-d₆) : 12.00 (s, 1H), 11.20 (s, 1H), 9.00 (s, 1H), 8.80 (s, 1H), 8.60 (s, 1H), 8.40-8.50 (m, 2H), 8.20 (s, 1H), 8.0 (d, 1H), 7.68 (m, 1H), 7.50-7.60 (m, 1H), 7.45 (s, 1H), 4.40-4.50 (m, 4H), 4.20 (s, 3H), 3.60-3.70 (m, 2H), 3.40-3.50 (m, 2H), 3.00-3.10 (m, 1H), 2.50-2.60 (m, 2H), 1.30-1.40 (m, 2H), 1.00-1.10 (m, 2H) :
MS (+ve ESI): 643, 645 (M+H)⁺.
3-chloro-*N*-{5-[(7-{3-[cyclopropyl(2-hydroxyethyl)amino]propoxy}-6-methoxyquinazolin-4-yl)amino]pyridin-2-yl}benzamide, used as the starting material, was obtained as follows:
a) A solution of triethylamine (15.6 g, 154 mmol) and cyclopropylamine (4.85 ml, 70 mmol) in diethyl ether (50 ml) was added dropwise to a stirred solution of ethyloxalyl chloride (8.59 ml, 77 mmol) in diethyl ether (75 ml) at 0 °C. The reaction was allowed to warm to ambient temperature over 1 hour, was filtered and then concentrated *in vacuo* to yield ethyl (cyclopropylamino)(oxo)acetate as a brown oil which was used without further purification (12.5 g, quantitative yield) :
   ¹H-NMR (DMSO-d₆): 8.85 (s, 1H), 4.20 (q, 2H), 2.70-2.80 (m, 1H), 1.25 (t, 3H), 0.60-0.70 (m, 2H), 0.56 (m, 2H) :
   MS (+ve ESI) : 158 (M+H)⁺.
b) Chloromethylsilane (68 ml, 539 mmol) was added to a stirred solution of lithium borohydride in tetrahydrofuran (135 ml of a 2.0 N solution, 270 mmol) at ambient temperature. The reaction was cooled to 0 °C and a solution of ethyl (cyclopropylamino)(oxo)acetate (12.5 g from previous experiment, assumed 70 mmol) in tetrahydrofuran (100 ml) was added over 5 minutes before the reaction as allowed to warm to ambient temperature over 2 hours. Methanol (10 ml) was added, the reaction was allowed to stand overnight and the volatiles were removed *in vacuo.* The residue was stirred with 1.0 N aqueous sodium hydroxide solution and extracted with dichloromethane (3 x 25 ml). Purification by flash chromatography on silica gel, eluting with 5-20% methanol in dichloromethane, yielded 2-(cyclopropylamino)ethanol (1.45 g, 20 % yield) as a colourless oil:
   ¹H-NMR (DMSO-d₆) : 3.25 (t, 2H), 2.45 (t, 2H), 1.85-2.00 (m, 1H), 0.15-0.55 (m, 2H), 0.02 (m, 2H) :
   MS (+ve ESI): 102 (M+H)⁺.
c) An analogous reaction to that described in example 2d, but starting with 2-(cyclopropylamino)ethanol (783 mg, 1.47 mmol) yielded 3-chloro-*N-*{5-[(7-{3-[cyclopropyl(2-hydroxyethyl)amino]propoxy}-6-methoxyquinazolin-4-yl)amino]pyridin-2-yl}benzamide (80 mg, 18 % yield) :
   ¹H-NMR (DMSO-d₆): 9.40 (br s, 1H), 8.50 (d, 1H), 8.20 (s, 1H), 8.00 (dd, 1H), 7.95 (d, 1H), 7.80 (s, 1H), 7.70 (d, 1H), 7.55 (s, 1H), 7.40 (dd, 1H), 7.25 (m, 1H), 6.90 (s, 1H), 4.00 (t, 1H), 3.90 (t, 2H), 3.70 (s, 3H), 3.22 (m, 2H), 2.50 (t, 2H), 2.40 (t, 2H), 1.65-1.80 (m, 2H), 1.50-1.60 (m, 2H), 0.10-0.20 (m, 2H), 0.02 (m, 2H) :
   MS (-ve ESI): 561, 563 (M-H)⁻,
   MS (+ve ESI): 563, 565 (M+H)⁺.

### Example 18 - Preparation of Compound 18 in Table 1 - [1-(3-{[4-({6-[(3-chlorobenzoyl)amino]pyridin-3-yl}amino)-6-methoxyquinazolin-7-yl]oxy}propyl)piperidin-4-yl]methyl dihydrogen phosphate

An analogous reaction to that described in example 12, but starting with 3-chloro-N-{5-[(7-{3-[4-(hydroxymethyl)piperidin-1-yl]propoxy}-6-methoxyquinazolin-4-yl)amino]pyridin-2-yl}benzamide (1.02 g, 1.77 mmol), initially yielded di-*tert*-butyl [1-(3-{[4-({6-[(3-chlorobenzoyl)amino]pyridin-3-yl}amino)-6-methoxyquinazolin-7-yl]oxy}propyl)piperidin-4-yl]methyl phosphate (881 mg, 64 % yield) as a pale yellow solid :
¹H-NMR (DMSO-d₆): 11.0 (br s, 1H), 9.60 (s, 1H), 8.85 (s, 1H), 8.50 (s, 1H), 8.30 (dd, 1H), 8.20 (d, 1H), 8.10 (s, 1H), 8.05 (d, 1H), 7.90 (s, 1H), 7.70 (d, 1H), 7.62 (m, 1H), 7.20 (s, 1H), 4.20 (t, 2H), 4.00 (s, 3H), 3.70 (t, 2H), 2.90-3.00 (m, 2H), 2.40-2.50 (m, 2H), 1.90-2.10 (m, 4H), 1.60-1.70 (m, 3H), 1.40 (s, 18H), 1.32 (m, 2H) :
MS (+ve ESI) : 767, 769 (M+H)⁺.
di-*tert*-butyl [1-(3-{[4-({6-[(3-chlorobenzoyl)amino]pyridin-3-yl}amino)-6-methoxyquinazolin-7-yl]oxy}propyl)piperidin-4-yl]methyl phosphate (771 mg, 1.00 mmol) was subjected to deprotection with hydrochloric acid (according to the method described in example 12 to yield the title compound (as the di-hydrochloride salt, 705 mg, 96 % yield) as a pale yellow solid:
¹H-NMR (DMSO-d₆) : 12.00 (s, 1H), 11.10 (s, 1H), 10.50 (s, 1H), 8.90 (s, 1H), 8.85 (s, 1H), 8.70 (s, 1H), 8.30-8.40 (m, 2H), 8.15 (s, 1H), 8.05 (d, 1H), 7.70 (d, 1H), 7.57 (m, 1H), 7.50 (s, 1H), 4.30-4.40 (m, 2H), 4.15 (s, 3H), 3.70 (t, 2H), 3.62 (m, 2H), 3.30-3.40 (m, 2H), 2.90-3.10 (m, 2H), 2.42 (m, 2H), 1.90-2.00 (m, 3H), 1.60-1.80 (m, 2H) :
MS (-ve ESI) : 655 (M-H)⁻,
MS (+ve ESI) : 657 (M+H)⁺.
3-chloro-*N*-{5-[(7-{3-[4-(hydroxymethyl)piperidin-1-yl]propoxy}-6-methoxyquinazolin-4-yl)amino]pyridin-2-yl}benzamide, used as the starting material, was obtained as follows:
a) An analogous reaction to that described in example 2d, but starting with 4-piperidinemethanol (647 mg, 5.63 mmol) yielded 3-chloro-*N-*{5-[(7-{3-[4-(hydroxymethyl)piperidin-1-yl]propoxy}-6-methoxyquinazolin-4-yl)amino]pyridin-2-yl}benzamide (1.02 g, 94 % yield) :
   ¹H-NMR (DMSO-d₆): 10.91 (br s, 1H), 9.65 (s, 1H), 8.80 (d, 1H), 8.40 (s, 1H), 8.30 (dd, 1H), 8.20 (d, 1H), 8.05 (s, 1H), 8.00 (d, 1H), 7.85 (s, 1H), 7.60 (d, 1H), 7.50-7.60 (m, 1H), 7.20 (s, 1H), 4.20-4.40 (m, 1H), 4.20 (t, 2H), 4.00 (s, 3H), 3.20-3.40 (m, 6H), 2.00-2.20 (m, 2H), 1.65-1.80 (m, 2H), 1.20-1.60 (m, 3H) :
   MS (+ve ESI) : 577, 579 (M+H)⁺.

### Example 19 - Preparation of Compound 19 in Table 1 - 2-[4-(3-{[4-({6-[(3-chlrobenzoyl)amino]pyridin-3-yl}amino)-6-methoxyquinazolin-7-yl]oxy}propyl)piperazin-1-yl]ethyl dihydrogen phosphate

An analogous reaction to that described in example 12, but starting with 3-chloro-N-{5-[(7-{3-[4-(hydroxymethyl)piperidin-1-yl]propoxy}-6-methoxyquinazolin-4-yl)amino]pyridin-2-yl}benzamide (842 mg, 1.42 mmol), initially yielded di-*tert*-butyl 2-[4-(3-{[4-({6-[(3-chlorobenzoyl)amino]pyridin-3-yl}amino)-6-methoxyquinazolin-7-yl]oxy}propyl)piperazin-1-yl]ethyl phosphate (609 mg, 55 % yield) as a pale yellow solid :
¹H-NMR (DMSO-d₆): 10.80 (s, 1H), 9.70 (s, 1H), 8.80 (s, 1H), 8.50 (s, 1H), 8.40 (dd, 1H), 8.20 (d, 1H), 8.10 (s, 1H), 8.00 (d, 1H), 7.90 (s, 1H), 7.70 (d, 1H), 7.50-7.60 (m, 1H), 7.20 (s, 1H), 4.20 (t, 2H), 4.00 (s, 3H), 3.90-4.00 (m, 2H), 2.60-2.70 (m, 2H), 2.30-2.50 (m, 10H), 2.00-2.10 (m, 2H), 1.40 (s, 18H) :
MS (-ve ESI): 782 (M-H)⁻,
MS (+ve ESI): 784 (M+H)⁺.
di-*tert*-butyl 2-[4-(3-{[4-({6-[(3-chlorobenzoyl)amino]pyridin-3-yl}amino)-6-methoxyquinazolin-7-yl]oxy}propyl)piperazin-1-yl]ethyl phosphate (609 mg, 0.78 mmol) was subjected to deprotection with hydrochloric acid (according to the method described in example 12 to yield the title compound (as the di-hydrochloride salt, 587 mg, 96 % yield) as a pale yellow solid :
¹H-NMR (DMSO-d₆): 12.00 (s, 1H), 11.20 (s, 1H), 8.90 (s, 1H), 8.80 (s, 1H), 8.60 (s, 1H), 8.30-8.40 (m, 2H), 8.20 (s, 1H), 8.00 (d, 1H), 7.70 (d, 1H), 7.50-7.60 (m, 1H), 7.40 (s, 1H), 4.20-4.30 (m, 2H), 4.10 (s, 3H), 3.50-3.90 (m, 6H), 3.30-3.40 (m, 2H), 3.23 (m, 2H), 3.10-3.20 (m, 2H), 2.30-2.40 (m, 2H) :
MS (-ve ESI) : 670 (M-H)⁻,
MS (+ve ESI) : 672 (M+H)⁺.
3-chloro-*N*-{5-[(7-{3-[4-(2-hydroxyethyl)piperazin-1-yl]propoxy}-6-methoxyquinazolin-4-yl)amino]pyridin-2-yl}benzamide, used as the starting material, was obtained as follows:
a) An analogous reaction to that described in example 2d, but starting with 4-hydroxyethyl piperazine (585 mg, 4.50 mmol) yielded 3-chloro-*N*-{5-[(7-{3-[4-(2-hydroxyethyl)piperazin-1-yl]propoxy}-6-methoxyquinazolin-4-yl)amino]pyridin-2-yl}benzamide (842 mg, 94 % yield) :
   ¹H-NMR (DMSO-d₆) : 10.90 (s, 1H), 9.60 (s, 1H), 8.80 (d, 1H), 8.45 (s, 1H), 8.25 (dd, 1H), 8.20 (d, 1H), 8.04 (m, 1H), 7.98 (d, 1H), 7.85 (s, 1H), 7.65 (d, 1H), 7.50-7.60 (m, 1H), 7.20 (s, 1H), 4.20 (t, 2H), 4.00 (s, 3H), 3.50-3.60 (m, 2H), 3.20 (s, 1H), 2.60-3.00 (m, 10H), 1.90-2.10 (m, 2H) :
   MS (+ve ESI) : 590, 592 (M+H)⁺.

### Example 20 - Preparation of Compound 20 in Table 1 - [(2S)-1-(3-{[4-({6-[(3-chlorobenzoyl)amino]pyridin-3-yl}amino)-6-methoxyquinazolin-7-yl]oxy}propyl)pyrrolidin-2-yl]methyl dihydrogen phosphate

An analogous reaction to that described in example 12, but starting with 3-chloro-N-{5-[(7-{3-[(2*S*)-2-(hydroxymethyl)pyrrolidin-1-yl]propoxy}-6-methoxyquinazolin-4-yl)amino]pyridin-2-yl}benzamide (670 mg, 1.19 mmol), initially yielded di-*tert*-butyl [(2*S*)-1-(3-{[4-({6-[(3-chlorobenzoyl)amino]pyridin-3-yl}amino)-6-methoxyquniazolin-7-yl]oxy}propyl)pyrrolidin-2-yl]methyl phosphate (512 mg, 57 % yield) as a pale yellow solid :
¹H-NMR (DMSO d₆): 10.90 (s, 1H), 9.60 (s, 1H), 8.79 (s, 1H), 8.45 (s, 1H), 8.25 (d, 1H), 8.20 (d, 1H), 8.09 (s, 1H), 8.00 (d, 1H), 7.85 (s, 1H), 7.67 (d, 1H), 7.46 (t, 1H), 7.19 (s, 1H), 4.21 (m, 2H), 3.98 (s, 3H), 3.79 (m, 1H), 3.58 (m, 1H), 3.09 (m, 1H), 2.95 (m, 1H), 2.69 (m, 1H), 2.22 (m, 1H), 1.95 (m, 2H), 1.87 (m, 1H), 1.71 (m, 2H), 1.61 (m, 1H), 1.38 (s, 9H) :
MS (+ve ESI) : 755 (M+H)⁺.
di-*tert*-butyl [(2*S*)-1-(3-{[4-({6-[(3-chlorobenzoyl)amino]pyridin-3-yl}amino)-6-methoxyquinazolin-7-yl]oxy}propyl)pyrrolidin-2-yl]methyl phosphate (512 mg, 0.68 mmol) was subjected to deprotection with hydrochloric acid (according to the method described in example 12 to yield the title compound (as the di-hydrochloride salt, 393 mg, 90 % yield) as a pale yellow solid:
¹H-NMR (DMSO d₆): 11.95 (s, 1H), 11.07 (s, 1H), 8.85 (s, 1H), 8.79 (s, 1H), 8.58 (s, 1H), 8.24 (m, 2H), 8.09 (s, 1H), 8.00 (d, 1H), 7.67 (d, 1H), 7.55 (t, 1H), 7.47 (s, 1H), 4.31 (m, 2H), 4.23 (m, 2H), 4.03 (s, 3H), 3.79 (m, 1H), 3.69 (m, 1H), 3.59 (m, 1H), 3.30 (m, 1H), 3.20 (m, 1H), 2.35 (m, 2H), 2.19 (m, 1H), 2.05 (m, 1H), 1.95 (m, 1H), 1.81 (m, 1H):
MS (+ve ESI) : 643 (M+H)⁺.
3-chloro-*N*-{5-[(7-{3-[(2*S*)-2-(hydroxymethyl)pyrrolidin-1-yl]propoxy}-6-methoxyquinazolin-4-yl)amino]pyridin-2-yl}benzamide, used as the starting material, was obtained as follows:
a) An analogous reaction to that described in example 2d, but starting with L-prolinol (606 mg, 6.00 mmol) yielded 3-chloro-*N*-{5-[(7-{3-[(2*S*)-2-(hydroxymethyl)pyrrolidin-1-yl]propoxy}-6-methoxyquinazolin-4-yl)amino]pyridin-2-yl}benzamide (694 mg, 62 % yield) as an off-white solid :
   ¹H-NMR (DMSO d₆): 10.10 (br s, 1H), 8.77 (s, 1H), 8.45 (s, 1H), 8.24 (d, 1H), 8.18 (d, 1H), 8.08 (s, 1H), 8.00 (d, 1H), 7.82 (s, 1H), 7.65 (d, 1H), 7.55 (t, 1H), 7.18 (s, 1H), 4.27 (m, 1H), 4.18 (t, 2H), 3.96 (s, 3H), 3.38 (dd, 1H), 3.18 (m, 1H), 3.07 (m, 1H), 2.95 (m, 1H), 2.41 (m, 2H), 2.13 (dd, 1H), 1.93 (m, 2H), 1.79 (m, 1H), 1.62 (m, 2H), 1.51 (m, 1H) :
   MS (+ve ESI) : 563 (M+H)⁺.

### Example 21 - Preparation of Compound 21 in Table 1 - 2-[(3-{[4-({6-[(3-chlorobenzoyl)amino]pyridin-3-yl}amino)-6-methoxyquinazolin-7-yl]oxy}propyl)(cyclobutyl)amino]ethyl dihydrogen phosphate

An analogous reaction to that described in example 12, but starting with 3-chloro-*N-*{5-[(7-{3-[cyclobutyl(2-hydroxyethyl)amino]propoxy}-6-methoxyquinazolin-4-yl)amino]pyridin-2-yl}benzamide (545 mg, 0.95 mmol), initially yielded di-*tert*-butyl 2-[(3-{[4-({6-[(3-chlorobenzoyl)amino]pyridin-3-yl}amino)-6-methoxyquinazolin-7-yl]oxy}propyl)(cyclobutyl)amino]ethyl phosphate (210 mg, 29 % yield) as a yellow solid :
1H-NMR (DMSO-d₆) : 11.00 (s, 1H), 9.70 (s, 1H), 8.80 (s, 1H), 8.50 (s, 1H), 8.40 (d, 1H), 8.20 (d, 1H), 8.10 (s, 1H), 8.05 (d, 1H), 7.90 (s, 1H), 7.70 (d, 1H), 7.50-7.60 (m, 1H), 7.20 (s, 1H), 4.20 (t, 2H), 4.00 (s, 3H), 3.80-3.90 (m, 2H), 2.60-2.70 (m, 4H), 1.90-2.00 (m, 4H), 1.70-1.80 (m, 2H), 1.50-1.60 (m, 2H), 1.40 (s, 18H):
MS (-ve ESI) : 767, 769 (M-H)⁻,
MS (+ve ESI) : 769, 771 (M+H)⁺.
di-*tert*-butyl 2-[(3-{[4-({6-[(3-chlorobenzoyl)amino]pyridin-3-yl}amino)-6-methoxyquinazolin-7-yl]oxy}propyl)(cyclobutyl)amino]ethyl phosphate (609 mg, 0.78 mmol) was subjected to deprotection with hydrochloric acid (according to the method described in example 12 to yield the title compound (as the di-hydrochloride salt, 180 mg, 90 % yield) as a pale yellow solid:
¹H-NMR (DMSO-d₆): 12.00 (s, 1H), 11.20 (s, 1H), 8.90 (s, 1H), 8.85 (s, 1H), 8.70 (s, 1H), 8.30-8.40 (m, 2H), 8.25 (s, 1H), 8.00 (d, 1H), 7.70 (m, 1H), 7.55-7.65 (m, 1H), 7.50 (s, 1H), 4.38 (m, 2H), 4.30 (m, 2H), 4.10 (s, 3H), 3.90-4.00 (m, 1H), 3.30-3.40 (m, 2H), 3.23 (m, 2H), 2.40-2.50 (m,2H), 2.20-2.35 (m, 4H), 1.80-1.95 (m, 2H) :
MS (-ve ESI) : 655 (M-H)⁻,
MS (+ve ESI) : 657 (M+H)⁺.
3-chloro-*N*-{5-[(7-{3-[cyclobutyl(2-hydroxyethyl)amino]propoxy}-6-methoxyquinazolin-4-yl)amino]pyridin-2-yl}benzamide, used as the starting material, was obtained as follows:
a) An analogous reaction to that described in example 17a, but starting with cyclobutylamine (3.50 g, 49.3 mmol) yielded ethyl (cyclobutylamino)(oxo)acetate (assumed 8.43 g, quantitative yield) :
   ¹H-NMR (CDCl₃) : 7.10 (s, 1H), 4.30-4.40 (m, 2H), 3.10-3.20 (m, 1H), 2.40-2.50 (m, 2H), 1.90-2.00 (m, 2H), 1.70-1.80 (m, 2H), 1.30-1.40 (m, 3H) :
   MS (+ve ESI) : 172 (M+H)⁺.
b) A solution of ethyl (cyclobutylamino)(oxo)acetate (8.43 g, 49.3 mmol) in tetrahydrofuran (100 ml) was added dropwise to a solution of lithium aluminium hydride in tetrahydrofuran (100 ml of a 1.0 N solution, 100 mmol) at ambient temperature. The reaction was heated at reflux for 10 hours, cooled, diluted with tetrahydrofuran (200 ml) and quenched by addition of water and 1.0 N aquoeous sodium hydroxide solution. The reaction was filtered and the filtrate evaporated *in vacuo* to yield 2-(cyclobutylamino)ethanol (5.50 g, 97 % yield) as a colourless oil :
   ¹H-NMR (DMSO-d₆) : 3.70-3.80 (m, 1H), 3.50-3.60 (m, 2H), 3.20-3.30 (m, 1H), 2.60-2.70 (m, 2H), 2.30-2.40 (m, 2H), 1.60-1.70 (m, 4H).
c) An analogous reaction to that described in example 2d, but starting with 2-(cyclobutylamino)ethanol (518 mg, 4.50 mmol) yielded 3-chloro-*N*-{5-[(7-{3-[cyclobutyl(2-hydroxyethyl)amino]propoxy}-6-methoxyquinazolin-4-yl)amino]pyridin-2-yl}benzamide (558 mg, 65 % yield) :
   ¹H-NMR (DMSO-d₆) : 11.00 (s, 1H), 9.70 (s, 1H), 8.80 (s, 1H), 8.60 (s, 1H), 8.30 (dd, 1H), 8.20 (d, 1H), 8.10 (s, 1H), 8.00 (d, 1H), 7.80 (s, 1H), 7.60 (d, 1H), 7.40-7.50 (m, 1H), 7.20 (s, 1H), 4.40 (br s, 1H), 4.20 (t, 2H), 4.00 (s, 3H), 3.30-3.40 (m, 2H), 3.10-3.20 (m, 1H), 2.60-2.70 (m, 2H), 1.90-2.10 (m, 4H), 1.70-1.80 (m, 2H), 1.40-1.50 (m, 2H):
   MS (-ve ESI) : 575 (M-H)⁻,
   MS (+ve ESI) : 577 (M+H)⁺.

### Example 22 - Preparation of Compound 22 in Table 1 - 2-[(3-{[4-({6-[(3-chlorobenzoyl)amino]pyridin-3-yl}amino)-6-methoxyquinazolin-7-yl]oxy}propyl)(prop-2-yn-1-yl)amino]ethyl dihydrogen phosphate

An analogous reaction to that described in example 12, but starting with 3-chloro-*N-*{5-[(7-{3-[(2-hydroxyethyl)(prop-2-yn-1-yl)amino]propoxy}-6-methoxyquinazolin-4-yl)amino]pyridin-2-yl}benzamide (795 mg, 1.42 mmol), initially yielded di-*tert*-butyl 2-[(3-{[4-({6-[(3-chlorobenzoyl)amino]pyridin-3-yl}amino)-6-methoxyquinazolin-7-yl]oxy}propyl)(prop-2-yn-1-yl)amino]ethyl phosphate (452 mg, 42 % yield) as a yellow gum:
¹H-NMR (DMSO d₆) : 10.92 (s, 1H), 9.61 (s, 1H), 8.80 (s, 1H), 8.47 (s, 1H), 8.28 (d, 1H), 8.21 (d, 1H), 8.10 (s, 1H), 8.01 (d, 1H), 7.85 (s, 1H), 7.68 (d, 1H), 7.58 (t, 1H), 7.19 (s, 1H), 4.20 (t, 2H), 3.99 (s, 3H), 3.92 (dd, 2H), 3.48 (s, 2H), 3.12 (s, 1H), 2.75 (m, 2H), 2.69 (m, 2H), 1.95 (m, 2H), 1.39 (s, 18H) :
MS (+ve ESI) : 753 (M+H)⁺.
di-*tert*-butyl 2-[(3-{[4-({6-[(3-chlorobenzoyl)amino]pyridin-3-yl}amino)-6-methoxyquinazolin-7-yl]oxy}propyl)(prop-2-yn-1-yl)amino]ethyl phosphate (445 mg, 0.59 mmol) was subjected to deprotection with hydrochloric acid (according to the method described in example 12 to yield the title compound (as the di-hydrochloride salt, 403 mg, 95 % yield) as a pale yellow solid :
¹H-NMR (DMSO d₆) : 12.07 (s, 1H), 11.10 (s, 1H), 8.88 (s, 1H), 8.81 (s, 1H), 8.65 (s, 1H), 8.28 (s, 2H), 8.10 (s, 1H), 8.02 (d, 1H), 7.68 (d, 1H), 7.57 (t, 1H), 7.49 (s, 1H), 4.30 (m, 6H), 4.08 (s, 3H), 3.89 (s, 1H), 3.50 (m, 2H), 3.42 (m, 2H), 2.35 (m, 2H) :
MS (+ve ESI) : 641 (M+H)⁺.
3-chloro-*N*-{5-[(7-{3-[(2-hydroxyethyl)(prop-2-yn-1-yl)amino]propoxy}-6-methoxyquinazolin-4-yl)amino]pyridin-2-yl}benzamide, used as the starting material, was obtained as follows:
a) An analogous reaction to that described in example 17a, but starting with propargylamine (2.75 g, 50 mmol) yielded ethyl oxo(prop-2-yn-1-ylamino)acetate (assumed 7.75 g, quantitative yield) :
   ¹H-NMR (CDCl₃): 7.37 (br s, 1H), 4.35 (dd, 2H), 4.15 (m, 2H), 2.31 (m, 1H), 1.39 (t, 3H) : MS (+ve ESI) : 156 (M+H)⁺.
b) An analogous reaction to that described in example 21b, but starting with ethyl oxo(prop-2-yn-1-ylamino)acetate (7.75 g, 50 mmol) yielded 2-(prop-2-yn-1-ylamino)ethanol (3.79 g, 77 % yield) as a pale brown oil:
   ¹H-NMR (CDCl₃): 3.68 (m, 2H), 3.45 (s, 2H), 2.87 (m, 2H), 2.25 (s, 1H).
c) An analogous reaction to that described in example 2d, but starting with 2-(prop-2-yn-1-ylamino)ethanol (540 mg, 5.45 mmol) yielded 3-chloro-*N*-{5-[(7-{3-[(2-hydroxyethyl)(prop-2-yn-1-yl)amino]propoxy}-6-methoxyquinazolin-4-yl)amino]pyridin-2-yl}benzamide (945 mg, 85 % yield) as an off-white solid :
   ¹H-NMR (DMSO d₆) : 10.30 (br s, 1H), 8.79 (s, 1H), 8.49 (s, 1H), 8.27 (d, 1H), 8.20 (d, 1H), 8.10 (s, 1H), 8.00 (d,1H), 7.83 (s, 1H), 7.65 (d, 1H), 7.53 (t, 1H), 7.18 (s, 1H), 4.40 (br s, 1H), 4.19 (m, 2H), 3.98 (s, 3H), 3.48 (m, 2H), 3.43 (s, 2H), 3.06 (s, 1H), 2.65 (m, 2H), 2.52 (m, 2H), 1.92 (m, 2H) :
   MS (+ve ESI) : 561 (M+H)⁺.

### Example 23 - Reparation of Compound 23 in Table 2 - 2-[(3-{[4-({2-[(3-chloro-4-fluorobenzoyl)amino]pyrimidin-5-yl}amino)-6-methoxyquinazolin-7-yl]oxy}propyl)(cyclohexy)amino]ethyl dihydrogen phosphate

Dry 1*H*-tetrazole (113 mg, 1.62 mmol) and di-*tert*-butyl diethylphosphoramidite (201 mg, 0.81 mmol) were added to a solution of 3-chloro-*N*-{5-[(7-{3-[cyclohexyl(2-hydroxyethyl)amino]propoxy}-6-methoxyquinazolin-4-yl)amino]pyrimidin-2-yl}-4-fluorobenzamide (126 mg, 0.57 mmol) in dimethylformamide (2 ml). The reaction was stirred under an inert atmosphere at ambient temperature for 20 hours. The reaction mixture was diluted with ethyl acetate (50 ml) and washed with three portions of aqueous sodium hydrogen carbonate. The organics were dried over magnesium sulphate and concentrated to an oil. The oil was dissolved in tetrahydrofuran (3 ml) and cooled to 0 °C. Hydrogen peroxide (69 µl of a 30 % w/v aqueous solution) was added and the reaction stirred for 48 hours at ambient temperature during which a further portion of hydrogen peroxide (35 µl) was added. The reaction was quenched with aqueous sodium metabisulphite, extracted into dichloromethane and dried over sodium sulphate. Purification by flash chromatography on alumina, eluting with 0-2 % methanol in dichloromethane, yielded a yellow solid after trituration with diethyl ether. The yellow solid (56 mg) was dissolved in 1,4 dioxane (2 ml) and hydrogen chloride (0.10 ml of a 4.0 N solution in 1,4-dioxane, 0.41 mmol) was added dropwise to the solution. The reaction mixture was stirred for 20 hours at ambient temperature. The reaction mixture was diluted with diethyl ether and the solid was collected by suction filtration and washed with diethyl ether to yield the title compound (as a solid dihydrochloride salt, 49 mg, 11 % yield) : ¹H-NMR (DMSO-d₆): 12.10 (s, 1H), 9.20 (s, 2H), 8.85 (s, 1H), 8.60 (s, 1H), 8.20 (dd, 1H), 7.95-8.05 (m, 1H), 7.50-7.60 (m, 2H), 4.40 (t, 2H), 4.20-4.30 (m, 2H), 4.00 (s, 3H), 3.20-3.45 (m, 4H), 2.20-2.40 (m, 2H), 2.00-2.10 (m, 2H), 1.75-1.90 (m, 2H), 1.55-1.65 (m, 1H), 1.25-1.50 (m, 4H), 1.10-1.20 (m, 1H) :
³¹P-NMR {¹H} (DMSO- d₆): 0.55 (s, 1P) :
MS (+ve ESI): 704.5/706.5 (M+H)⁺.
3-chloro-*N*-{5-[(7-{3-[cyclohexyl(2-hydroxyethyl)amino]propoxy}-6-methoxyquinazolin-4-yl)amino]pyrimidin-2-yl}-4-fluorobenzamide used as starting material was obtained as follows:
a) 3-Chloro-4-fluorobenzoyl chloride (1.52 g, 7.88 mmol) was added to a stirred solution of 2-amino-5-nitropyrimidine (1.00 g, 7.14 mmol) in anhydrous pyridine (20 ml) at ambient temperature and the reaction heated at reflux for 7 hours. The reaction was cooled to ambient temperature, poured into water (200 ml) and allowed to stand for 18 hours. The aqueous phase was extracted with dichloromethane (3 x 75 ml) and the combined organic extracts were washed with brine (2 x 100 ml) and dried (magnesium sulphate). Solvent evaporation *in vacuo* followed by purification by flash chromatography on silica gel, eluting with dichloromethane : methanol (99:1 then 98:2) yielded 3-chloro-4-fluoro-*N*-(5-nitropyrimidin-2-yl)benzamide (1.71 g, 81 % yield) as a brown gum :
   ¹H-NMR (DMSO-d₆) : 11.86 (br s, 1H), 9.44 (s, 2H), 8.20 (m, 1H), 7.98 (m, 1H), 7.58 (t, 1H):
   MS (-ve ESI) : 295 (M-H)⁻,
   MS (+ve ESI) : 297 (M+H)⁺.
b) 10 % Platinum on carbon (149 mg) was added to a stirred suspension of 3-chloro-4-fluoro-*N*-(5-nitropyrimidin-2-yl)benzamide (743 mg, 2.51 mmol) in ethanol (50 ml) at ambient temperature and the reaction stirred for 18 hours under an atmosphere of hydrogen. The reaction was filtered through a pad of celite and the solvent evaporated *in vacuo* to yield a solid which was taken up in ethyl acetate : ethanol (4:1) and filtered (0.45µM PTFE filter). The solvent was evaporated to yield *N*-(5-aminopyrimidin-2-yl)-3-chloro-4-fluorobenzamide (5.77 g, 99 % yield) as a brown solid :
   ¹H-NMR (DMSO-d₆) : 10.64 (br s, 1H), 8.15 (m, 1H), 8.07 (s, 2H), 7.94 (m, 1H), 7.54 (t, 1H), 5.41 (s, 2H) :
   MS (-ve ESI) : 265 (M-H)⁻,
   MS (+ve ESI) : 267 (M+H)⁺.
c) Hydrogen chloride (3.0 ml of a 4.0 N solution in dioxane, 12.0 mmol) was added to a stirred solution of 7-(benzyloxy)-4-chloro-6-methoxyquinazoline (3.40 g, 11.25 mmol) and N-(5-aminopyrimidin-2-yl)-3-chloro-4-fluorobenzamide (3.00 g, 11.25 mmol) in dimethylacetamide (50 ml) and the reaction heated at 50 °C for 3.5 hours. The reaction was allowed to cool to ambient temperature and the precipitated solid was collected by suction filtration. Washing of the solid with diethyl ether followed by drying *in vacuo* yielded *N*-(5-{[7-(benzyloxy)-6-methoxyquinazolin-4-yl]amino}pyrimidin-2-yl)-3-chloro-4-fluorobenzamide (5.33 g, 79 % yield) as a cream solid :
   ¹H-NMR (DMSO-d₆) : 11.80 (br s, 1H), 11.27 (br s, 1H), 9.11 (s, 2H), 8.85 (s, 1H), 8.42 (s, 1H), 8.10 (m, 1H), 8.00 (m, 1H), 7.37-7.60 (m, 7H), 5.34 (s, 2H), 4.03 (s, 3H):
   MS (-ve ESI) : 529 (M-H)⁻,
   MS (+ve ESI) : 531 (M+H)⁺.
d) *N*-(5-{[7-(benzyloxy)-6-methoxyquinazolin-4-yl]amino}pyrimidin-2-yl)-3-chloro-4-fluorobenzamide (5.33 g, 8.83 mmol) was added to trifluoroacetic acid (50 ml) and the reaction heated at reflux for 2 hours. The reaction was cooled to ambient teperature, the volatiles were evaporated *in vacuo* and the resultant brown oil was azeotroped with dichloromethane (2 x 50 ml). Addition of diethyl ether (50 ml) caused the formation of a precipitate which was collected by suction filtration and dried *in vacuo* to yield 3-chloro-4-fluoro-*N*-{5-[(7-hydroxy-6-methoxyquinazolin-4-yl)amino]pyrimidin-2-yl}benzamide (trifluoroacetate salt, 4.73 g, 97 % yield) as an off-white solid :
   ¹H-NMR (DMSO-d₆) : 11.27 (br s, 1H), 9.03 (s, 2H), 8.81 (s, 1H), 8.19 (s, 1H), 8.00 (m, 2H), 7.57 (t, 1H), 7.21 (s, 1H), 4.00 (s, 3H) :
   MS (-ve ESI) : 439 (M-H)⁻,
   MS (+ve ESI) : 441 (M+H)⁺.
e) A mixture of 3-chloro-4-fluoro-*N*-{5-[(7-hydroxy-6-methoxyquinazolin-4-yl)amino]pyrimidin-2-yl}benzamide (750 mg, 1.35 mmol), 1,3-dibromopropane (327 mg, 1.62 mmol) and potassium carbonate (411 mg, 2.98 mmol) in dimethylacetamide (3 ml) were stirred at ambient temperature for 20 hours before N-cyclohexyl(ethanol)amine (965 mg, 6.75 mmol) was added to the solution. The reaction mixture was stirred at ambient temperature for 48 hours then concentrated under reduced pressure. Purification by flash chromatography on silica gel, eluting with dichloromethane : methanol: saturated ammonia (93:5:2 to 78:20:2) yielded 3-chloro-*N*-{5-[(7-{3-[cyclohexyl(2-hydroxyethyl)amino]propoxy}-6-methoxyquinazolin-4-yl)amino]pyrimidin-2-yl}-4-fluorobenzamide (126 mg, 15 % yield) as a yellow solid:
   ¹H-NMR (DMSO-d₆): 11.10 (s, 1H), 9.80 (s, 1H), 9.15 (s, 2H), 8.45 (s, 1H), 8.20 (d, 2H), 7.95-8.05 (m, 1H), 7.80 (s, 1H), 7.50-7.60 (m, 1H), 7.20 (s, 1H), 4.25 (t, 2H), 4.00 (s, 3H), 3.30-3.50 (m, 4H), 2.50-2.80 (m, 4H), 1.80-2.00 (m, 2H), 1.50-1.75 (m, 4H), 1.10-1.30 (m, 5H) :
   MS (-ve ESI): 622 (M-H)⁻,
   MS (+ve ESI): 624 (M+H)⁺.

### Example 24 - Preparation of Compound 24 in Table 2 - 2-[(3-{[4-({2-[(3-chloro-4-fluorobenzoyl)amino]pyrimidin-5-yl}amino)-6-methoxyquinazolin-7-yl]oxy}propyl)(ethyl)amino]ethyl dihydrogen phosphate

Dry 1*H*-tetrazole (1.34 g, 19.21 mmol) and di-*tert*-butyl diethylphosphoramidite (2.95 g, 11.85 mmol) were added in 3 aliquots to a solution of 3-chloro-*N*-{5-[(7-{3-[ethyl(2-hydroxyethyl)amino]propoxy}-6-methoxyquinazolin-4-yl)amino]pyrimidin-2-yl}-4-fluorobenzamide (1.5 g, 2.63 mmol) in dimethylacetamide (60 ml). The reaction was stirred under an inert atmosphere at ambient temperature for 72 hours. The reaction mixture was diluted with ethyl acetate (200 ml) and washed with three portions of aqueous sodium hydrogen carbonate. The organics were dried over sodium sulphate and concentrated to an oil. The oil was dissolved in tetrahydrofuran (25 ml) and cooled to 0 °C. Hydrogen peroxide (599 µl of a 30 % w/v aqueous solution) was added and the reaction stirred for 20 hours at ambient temperature during which a further portion of hydrogen peroxide (300 µl) was added. The reaction was quenched at 0 °C with aqueous sodium metabisulphite, extracted into ethyl acetate, dichloromethane and methanol and dried over magnesium sulphate. Purification by flash chromatography on alumina, eluting with 0-5 % methanol in dichloromethane yielded a yellow foam. The yellow foam (859 mg) was dissolved in 1,4 dioxane (50 ml) and hydrogen chloride (1.7 ml of a 4.0 N solution in 1,4-dioxane, 6.78 mmol) was added dropwise to the solution. The reaction mixture was stirred for 20 hours at ambient temperature. The reaction mixture was diluted with diethyl ether (300 ml) the solid collected by suction filtration and washed with diethyl ether to yield the title compound (as a solid dihydrochloride salt, 799 mg, 42 % yield) :
¹H-NMR (DMSO-d₆): 12.30 (s, 1H), 11.20 (s, 1H), 9.20 (s, 2H), 8.90 (s, 1H), 8.20 (dd, 1H), 7.95-8.05 (m, 1H), 7.50-7.60 (m, 1H), 7.40 (s, 1H), 4.20-4.40 (t, 4H), 4.00 (s, 3H), 3.50-3.60 (m, 2H), 3.20-3.20 (m, 4H), 2.20-2.40 (m, 2H), 1.30 (t, 3H) :
³¹P-NMR {¹H} (DMSO-d₆): -0.23 (s, 1P) :
MS (+ve ESI): 650, 652 (M+H)⁺.
3-chloro-*N*-{5-[(7-{3-[ethyl(2-hydroxyethyl)amino]propoxy}-6-methoxyquinazolin-4-yl)amino]pyrimidin-2-yl}-4-fluorobenzamide used as starting material was obtained as follows:
A mixture 3-chloro-4-fluoro-*N*-{5-[(7-hydroxy-6-methoxyquinazolin-4-yl)amino]pyrimidin-2-yl}benzamide (8 g, 14.4 mmol), 1-bromo-3-chloropropane (2.49 g, 15.9 mmol) and caesium carbonate (9.38 g, 28.8 mmol) in dimethylformamide (80 ml) were stirred at ambient temperature for 20 hours before N-ethyl(ethanol)amine (6.4 g, 72 mmol) was added to the solution. The reaction mixture was stirred at 70 °C for 48 hours then concentrated under reduced pressure. Purification by flash chromatography on silica gel, eluting with 1-20 % methanol in dichloromethane containing 2 % concentrated ammonia, yielded 3-chloro-*N*-{5-[(7-{3-[ethyl(2-hydroxyethyl)amino]propoxy}-6-methoxyquinazolin-4-yl)amino]pyrimidin-2-yl}-4-fluorobenzamide (2.5 g, 30 % yield) as a yellow solid :
¹H-NMR (DMSO-d₆): 11.10 (s, 1H), 9.75 (s, 1H), 9.15 (s, 2H), 8.45 (s, 1H), 8.20 (dd, 1H), 7.95-8.05 (m, 1H), 7.80 (s, 1H), 7.50-7.60 (m, 1H), 7.20 (s, 1H), 4.25 (br s, 1H), 4.20 (t, 2H), 4.00 (s, 3H), 3.40-3.50 (m, 2H), 2.50-2.65 (m, 6H), 1.80-2.00 (m, 2H), 0.95 (t, 3H) :
MS (+ve ESI): 570, 572 (M+H)⁺,
MS (-ve ESI): 568, 570 (M-H)⁻.

### Example 25 - Preparation of Compound 25 in Table 2 - 3-{[4-({2-[(3-chlorobenzoyl)amino]pyrimidin-5-yl}amino)-6-methoxyquinazolin-7-yl]oxy}propyl dihydrogen phosphate

An analogous reaction to that described in example 9, but starting with 3-chloro-*N*-(5-{[7-(3-hydroxypropoxy)-6-methoxyquinazolin-4-yl]amino}pyrimidin-2-yl)benzamide (100 mg, 0.21 mmol) yielded the title compound as a pale yellow solid (66 mg, 56 % yield) :
¹H-NMR (DMSO-d₆): 12.10 (s, 1H), 11.26 (s, 1H), 9.13 (s, 2H), 8.89 (s, 1H), 8.54 (s, 1H), 8.00 (s, 1H), 7.92 (d, 1H), 7.68 (m, 1H), 7.54 (t, 1H), 7.41 (s, 1H), 4.28 (t, 2H), 4.03 (s, 3H), 4.02 (q, 2H), 2.15 (m, 2H):
MS (+ve ESI): 561 (M+H)⁺.

3-chloro-*N*-(5-{[7-(3-hydroxypropoxy)-6-methoxyquinazolin-4-yl]amino}pyrimidin-2-yl)benzamide used as the starting material was obtained in an analogous reaction to that described in example 23e, but starting with 3-bromopropanol (154 mg, 1.10 mmol) and 3-chloro -*N*-{5-[(7-hydroxy-6-methoxyquinazolin-4-yl)amino]pyrimidin-2-yl}benzamide (520 mg, 1 mmol - see WO 01/21597) but no amine was added. The reaction yielded the title compound as an off-white solid (151 mg, 31 % yield) :
¹H-NMR (DMSO-d₆): 11.11 (s, 1H), 9.72 (s, 1H), 9.11 (s, 2H), 8.47 (s, 1H), 8.02 (s, 1H), 7.93 (d, 1H), 7.79 (s, 1H), 7.65 (d, 1H), 7.53 (t, 1H), 7.21 (s, 1H), 4.56 (t, 1H), 4.20 (t, 2H), 3.96 (s, 3H), 3.58 (q, 2H), 1.94 (m, 2H) :
MS (+ve ESI): 481 (M+H)⁺.

### Example 26 - Preparation of Compound 26 in Table 2 - 1-[3-({4-[(2-{[(3-chloro-4-fluorophenyl)amino]methyl}pyrimidin-5-yl)amino]-6-methoxyquinazolin-7-yl}oxy)propyl]piperidin-4-yl dihydrogen phosphate

An analogous reaction to that described in example 9, but starting with 1-[3-({4-[(2-{[(3-chloro-4-fluorophenyl)amino]methyl}pyrimidin-5-yl)amino]-6-methoxyquinazolin-7-yl}oxy)propyl]piperidin-4-ol (500 mg, 0.88 mmol) yielded the title compound (121 mg, 21 % yield) :
¹H-NMR (DMSO-d₆) : 12.20 (s, 1H), 10.69 (s, 1H), 9.27 (s, 2H), 9.00 (s, 1H), 8.63 (s, 1H), 7.54 (s, 1H), 7.11 (dd, 1H), 6.80 (dd, 1H), 6.65 (m, 1H), 5.22 (br s, 5H), 4.51 (s, 1H), 4.33 (t, 2H), 4.07 (s, 3H), 3.58-3.39 (m, 2H), 3.30-3.09 (m, 4H), 2.35 (m, 2H), 2.17 (m, 2H), 2.00 (m, 2H) :
³¹P-NMR {¹H} (DMSO-d₆): -1.0 (s, 1P) :
MS (-ve ESI) : 646/648 (M-H)⁻,
MS (+ve ESI) : 648/650 (M+H)⁺.
1-[3-({4-[(2-{[(3-chloro-4-fluorophenyl)amino]methyl}pyrimidin-5-yl)amino]-6-methoxyquinazolin-7-yl}oxy)propyl]piperidin-4-ol used as starting material was obtained as follows:
a) Sodium hydride (1.20 g, 31.65 mmol) was added at ambient temperature to a solution of 2-(diethoxymethyl)pyrimidin-5-amine (1.87 g, 9.49 mmol, see-WO 01/21597) in tetrahydrofuran (60 ml). The reaction mixture was stirred for 10 minutes before the addition of 4-chloro-6-methoxy-7-(3-chloropropoxy)quinazoline (4.00 g, 13.93 mmol). The reaction mixture was heated to reflux for 3 hours, cooled to ambient temperature and diluted with ethyl acetate (80 ml). The resultant precipitate was removed by filtration through celite and the filtrate concentrated to a brown powder. This was dissolved in dichloromethane : methanol (9:1) and purified by flash chromatography on silica gel. Elution with dichloromethane : methanol (9:1), afforded 7-(3-chloropropoxy)-*N*-[2-(diethoxymethyl)pyrimidin-5-yl]-6-methoxyquinazolin-4-amine as an orange powder (5.47 g, 88 % yield, 74 % pure), used crude in the next reaction.
   MS (+ve ESI) : 448 (M+H)⁺.
b) 7-(3-Chloropropoxy)-*N*-[2-(diethoxymethyl)pyrimidin-5-yl]-6-methoxyquinazolin-4-amine (4.62 g, 10.3 mmol) was dissolved in a mixture of dichloromethane : trifluoroacetic acid : water (6:1:1, 200 ml) and stirred at ambient temperature for 7 hours. The dichloromethane was removed and the residue suspended in diethyl ether (500 ml), filtered and dried in a vacuum oven for 24 hours to afford 5-{[7-(3-chloropropoxy)-6-methoxyquinazolin-4-yl]amino}pyrimidine-2-carbaldehyde as the trifluoroacetic acid salt (4.64 g, 92 % yield), used crude in the next reaction.
c) Sodium cyanoborohydride (386 mg, 6.15 mmol) and acetic acid (586 µl, 10.3 mmol) were added to a solution of 5-{[7-(3-chloropropoxy)-6-methoxyquinazolin-4-yl]amino}pyrimidine-2-carbaldehyde (2.50 g, 5.12 mmol) and 4-fluoro-3-chloroaniline (3.73 g, 25.6 mmol) in methanol (25 ml) under nitrogen. The reaction was stirred at ambient temperature for 2 hours, diluted with diethyl ether (180 ml) and the resultant solid collected by filtration. The solid was washed with diethyl ether and dried in a vacuum oven to give N-(2-{[(3-chloro-4-fluorophenyl)amino]methyl}pyrimidin-5-yl)-7-(3-chloropropoxy)-6-methoxyquinazolin-4-amine as a yellow powder (1.81 g, 70 % yield). The product was used directly in the next reaction.
d) An analogous reaction to that described in example 2d was performed, but starting with 4-hydroxypiperidine (1.00 g, 9.95 mmol) and *N*-(2-{[(3-chloro-4-fluorophenyl)amino]methyl}pyrimidin-5-yl)-7-(3-chloropropoxy)-6-methoxyquinazolin-4-amine (1.0 g, 2.0 mmol). Purification by flash chromatography, eluting with dichloromethane : methanol (9:1) followed by increased polarity to dichloromethane : methanol : ammonia (9:1:0.6) yielded 1-[3-({4-[(2-{[(3-chloro-4-fluorophenyl)amino]methyl}pyrimidin-5-yl)amino]-6-methoxyquinazolin-7-yl}oxy)propyl]piperidin-4-ol as a pale yellow solid (700 mg, 62 % yield) :
   ¹H-NMR (DMSO-d₆) : 9.73 (s, 1H), 9.20 (s, 2H), 8.50 (s, 1H), 7.80 (s, 1H), 7.22 (s, 1H), 7.10 (dd, 1H), 6.78 (dd, 1H), 6.64 (m, 1H), 6.48 (dd, 1H), 4.50 (d, 1H), 4.44 (d, 2H), 4.18 (t, 2H), 3.97 (s, 3H), 3.44 (m, 1H), 2.73 (m, 2H), 2.42 (m, 2H), 2.05-1.91 (m, 4H), 1.71 (m, 2H), 1.41 (m, 2H) :
   ¹⁹F-NMR (DMSO-d₆) : -134.4 (m) :
   MS (-ve ESI) : 566, 568 (M-H)⁻,
   MS (+ve ESI) : 568, 570 (M+H)⁺.

### Example 27 - Preparation of Compound 27 in Table 2 - 3-[(3-{[4-({2-[(3-chloro-4-fluorobenzyl)oxy]pyrimidin-5-yl}amino)-6-methoxyquinazolin-7-yl]oxy}propyl)amino]-3-methylbutyl dihydrogen phosphate

An analogous reaction to that described in example 9, but starting with 3-[(3-{[4-({2-[(3-chloro-4-fluorobenzyl)oxy]pyrimidin-5-yl}amino)-6-methoxyquinazolin-7-yl]oxy}propyl)amino]-3-methylbutan-1-ol (1.14 g, 2.00 mmol) yielded the title compound as a pale yellow solid (149 mg, 17 % yield) :
¹H-NMR (DMSO-d₆): 9.73 (s, 1H), 8.98 (s, 2H), 8.43 (s, 1H), 7.82 (s, 1H), 7.74 (m, 1H), 7.53 (m, 1H), 7.46 (t, 1H), 7.25 (s, 1H), 5.41 (s, 2H), 4.24 (t, 2H), 3.99 (s, 3H), 3.92 (m, 2H), 3.0 (t, 2H), 2.21 (m, 2H), 1.85 (t, 2H), 1.31 (s, 6H) :
MS (+ve ESI): 651 (M+H)⁺.
3-[(3-{[4-({2-[(3-chloro-4-fluorobenzyl)oxy]pyrimidin-5-yl}amino)-6-methoxyquinazolin-7-yl]oxy}propyl)amino]-3-methylbutan-1-ol, used as the starting material, was obtained as follows:
a) 2-[(3-chloro-4-fluorobenzyl)oxy]pyrimidin-5-amine (2.54 g, 10.0 mmol, see WO 01/21597) and 4-chloro-6-methoxy-7-(3-chloroxypropoxy)quinazoline (2.87 g, 10 mmol) were dissolved in dimethylacetamide (60 ml) and warmed to 40 °C. Hydrogen chloride (4 N solution in 1,4 dioxane, 2.5 ml, 10 mmol) was added slowly and the reaction heated at 70 °C for 30 minutes. The reaction was cooled, diluted with ether and the resultant precipitate collected by filtration and washed with diethyl ether. The solid was triturated with acetonitrile to afford *N*-{2-[(3-chloro-4-fluorobenzyl)oxy]pyrimidin-5-yl}-7-(3-chloropropoxy)-6-methoxyquinazolin-4-amine (di- hydrochloride salt) as an off-white solid (4.88 g, 90 % yield):
   ¹H-NMR (DMSO-d₆): 8.96 (s, 2H), 8.82 (s, 1H), 8.40 (s, 1H), 7.70 (m, 1H), 7.51 (m, 1H), 7.43 (m, 1H), 7.38 (s, 1H), 5.41 (s, 2H), 4.30 (t, 2H), 4.02 (s, 3H), 3.82 (t, 2H), 2.28 (t, 2H) : MS (+ve ESI): 504 (M+H)⁺.
b) An analogous reaction to that described in example 2d, but starting with *N*-{2-[(3-chloro-4-fluorobenzyl)oxy]pyrimidin-5-yl}-7-(3-chloropropoxy)-6-methoxyquinazolin-4-amine (2.7 g, 5.00 mmol) and 3-amino-3-methylbutanol (2.57 g, 25.0 mmol). The reaction yielded 3-[(3-{[4-({2-[(3-chloro-4-fluorobenzyl)oxy]pyrimidin-5-yl}amino)-6-methoxyquinazolin-7-yl]oxy}propyl)amino]-3-methylbutan-1-ol as a colourless solid (2.22 g, 78 % yield) :
   ¹H-NMR (DMSO-d₆): 9.65 (s, 1H), 8.97 (s, 2H), 8.47 (s, 2H), 7.79 (s, 1H), 7.72 (m, 1H), 7.51 (m, 1H), 7.45 (t, 1H), 7.21 (s, 1H), 5.41 (s, 2H), 4.21 (t, 2H), 3.98 (s, 3H), 3.82 (t, 2H), 2.69 (t, 2H), 1.90 (t, 2H), 1.53 (t, 2H), 1.04 (s, 6H) :
   MS (+ve ESI): 571 (M+H)⁺.

### Example 28 - Preparation of Compound 28 in Table 2 - 2-[(3-{(4-({2-[(3-chlorobenzoyl)amino]pyrimidin-5-yl}amino)-6-methoxyquinazolin-7-yl]oxy}propyl)(2,2-dimethylpropyl)amino]ethyl dihydrogen phosphate

An analogous reaction to that described in example 12, but starting with 3-chloro-*N-*{5-[(7-{3-[(2-hydroxyethyl)(2,2-dimethylpropyl)amino]propoxy}-6-methoxyquinazolin-4-yl)amino]pyrimidin-2-yl}benzamide (600 mg, 1.01 mmol), initially yielded di-*tert*-butyl 2-[(3-{[4-({2-[(3-chlorobenzoyl)amino]pyrimidin-5-yl}amino)-6-methoxyquinazolin-7-yl]oxy}propyl)(2,2-dimethylpropyl)amino]ethyl phosphate (610 mg, 77 % yield) as a pale yellow solid:
¹H-NMR (DMSO d₆): 11.09 (s, 1H), 9.74 (s, 1H), 9.12 (s, 2H), 8.50 (s, 1H), 8.03 (s, 1H), 7.95 (d, 1H), 7.82 (s, 1H), 7.69 (d, 1H), 7.57 (t, 1H), 7.20 (s, 1H), 4.20 (t, 2H), 3.98 (s, 3H), 3.88 (dd, 2H), 2.70 (m, 4H), 2.27 (s, 2H), 1.93 (m, 2H), 1.38 (s, 18H), 0.84 (s, 9H) :
MS (+ve ESI): 786 (M+H)⁺.
di-*tert*-butyl 2-[(3-{[4-({2-[(3-chlorobenzoyl)amino]pyrimidin-5-yl}amino)-6-methoxyquinazolin-7-yl]oxy}propyl)( 2,2-dimethylpropyl)amino]ethyl phosphate (600 mg, 0.76 mmol) was subjected to deprotection with hydrochloric acid (according to the method described in example 12 to yield the title compound (as the tri-hydrochloride salt, 600 mg, quantitative yield) as a pale yellow solid :
¹H-NMR (DMSO d₆): 12.30 (s, 1H), 11.27 (s, 1H), 9.18 (s, 2H), 8.90 (s, 1H), 8.69 (s, 1H), 8.03 (s, 1H), 7.95 (d, 1H), 7.71 (d, 1H), 7.58 (t, 1H), 7.52 (s, 1H), 4.35 (m, 4H), 4.05 (s, 3H), 3.51 (m, 2H), 3.40 (m, 2H), 3.19 (s, 2H), 2.50 (m, 2H), 1.13 (s, 9H) :
MS (+ve ESI): 674 (M+H)⁺.
3-chloro-*N*-{5-[(7-{3-[(2-hydroxyethyl)( 2,2-dimethylpropyl)amino]propoxy}-6-methoxyquinazolin-4-yl)amino]pyrimidin-2-yl}benzamide, used as the starting material, was obtained as follows:
a) An analogous reaction to that described in example 2d, but starting with 2-[(2,2-dimethylpropyl)amino]ethanol (1.10 mg, 12.0 mmol) yielded 3-chloro-*N*-{5-[(7-{3-[(2-hydroxyethyl)( 2,2-dimethylpropyl)amino]propoxy}-6-methoxyquinazolin-4-yl)amino]pyrimidin-2-yl}benzamide (663 mg, 28 % yield) as an off-white solid :
   ¹H-NMR (DMSO d₆): 11.10 (br s, 1H), 9.75 (br s, 1H), 9.12 (s, 2H), 8.50 (s, 1H), 8.02 (s, 1H), 7.93 (d, 1H), 7.82 (s, 1H), 7.68 (d, 1H), 7.56 (t, 1H), 7.20 (s, 1H), 4.29 (t, 1H), 4.19 (t, 2H), 3.98 (s, 3H), 3.46 (m, 2H), 2.68 (t, 2H), 2.50 (t, 2H), 2.20 (s, 2H), 1.92 (m, 2H), 0.82 (s, 9H) :
   MS (+ve ESI): 594 (M+H)⁺.

### Example 29 - Preparation of Compound 29 in Table 3 - [2-({[4-({2-[(3-chloro-4-fluorobenzoyl)amino]pyrimidin-5-yl}amino)-6-methoxyquinazolin-7-yl]oxy}methyl)cyclopropyl]methyl dihydrogen phosphate

An analogous reaction to that described in example 9, but starting with 3-chloro-4-fluoro-*N*-{5-[(7-{[2-(hydroxymethyl)cyclopropyl]methoxy}-6-methoxyquinazolin-4-yl)amino]pyrimidin-2-yl}benzamide (234 mg, 0.44 mmol) yielded the title compound as a pale orange solid (49 mg, 58 % yield) :
¹H-NMR (DMSO-d₆): 11.30 (s, 1H), 9.15 (s, 2H), 8.75 (s, 1H), 8.20 (d, 1H), 8.05 (m, 2H), 7.70 (t, 1H), 7.25 (s, 1H), 4.20 (m, 1H), 4.10-4.00 (m, 4H), 3.90 (m, 2H), 1.40-1.00 (m, 2H), 0.70 (m 2H) :
MS (+ve ESI): 605 (M+H)⁺.
3-chloro-4-fluoro-*N*-{5-[(7-{[2-(hydroxymethyl)cyclopropyl]methoxy}-6-methoxyquinazolin-4-yl)amino]pyrimidin-2-yl}benzamide, used as the starting material was obtained as follows:
a) 3-chloro-4-fluoro-*N*-{5-[(7-hydroxy-6-methoxyquinazolin-4-yl)amino]pyrimidin-2-yl}benzamide (3.50 g, 6.31 mmol), ethyl 2-(bromomethyl)cyclopropykcarboxylate (1.55 g, 7.49 mmol, see WO 92/04339) and potassium carbonate (4.12 g, 33.55 mmol) were combined in dimethylacetamide and heated to 60 °C for 18 hours. The reaction mixture was poured into water (400 ml) and the resultant precipitate filtered, dried and washed with diethyl ether to afford ethyl 2-({[4-({2-[(3-chloro-4-fluorobenzoyl)amino]pyrimidin-5-yl}amino)-6-methoxyquinazolin-7-yl]oxy}methyl)cyclopropylcarboxylate as a beige solid (2.75 g, 77 % yield) :
   MS (+ve ESI): 565 (M+H)⁺.
b) A solution of lithium aluminium hydride (5.5 ml, 1M solution in tetrahydrofuran) in tetrahydrofuran (60 ml) was cooled to 0 °C and a solution of ethyl 2-({[4-({2-[(3-chloro-4-fluorobenzoyl)amino]pyrimidin-5-yl}amino)-6-methoxyquinazolin-7-yl]oxy}methyl)cyclopropylcarboxylate (1.56 g, 2.75 mmol) in tetrahydrofuran (20 ml) was added slowly. The solution was stirred at 0 °C for 3 hours and further portions of lithium aluminium hydride (3 x 2.75 ml) were added at 0 °C at 18, 28 and 36 hours with warming to ambient temperature between each addition. The reaction mixture was cooled to 0 °C and hydrochloric acid (15 ml, 1 N solution in water) added slowly. Water (200 ml) was added and the reaction extracted with ethyl acetate (3 x 200 ml). The organics were combined, dried (magnesium sulphate) and concentrated (< 20 °C ) to afford a yellow solid. Trituration with diethyl ether afforded 3-chloro-4-fluoro-*N*-{5-[(7-{[2-(hydroxymethyl)cyclopropyl]methoxy}-6-methoxyquinazolin-4-yl)amino]pyrimidin-2-yl}benzamide as a yellow solid (726 mg, 50 % yield) :
   ¹H-NMR (DMSO-d₆): 9.80 (s, 1H), 9.20 (s, 2H), 8.50 (s, 1H), 8.20 (d, 1H), 8.05 (m, 2H), 7.81 (s, 1H), 7.65 (t, 1H), 7.25 (s, 1H), 4.50 (t, 1H), 4.20-3.80 (m, 2H), 3.40-3.20 (m, 2H), 1.40-1.00 (m, 2H), 0.61 (m 2H) :
   MS (+ve ESI): 525 (M+H)⁺.

### Example 30 - Preparation of Compound 30 in Table 3 - 2-[4-({[4-({2-[(3-chloro-4-fluorobenzoyl)amino]pyrimidin-5-yl}amino)-6-methoxyquinazolin-7-yl]oxy}methyl)piperidin-1-yl]ethyl dihydrogen phosphate

A solution of 3-chloro-4-fluoro-*N*-{5-[(7-{[1-(2-hydroxyethyl)piperidin-4-yl]methoxy}-6-methoxyquinazolin-4-yl)amino]pyrimidin-2-yl}benzamide (79 mg, 0.13 mmol) in dimethylformamide (2 ml) was reacted with 1-*H* tetrazole (41 mg, 0.58 mmol) and dibenzyl diethylphosphoramidite (60 µl, 0.20 mmol) and stirred at ambient temperature for 18 hours. Further portions of dibenzyl diethylphosphoramidite (100 µl) were added after 2 hours and 5 hours. The reaction mixture was cooled to -50 °C and 3-chloroperoxybenzoic acid (81 mg, 0.46 mmol) was added and the reaction mixture warmed to 0 °C. A further portion of 3-chloroperoxybenzoic acid (60 mg) was added at -50 °C and the solution was stirred for 4 hours then cooled to 0 °C. A solution of sodium metabisulphite (0.53 N aqueous solution) was added and the reaction was warmed to ambient temperature over 15 minutes then extracted with dichloromethane. The combined organics were dried (magnesium sulphate), filtered and concentrated under reduced pressure to yield the crude phosphate ester as a viscous, yellow oil. Purification by flash chromatography on silica gel, eluting with dichloromethane : methanol : 7.0 N ammonia in methanol (9:1:0 to 9:1:0.8) yielded dibenzyl 2-[4-({[4-({2-[(3-chloro-4-fluorobenzoyl)amino]pyrimidin-5-yl}amino)-6 methoxyquinazolin-7-yl]oxy}methyl)piperidin-1-yl]ethyl phosphate as a yellow oil (41 mg, 46 % yield) :
MS (+ve ESI) : 842 (M+H)⁺.
Dibenzyl 2-[4-({[4-({2-[(3-chloro-4-fluorobenzoyl)amino]pyrimidin-5-yl}amino)-6 methoxyquinazolin-7-yl]oxy}methyl)piperidin-1-yl]ethyl phosphate (41 mg, 0.048 mmol) was dissolved in dichloromethane and cooled to -78 °C. Bromotrimethylsilane (70 µl, 0.05 mmol) was added and the solution allowed to warm to ambient temperature and stirred for 1 hour. Methanol (3 ml) was added and the reaction concentrated, redissolved in methanol and evaporated to dryness. The residue was triturated with diethyl ether and filtered under nitrogen to yield the title compound as a yellow hydrobromide salt (41 mg, 98 % yield) :
¹H-NMR (DMSO-d₆): 9.20 (s, 2H), 8.80 (s, 1H), 8.55 (s, 1H), 8.25 (s, 1H), 8.20 (m, 1H), 8.05 (m, 1H), 7.51 (t, 1H), 7.41 (s, 1H), 4.30 (m, 2H), 4.20 (m, 2H), 4.05 (s, 3H), 3.60 (m, 2H), 3.41 (t, 2H), 3.20 (t, 2H), 2.35 (m, 1H), 2.25 (m, 2H), 1.90-1.70 (m, 2H) :
MS (-ve ESI): 660 (M-H)⁻.
3-chloro-4-fluoro-*N*-{5-[(7-{[1-(2-hydroxyethyl)piperidin-4-yl]methoxy}-6-methoxyquinazolin-4-yl)amino]pyrimidin-2-yl}benzamide used as the starting material was obtained as follows:
a) 3-chloro-4-fluoro-*N*-{5-[(7-hydroxy-6-methoxyquinazolin-4-yl)amino]pyrimidin-2-yl}benzamide (3.85 g, 6.94 mmol), *tert*-butyl 4-({[(4-methylphenyl)sulfonyl]oxy}methyl)piperidine-1-carboxylate (3.16 g, 8.55 mmol see-patent WO 02/00649) and potassium carbonate (4.2 g, 30.4 mmol) were dissolved in dimethylacetamide (50 ml) and stirred at 60 °C for 18 hours. The reaction mixture was cooled, poured into water (100 ml) and the resultant precipitate isolated. This was dissolved in a mixture of dichloromethane : trifluoroacetic acid (2:1, 30 ml) and stirred at ambient temperature for 5 hours. The reaction mixture was concentrated, toluene added and reconcentrated. The resultant solid was triturated with ether, filtered and dried to yield 3-chloro-4-fluoro-*N*-(5-{[6-methoxy-7-(piperidin-4-ylmethoxy)quinazolin-4-yl]amino}pyrimidin-2-yl)benzamide di-trifluoroacetate as a beige solid (4.6 g, 86 % yield) :
   MS (+ve ESI): 538 (M+H)⁺.
b) 3-chloro-4-fluoro-*N*-(5-{[6-methoxy-7-(piperidin-4-ylmethoxy)quinazolin-4-yl]amino}pyrimidin-2-yl)benzamide di-trifluoroacetate (4.6 g, 6.0 mmol), triethylamine (5 ml, 26.6 mmol) and 2-bromoethanol (2.20 ml, 31.0 mmol) were dissolved in dimethylformamide (100 ml) and heated at 60 °C for 18 hours. The reaction mixture was cooled and concentrated. Purification by flash chromatography on silica gel, eluting with dichloromethane : methanol (4:1) followed by increased polarity to dichloromethane :
   methanol : ammonia (4:1:0.1) yielded 3-chloro-4-fluoro-*N*-{5-[(7-{[1-(2-hydroxyethyl)piperidin-4-yl]methoxy}-6-methoxyquinazolin-4-yl)amino]pyrimidin-2-yl}benzamide as a beige solid (1.7 g, 48 % yield) :
   ¹H-NMR (DMSO-d₆): 9.80 (s, 1H), 9.15 (s, 2H), 8.80 (s, 1H), 8.20 (m, 1H), 8.00 (m, 1H), 7.80 (s, 1H), 7.60 (t, 1H), 4.20 (s, 1H), 4.05 (m, 5H), 3.65 (m, 2H), 3.15 (m, 2H), 2.40 (m, 2H), 2.25-2.00 (m, 2H), 1.90-1.70 (m, 3H), 1.55 (m, 2H) :
   MS (-ve ESI): 580 (M-H)⁻.

## Claims

1. A compound of formula (I): 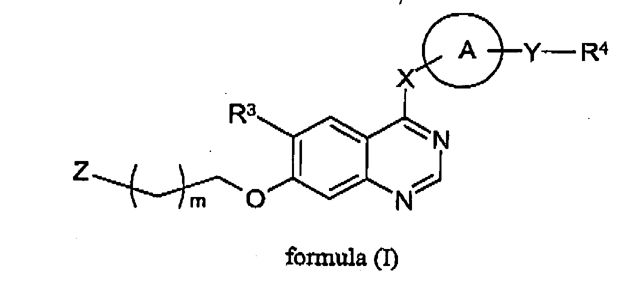
wherein **A** is 6-membered heteroaryl containing a nitrogen atom and optionally containing one or two further nitrogen atoms;
**X** is O, S, S(O), S(O)₂ or NR¹⁴;
**m** is 0, 1, 2, 3 or 4;
**Y** is a group selected from O, NR⁵CO, CONR⁵, CR⁶R⁷CONR⁵ and CR⁶R⁷NR⁵;
**Z** is a group selected from NR¹R², phosphonooxy, C₃₋₆cycloalkyl which C₃₋₆cycloalkyl is substituted by phosphonooxy or C₁₋₄alkyl substituted by phosphonooxy, and a 4- to 7-membered ring linked via a carbon atom containing a nitrogen atom and optionally containing a further nitrogen atom, which ring may be saturated, unsaturated or partially saturated which ring is substituted on carbon or nitrogen by phosphonooxy or C₁₋₄alkyl (substituted by phosphonooxy) and which ring is optionally further substituted on carbon or nitrogen by 1, 2 or 3 halo or C₁₋₄alkyl groups;
**R¹** is a group selected from -COR⁸, -CONR⁸R⁹ and C₁₋₆alkyl which C₁₋₆alkyl is substituted by phosphonooxy and optionally further substituted by 1 or 2 halo or methoxy groups;
**R²** is a group selected from hydrogen, -COR¹⁰, -CONR¹⁰R¹¹ and C₁₋₆alkyl which C₁₋₆alkyl is optionally substituted by 1, 2 or 3 halo or C₁₋₄alkoxy groups, -S(O)ₚR¹¹ (where p is 0, 1 or 2) or phosphonooxy, or R² is a group selected from C₂₋₆alkenyl, C₂₋₆alkynyl, C₃₋₆cycloalkyl and C₃₋₆cycloalkylC₁₋₄alkyl;
or **R¹** and **R²** together with the nitrogen to which they are attached form a 4- to 7- membered ring optionally containing a further nitrogen atom which ring may be saturated, unsaturated or partially saturated which ring is substituted on carbon or nitrogen by a group selected from phosphonooxy and C₁₋₄alkyl substituted by phosphonooxy or -NR⁸R⁹, and which ring is optionally further substituted on carbon or nitrogen by 1, 2 or 3 halo or C₁₋₄alkyl groups;
**R³** is a group selected from hydrogen, halo, cyano, nitro, C₁₋₆alkoxy, C₁₋₆alkyl, -OR¹², - CHR¹²R¹³, -OC(O)R¹², -C(O)R¹², -NR¹²C(O)R¹³, -C(O)NR¹²R¹³, -NR¹²SO₂R¹³ and - NR¹²R¹³;
**R⁴** is hydrogen or a group selected from C₁₋₄alkyl, heteroaryl, heteroarylC₁₋₄alkyl, aryl and arylC₁₋₄alkyl which group is optionally substituted by 1, 2 or 3 substitutents selected from halo, methyl, ethyl, cyclopropyl and ethynyl;
**R⁵** is a group selected from hydrogen, C₁₋₄alkyl, C₂₋₄alkenyl, C₂₋₄alkynyl, C₃₋₆cycloalkyl and C₃-₆cycloalkylC₁₋₄alkyl;
**R⁶** and **R⁷** are independently selected from hydrogen, halo, C₁₋₄alkyl, C₃₋₆cycloalkyl, hydroxy and C₁₋₄aldoxy;
**R⁸** is C₁₋₄alkyl substituted by phosphonooxy and optionally further substituted by 1 or 2 halo or methoxy groups;
**R⁹** is selected from hydrogen and C₁₋₄alkyl;
**R¹⁰** is selected from hydrogen and C₁₋₄alkyl which C₁₋₄alkyl is optionally substituted by halo, C₁₋₄alkoxy, S(O)q (where q is 0, 1 or 2) or phosphonoxy;
**R¹¹, R¹², R¹³** and **R¹⁴** are independently selected from hydrogen, C₁₋₄alkyl and heterocyclyl; or a pharmaceutically acceptable salt thereof.

2. A compound according to claim 1 wherein A is a group of formula (a), (b), (c) or (d): 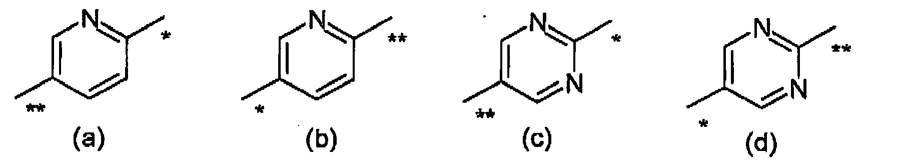 where * is the point of attachment to the X group of formula (I) and ** is the point of attachment to the Y group of formula (I); or a pharmaceutically acceptable salt thereof.

3. A compound according to claim 2 wherein A is a group of formula (b) or (d) as defined in claim 2; or a pharmaceutically acceptable salt thereof.

4. A compounds according to any one of claims 1, 2 or 3 wherein X is NH; or a pharmaceutically acceptable salt thereof.

5. A compound according to any one of the preceding claims wherein Z is a group selected from -NR¹R², phosphonooxy, cyclopropyl which cyclopropyl is substituted by C₁₋₄alkyl substituted by phosphonooxy, and a piperidine or piperazine ring linked via carbon which ring is substituted on carbon or nitrogen by phosphonooxy or C₁₋₄alkyl substituted by phosphonooxy; or a pharmaceutically acceptable salt thereof.

6. A compound according to any one of the preceding claims wherein R¹ is C₁₋₅alkyl substituted by phosphonooxy and R² is hydrogen, C₁₋₅alkyl, C₂₋₄alkynyl or C₃₋₆cycloalkyl; or a pharmaceutically acceptable salt thereof.

7. A compound according to any one of claims 1 to 5 wherein R¹ and R² together with the nitrogen to which they are attached form a piperidine, pyrrolidine or piperazine ring which is substituted on carbon or nitrogen by a group selected from phosphonooxy, phosphonooxymethyl and 2-phosphonooxyethyl and where the ring is optionally further substituted on carbon or nitrogen by 1 or 2 methyl.

8. A compound according to any one of the preceding claims wherein R³ is methoxy or hydrogen; or a pharmaceutically acceptable salt thereof.

9. A compound according to any one of the preceding claims wherein R⁴ is phenyl or benzyl optionally substituted by 1 or 2 of fluoro or chloro; or a pharmaceutically acceptable salt thereof.

10. A compound according to claim 1 selected from:
3-[(3-{[4-({6-[(3-chlorobenzyl)oxy]pyridin-3-yl}amino)-6-methoxyquinazolin-7-yl]oxy}propyl)amino]-3-methylbutyl dihydrogen phosphate;
3-[(3-{[4-({6-[(3-chlorobenzoyl)amino]pyridin-3-yl}amino)-6-methoxyquinazolin-7 yl]oxy}propyl)amino]-3-methylbutyl dihydrogen phosphate;
2-[(3-{[4-({6-[(3 chlorobenzoyl)amino]pyridin-3-yl}amino)-6-methoxyquinazolin-7-yl]oxy}propyl)(ethyl)amino]ethyl dihydrogen phosphate;
2-[1-(3-{[4-({6-[(3-chlorobenzoyl)amino]pyridin-3-yl}amino)-6-methoxyquinazolin-7 yl]oxy}propyl)piperidin-2-yl]ethyl dihydrogen phosphate;
[(2R)-1-(3-{[4-({6-[(3-chlorobenzoyl)amino]pyridin-3-yl}amino)-6-methoxyquinazolin-7-yl]oxy}propyl)pyrrolidin-2-yl]methyl dihydrogen phosphate;
2-[1-(3-{[4-({6-[(3-chlorobenzoyl)amino]pyridin-3-yl}amino)-6-methoxyquinazolin-7-yl]oxy}propyl)piperidin-4-yl]ethyl dihydrogen phosphate;
2-[ethyl(3-{[4-({6-[(3-fluorobenzoyl)amino]pyridin-3-yl}amino-6-methoxyquinazolin-7-yl]oxy}propyl)amino]ethyl dihydrogen phosphate;
2-[(3-{[4-({6-[(3,4-difluorobenzoyl)amino]pyridin-3-yl}amino)-6-methoxyquinazolin-7-yl]oxy}propyl)(isopropyl)amino]ethyl dihydrogen phosphate;
(3-{[4-({6-[(3-chlorobenzoyl)amino]pyridin-3-yl}amino)-6-methoxyquinazolin-7-yl]oxy}propyl)pipendin-4-yl dihydrogen phosphate;
4-{[4-({6-[(3-chlorobenzoyl)amino]pyridin-3-yl}amino)-6-methoxyquinazolin-7-yl]oxy}butyl dihydrogen phosphate;
2-[(3-{[4-({6-[(3-chlorobenzoyl)amino]pyridin-3-yl}amino)-6-methoxyquinazolin-7-yl]oxy}propyl)(methyl)amino]ethyl dihydrogen phosphate;
[1-(3-{[4-({6-[(3-chlorobenzoyl)amino]pyridin-3-yl}amino)-6-methoxyquinazolin-7-yl]oxy}propyl)piperidin-2-yl]methyl dihydrogen phosphate;
2-[(5-{[4-({6-[(3-chlorobenzoyl)amino]pyridin-3-yl}amino)-6-methoxyquinazolin-7-yl]oxy}pentyl)(ethyl)amino]ethyl dihydrogen phosphate;
4-[(3-{[4-({6-[(3-chlorobenzoyl)amino]pyridin-3-yl}amino)-6-methoxyquinazolin-7-yl]oxy}propyl)(ethyl)amino]butyl dihydrogen phosphate;
2-[(3-{[4-({6-[(3-fluorobenzoyl)amino]pyridin-3-yl}amino)-6-methoxyquinazolin-7-yl]oxy}propyl)(methyl)amino]ethyl dihydrogen phosphate;
2-[(3-{[4-({6-[(3-chlorobenzoyl)amiao]pyridin-3-yl}amino)-6-methoxyquinazolin-7-yl]oxy}propyl)(isobutyl)amino]ethyl dihydrogen phosphate;
2-[(3-{[4-({6-[(3-chlorobenzoyl)amino]pyridin-3-yl}amino)-6-methoxyquinazolin-7-yl]oxy}propyl)(cyclopropyl)amino]ethyl dihydrogen phosphate;
[1-(3-{[4-({6-[(3-chlorobenzoyl)amino]pyridin-3-yl}amino)-6-methoxyquinazolin-7-yl]oxy}propyl)piperidin-4-yl]methyl dihydrogen phosphate;
2-[4-(3-{[4-({6-[(3-chlorobenzoyl)amino]pyridin-3-yl}amino)-6-methoxyquinazolin-7-yl]oxy}propyl)piperazin-1-yl]ethyl dihydrogen phosphate;
[(2*S*)-1-(3-{[4-({6-[(3-chlorobenzoyl)amino]pyridin-3-yl}amino)-6-methoxyquinazolin-7-yl]oxy}propyl)pyrrolidin-2-yl]methyl dihydrogen phosphate;
2-[(3-{[4-({6-[(3-chlorobenzoyl)amino]pyridin-3-yl}amino)-6-methoxyquinazolin-7-yl]oxy}propyl)(cyclobutyl)amino]ethyl dihydrogen phosphate;
2-[(3- {[4-({6-[(3-chlorobenzoyl)amino]pyridin-3-yl}amino)-6-methoxyquinazolin-7-yl]oxy}propyl)(prop-2-yn-1-yl)amino]ethyl dihydrogen phosphate;
2-[(3-{[4-({2-[(3-chloro-4-fluorobenzoyl)amino]pyrimidin-5-yl}amino)-6-methoxyquinazolin-7-yl]oxy}propyl)(cyclohexyl)amino]ethyl dihydrogen phosphate;
2-[(3-{[4-({2-[(3-chloro-4-fluorobenzoyl)amino]pyrimidin-5-yl}amino)-6-methoxyquinazolin-7-yl]oxy}propyl)(ethyl)amino]ethyl dihydrogen phosphate;
3-{[4-({2-[(3-chorobenzoyl)amino]pyrimidin-5-yl}amino)-6-methoxyquinazolin-7-yl]oxy}propyl dihydrogen phosphate;
1-[3-({4-[(2-{[(3-chloro-4-fluorophenyl)amino]methyl}pyrimidin-5-yl)amino]-6-methoxyquinazolin-7-yl}oxy)propyl]piperidin-4-yl dihydrogen phosphate;
3-[(3- {[4-({2-[(3-chloro-4-fluorobenzyl)oxy]pyrimidin-5-yl}amino)-6-methoxyquinazolin-7-yl]oxy}propyl)amino]-3-methylbutyl dihydrogen phosphate;
2-[(3-{[4-({2-[(3-chlorobenzoyl)amino]pyrimidin-5-yl}amino)-6-methoxyquinazolin-7-yl]oxy}propyl)(2,2-dimethylpropyl)amino]ethyl dihydrogen phosphate;
[2-({[4-({2-[(3-chloro-4-fluorobenzoyl)amino]pyrimidin-5-yl}amino)-6-methoxyquinazolin-7-yl]oxy}methyl)cyclopropyl]methyl dihydrogen phosphate; and
2-[4-({[4-({2-[(3-chloro-4-fluorobenzoyl)amino]pyrimidin-5-yl}amino)-6-methoxyquinazolin-7-yl]oxy}methyl)piperidin-1-yl]ethyl dihydrogen phosphate;
or a pharmaceutically acceptable salt thereof.

11. A pharmaceutical composition comprising a compound according to any one of the preceding claims or a pharmaceutically acceptable salt thereof in association with a pharmaceutically acceptable diluent or carrier.

12. A compound according to any one of claims 1 to 10, or a pharmaceutically acceptable salt thereof, for use as a medicament.

13. A compound according to any one of claims 1 to 10, or a pharmaceutically acceptable salt thereof, for use in the treatment of cancer.

14. Use of a compound according to any one of claims 1 to 10 in the preparation of a medicament for the treatment of a disease where the inhibition of one or more Aurora kinase is beneficial.

15. Use according to claim 13 wherein Aurora kinase is Aurora-A kinase or Aurora-B kinase.

16. Use of a compound according to any one of claims 1 to 10 or a pharmaceutically acceptable salt thereof, in the preparation of a medicament for the treatment of hyperproliferative diseases such as cancer and in particular colorectal, breast, lung, prostate, pancreatic or bladder and renal cancer or leukemias or lymphomas.

17. Use of a compound according to any one of claims 1 to 10, or a pharmaceutically acceptable salt thereof and a chemotherapeutic agent selected from one or more of the following categories of anti-tumour agents:-
(i) antiproliferative/antineoplastic drugs and combinations thereof;
(ii) cytostatic agents;
(iii) agents which inhibit cancer cell invasion;
(iv) inhibitors of growth factor function;
(v) antiangiogenic agents;
(vi) vascular damaging agents;
(vii) antisense therapies;
(viii) gene therapy approaches; and
(ix) immunotherapy approaches;
in the manufacture of a medicament for use in the treatment of cancer.

18. A process for the preparation of a compound of formula (I) as defined in claim 1 or a pharmaceutically acceptable salt thereof, which process comprises converting a compound of formula (II) into a compound of formula (I) by phosphorylation of an appropriate hydroxy group: 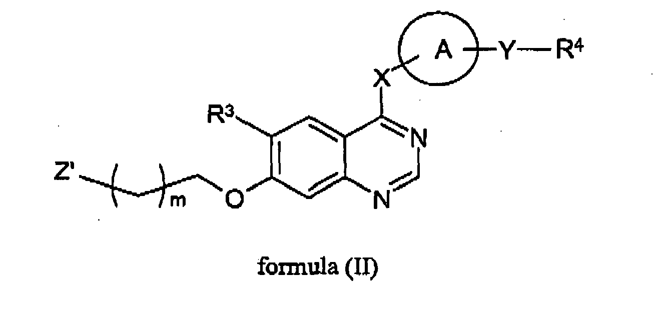 where A, X, m, Y, R³ and R⁴ are as defined for formula (I); and **Z'** is a group selected from - NR^{1'}R^{2'}, hydroxy, C₃₋₆cycloalkyl which C₃₋₆cycloalkyl is substituted by hydroxy or C₁₋₄alkyl substituted by hydroxy, and a 4- to 7-membered ring linked via a carbon atom, containing a nitrogen atom and optionally containing a further nitrogen atom, which ring may be saturated, unsaturated or partially saturated and which ring is substituted on carbon or nitrogen by hydroxy or C₁₋₄alkyl substituted by hydroxy and which ring is optionally further substituted on carbon or nitrogen by 1, 2 or 3 halo or C₁₋₄alkyl groups; **R^{1'}** is a group selected from-COR^{8'}, -CONR^{8'}R⁹ and C₁₋₆alkyl which C₁₋₆alkyl is substituted by hydroxy and optionally further substituted by 1 or 2 halo or methoxy groups; **R^{2'}** is a group selected from hydrogen, - COR¹⁰, -CONR¹⁰R¹¹ and C₁₋₆alkyl which C₁₋₆alkyl is optionally substituted by 1, 2 or 3 halo or C₁₋₄alkoxy groups, -S(O)ₚR¹¹ (where p is 0, 1 or 2) or hydroxy, or R^{2'} is a group selected from C₂₋₆alkenyl, C₂₋₆alkynyl, C₃₋₆cycloalkyl and C₃₋₆cycloalkylC₁₋₄alkyl; or **R^{1'}** and **R^{2'}** together with the nitrogen to which they are attached form a 4- to 7- membered ring optionally containing a further nitrogen atom which ring may be saturated, unsaturated or partially saturated and which ring is substituted on carbon or nitrogen by a group selected from hydroxy and C₁₋₄alkyl which C₁₋₄alkyl is substituted by hydroxy or -NR^{8'}R⁹ and which ring is optionally further substituted on carbon or nitrogen by 1, 2 or 3 halo or C₁₋₄alkyl groups; and where **R^{8'}** is C₁₋₄alkyl substituted by hydroxy and optionally further substituted by 1 or 2 halo or methoxy groups:
and thereafter if necessary:
i) converting a compound of the formula (I) into another compound of the formula (I); and/or
ii) removing any protecting groups; and/or
iii) forming a pharmaceutically acceptable salt thereof.

## Patentansprüche

1. Verbindung der Formel (I): 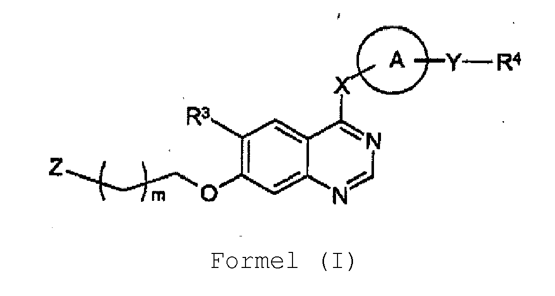
worin A für 6-gliedriges Heteroaryl, das ein Stickstoffatom und gegebenenfalls ein oder zwei weitere Stickstoffatome enthält, steht;
X für O, S, S(O), S(O)₂ oder NR¹⁴ steht;
m für 0, 1, 2, 3, oder 4 steht;
Y für eine unter O, NR⁵CO, CONR⁵, CR⁶R⁷CONR⁵ und CR⁶R⁷NR⁵ ausgewählte Gruppe steht;
Z für eine unter -NR¹R², Phosphonooxy, C₃₋₆-Cycloalkyl, das durch Phosphonooxy oder durch Phosphonooxy substituiertes C₁₋₄-Alkyl substituiert ist, und einem 4- bis 7-gliedrigen Ring, der über ein Kohlenstoffatom gebunden ist, ein Stickstoffatom und gegebenenfalls ein weiteres Stickstoffatom enthält, gesättigt, ungesättigt oder teilweise gesättigt sein kann, an Kohlenstoff oder Stickstoff durch Phosphonooxy oder C₁₋₄-Alkyl (substituiert durch Phosphonooxy) substituiert ist und gegebenenfalls ferner an Kohlenstoff oder Stickstoff durch 1, 2 oder 3 Halogen- oder C₁₋₄-Alkylgruppen substituiert ist, ausgewählte Gruppe steht;
R¹ für eine unter -COR⁸, -CONR⁸R⁹ und C₁₋₆-Alkyl, das durch Phosphonooxy substituiert ist und gegebenenfalls ferner durch 1 oder 2 Halogen- oder Methoxygruppen substituiert ist, ausgewählte Gruppe steht;
R² für eine unter Wasserstoff, -COR¹⁰, -CONR¹⁰R¹¹ und C₁₋₆-Alkyl, das gegebenenfalls durch 1, 2 oder 3 Halogen- oder C₁₋₄-Alkoxygruppen, -S(O)ₚR¹¹ (wobei p für 0, 1 oder 2 steht) oder Phosphonooxy substituiert ist, ausgewählte Gruppe steht oder R² für eine unter C₂₋₆-Alkenyl, C₂₋₆-Alkinyl, C₃₋₆-Cycloalkyl und C₃₋₆-Cycloalkyl-C₁₋₄-alkyl ausgewählte Gruppe steht;
oder R¹ und R² gemeinsam mit dem Stickstoffatom, an das sie gebunden sind, einen 4- bis 7-gliedrigen Ring bilden, der gegebenenfalls ein weiteres Stickstoffatom enthalten kann, gesättigt, ungesättigt oder teilweise gesättigt sein kann, an Kohlenstoff oder Stickstoff durch eine unter Phosphonooxy und durch Phosphonooxy oder -NR⁸R⁹ substituiertem C₁₋₄-Alkyl ausgewählte Gruppe substituiert ist und gegebenenfalls ferner an Kohlenstoff oder Stickstoff durch 1, 2 oder 3 Halogen- oder C₁₋₄-Alkylgruppen substituiert ist;
R³ für eine unter Wasserstoff, Halogen, Cyano, Nitro, C₁₋₆-Alkoxy, C₁₋₆-Alkyl, -OR¹², -CHR¹²R¹³, -OC(O)R¹², -C(O)R¹², -NR¹²C(O)R¹³, **-**C(O)NR¹²R¹³, -NR¹²SO₂R¹³ und -NR¹²R¹³ ausgewählte Gruppe steht;
R⁴ für Wasserstoff oder eine unter C₁₋₄-Alkyl, Heteroaryl, Heteroaryl-C₁₋₄-Alkyl und Aryl-C₁₋₄-alkyl ausgewählte Gruppe, die gegebenenfalls durch 1, 2 oder 3 unter Halogen, Methyl, Ethyl, Cyclopropyl und Ethinyl ausgewählte Substituenten substituiert ist, steht;
R⁵ für eine unter Wasserstoff, C₁₋₄-Alkyl, C₂₋₄-Alkenyl, C₂₋₄-Alkinyl, C₃₋₆-Cycloalkyl und C₃₋₆-Cycloalkyl-C₁₋₄-alkyl ausgewählte Gruppe steht;
R⁶ und R⁷ unabhängig voneinander unter Wasserstoff, Halogen, C₁₋₄-Alkyl, C₃₋₆-Cycloalkyl, Hydroxy und C₁₋₄-Alkoxy ausgewählt sind;
R⁸ für C₁₋₄-Alkyl, das durch Phosphonooxy substituiert ist und gegebenenfalls ferner durch 1 oder 2 Halogen- oder Methoxygruppen substituiert ist, steht;
R⁹ unter Wasserstoff und C₁₋₄-Alkyl ausgewählt ist;
R¹⁰ unter Wasserstoff und C₁₋₄-Alkyl, das gegebenenfalls durch Halogen, C₁₋₄-Alkoxy, -S(O)q (wobei q für 0, 1 oder 2 steht) oder Phosphonooxy substituiert ist, ausgewählt ist;
R¹¹, R¹², R¹³ und R¹⁴ unabhängig voneinander unter Wasserstoff, C₁₋₄-Alkyl und Heterocyclyl ausgewählt sind;
oder ein pharmazeutisch annehmbares Salz davon.

2. Verbindung nach Anspruch 1, in der A für eine Gruppe der Formel (a), (b), (c) oder (d) steht: 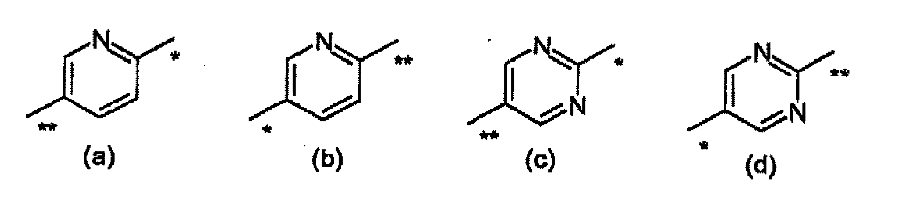 wobei * der Verknüpfungspunkt mit der Gruppe X der Formel (I) und ** der Verknüpfungspunkt mit der Gruppe Y der Formel (I) ist; oder ein pharmazeutisch annehmbares Salz davon.

3. Verbindung nach Anspruch 2, in der A für eine Gruppe der Formel (b) oder (d) gemäß Anspruch 2 steht; oder ein pharmazeutisch annehmbares Salz davon.

4. Verbindungen nach einem der Ansprüche 1, 2 oder 3, in denen X für NH steht; oder ein pharmazeutisch annehmbares Salz davon.

5. Verbindung nach einem der vorhergehenden Ansprüche, in der Z für eine unter -NR¹R², Phosphonooxy, Cyclopropyl, das durch durch Phosphonooxy substituiertes C₁₋₄-Alkyl substituiert ist, und einen Piperidin- oder Piperazinring, der über Kohlenstoff gebunden ist und an Kohlenstoff oder Stickstoff durch Phosphonooxy oder durch Phosphonooxy substituiertes C₁₋₄-Alkyl substituiert ist, ausgewählte Gruppe steht; oder ein pharmazeutisch annehmbares Salz davon.

6. Verbindung nach einem der vorhergehenden Ansprüche, in der R¹ für durch Phosphonooxy substituiertes C₁₋₅-Alkyl steht und R² für Wasserstoff, C₁₋₅-Alkyl, C₂₋₄-Alkinyl oder C₃₋₆-Cycloalkyl steht; oder ein pharmazeutisch annehmbares Salz davon.

7. Verbindung nach einem der Ansprüche 1 bis 5, in der R¹ und R² gemeinsam mit dem Stickstoffatom, an das sie gebunden sind, einen Piperidin-, Pyrrolidin- oder Piperazinring bilden, der an Kohlenstoff oder Stickstoff durch eine unter Phosphonooxy, Phosphonooxymethyl und 2-Phosphonooxyethyl ausgewählte Gruppe substituiert ist und gegebenenfalls ferner an Kohlenstoff oder Stickstoff durch 1 oder 2 Methyl substituiert ist.

8. Verbindung nach einem der vorhergehenden Ansprüche, in der R³ für Methoxy oder Wasserstoff steht; oder ein pharmazeutisch annehmbares Salz davon.

9. Verbindung nach einem der vorhergehenden Ansprüche, in der R⁴ für Phenyl oder Benzyl, das gegebenenfalls durch 1 oder 2 Fluor oder Chlor substituiert ist, steht; oder ein pharmazeutisch annehmbares Salz davon.

10. Verbindung nach Anspruch 1, ausgewählt unter:
3-[(3-{[4-({6-[(3-Chlorbenzyl)oxy]pyridin-3-yl}-amino)-6-methoxychinazolin-7-yl]oxy}propyl)amino]-3-methylbutyldihydrogenphosphat;
3-[(3-{[4-({6-[(3-Chlorbenzoyl)amino]pyridin-3-yl}amino)-6-methoxychinazolin-7-yl]oxy}-propyl)amino]-3-methylbutyldihydrogenphosphat;
2-[(3-{[4-({6-[(3-Chlorbenzoyl)amino]pyridin-3-yl}amino)-6-methoxychinazolin-7-yl]oxy}propyl)-(ethyl)amino]ethyldihydrogenphosphat;
2-[1-(3-{[4-({6-[(3-Chlorbenzoyl)amino]pyridin-3-yl}amino)-6-methoxychinazolin-7-yl]oxy}propyl)-piperidin-2-yl]ethyldihydrogenphosphat;
[(2R)-1-(3-{[4-({6-[(3-Chlorbenzoyl)amino]pyridin-3-yl}amino)-6-methoxychinazolin-7-yl]oxy}propyl)-pyrrolidin-2-yl]methyldihydrogenphosphat;
2-[1-(3-{[4-({6-[(3-Chlorbenzoyl)amino]pyridin-3-yl}amino)-6-methoxychinazolin-7-yl]oxy}propyl)-piperidin-4-yl]ethyldihydrogenphosphat;
2-[Ethyl(3-{[4-({6-[(3-Fluorbenzoyl)amino]pyridin-3-yl}amino)-6-methoxychinazolin-7-yl]oxy}propyl)-amino]ethyldihydrogenphosphat;
2-[(3-{[4-({6-[(3,4-Difluorbenzoyl)amino]pyridin-3-yl}amino)-6-methoxychinazolin-7-yl]oxy}propyl)-(isopropyl)amino]ethyldihydrogenphosphat;
(3-{[4-({6-[(3-Chlorbenzoyl)amino]pyridin-3-yl}-amino)-6-methoxychinazolin-7-yl]oxy}propyl)-piperidin-4-yldihydrogenphosphat;
4-{[4-({6-[(3-Chlorbenzoyl)amino]pyridin-3-yl}-amino)-6-methoxychinazolin-7-yl]oxy}butyl-dihydrogenphosphat;
2-[(3-{[4-({6-[(3-Chlorbenzoyl)amino]pyridin-3-yl}amino)-6-methoxychinazolin-7-yl]oxy}propyl)-(methyl)amino]ethyldihydrogenphosphat;
[1-(3-{[4-({6-[(3-Chlorbenzoyl)amino]pyridin-3-yl}amino)-6-methoxychinazolin-7-yl]oxy}propyl)-piperidin-2-yl]methyldihydrogenphosphat;
2-[(5-{[4-({6-[(3-Chlorbenzoyl)amino]pyridin-3-yl}amino)-6-methoxychinazolin-7-yl]oxy}pentyl)-(ethyl)amino]ethyldihydrogenphosphat;
4-[(3-{[4-({6-[(3-Chlorbenzoyl)amino]pyridin-3-yl}amino)-6-methoxychinazolin-7-yl]oxy}propyl)-(ethyl)amino]butyldihydrogenphosphat;
2-[(3-{[4-({6-[(3-Fluorbenzoyl)amino]pyridin-3-yl}amino)-6-methoxychinazolin-7-yl]oxy}propyl)-(methyl)amino]ethyldihydrogenphosphat;
2-[(3-{[4-({6-[(3-Chlorbenzoyl)amino]pyridin-3-yl}amino)-6-methoxychinazolin-7-yl]oxy}propyl)-(isobutyl)amino]ethyldihydrogenphosphat;
2-[(3-{[4-({6-[(3-Chlorbenzoyl)amino]pyridin-3-yl}amino)-6-methoxychinazolin-7-yl]oxy}propyl)-(cyclopropyl)amino]ethyldihydrogenphosphat;
[1-(3-{[4-({6-[(3-Chlorbenzoyl)amino]pyridin-3-yl}amino)-6-methoxychinazolin-7-yl]oxy}propyl)-piperidin-4-yl]methyldihydrogenphosphat;
2-[4-(3-{[4-({6-[(3-Chlorbenzoyl)amino]pyridin-3-yl}amino)-6-methoxychinazolin-7-yl]oxy}propyl)-piperazin-1-yl]ethyldihydrogenphosphat;
[(2S)-1-(3-{[4-({6-[(3-Chlorbenzoyl)amino]pyridin-3-yl}amino)-6-methoxychinazolin-7-yl]oxy}propyl)-pyrrolidin-2-yl]methyldihydrogenphosphat;
2-[(3-{[4-({6-[(3-Chlorbenzoyl)amino]pyridin-3-yl}amino)-6-methoxychinazolin-7-yl]oxy}propyl)-(cyclobutyl)amino]ethyldihydrogenphosphat;
2-[(3-{[4-({6-[(3-Chlorbenzoyl)amino]pyridin-3-yl}amino)-6-methoxychinazolin-7-yl]oxy}propyl)-(prop-2-in-1-yl)amino]ethyldihydrogenphosphat;
2-[(3-{[4-({2-[(3-Chlor-4-fluorbenzoyl)amino]-pyrimidin-5-yl}amino)-6-methoxychinazolin-7-yl]oxy}propyl)(cyclohexyl)amino]ethyldihydrogenphosphat;
2-[(3-{[4-({2-[(3-Chlor-4-fluorbenzoyl)amino]-pyrimidin-5-yl}amino)-6-methoxychinazolin-7-yl]oxy}propyl)(ethyl)amino]ethyldihydrogenphosphat;
3-{[4-({2-[(3-Chlorbenzoyl)amino]pyrimidin-5-yl}-amino)-6-methoxychinazolin-7-yl]oxy}propyldihydrogenphosphat;
1-[3-({4-[(2-{[(3-Chlor-4-fluorphenyl)amino]-methyl}pyrimidin-5-yl)amino]-6-methoxychinazolin-7-yl}oxy)propyl]piperidin-4-yldihydrogenphosphat;
3-[(3-{[4-({2-[(3-Chlor-4-fluorbenzyl)oxy]-pyrimidin-5-yl}amino)-6-methoxychinazolin-7-yl]oxy}propyl)amino]-3-methylbutyldihydrogenphosphat;
2-[(3-{[4-({2-[(3-Chlorbenzoyl)amino]pyrimidin-5-yl}amino)-6-methoxychinazolin-7-yl]oxy}propyl)-(2,2-dimethylpropyl)amino]ethyldihydrogenphosphat;
[2-({[4-({2-[(3-Chlor-4-fluorbenzoyl)amino]-pyrimidin-5-yl}amino)-6-methoxychinazolin-7-yl]-oxy}methyl)cyclopropyl]methyldihydrogenphosphat und
2-[4-({[4-({2-[(3-Chlor-4-fluorbenzoyl)amino]-pyrimidin-5-yl}amino)-6-methoxychinazolin-7-yl]-oxy}methyl)piperidin-1-yl]ethyldihydrogenphosphat;
oder ein pharmazeutisch annehmbares Salz davon.

11. Pharmazeutische Zusammensetzung, enthaltend eine Verbindung nach einem der vorhergehenden Ansprüche oder ein pharmazeutisch annehmbares Salz davon zusammen mit einem pharmazeutisch annehmbaren Verdünnungsmittel oder Träger.

12. Verbindung nach einem der Ansprüche 1 bis 10 oder ein pharmazeutisch annehmbares Salz davon zur Verwendung als Arzneimittel.

13. Verbindung nach einem der Ansprüche 1 bis 10 oder ein pharmazeutisch annehmbares Salz davon zur Behandlung von Krebs.

14. Verwendung einer Verbindung nach einem der Ansprüche 1 bis 10 bei der Herstellung eines Arzneimittels zur Behandlung einer Erkrankung, bei der die Inhibierung einer oder mehrerer Aurora-Kinasen vorteilhaft ist.

15. Verwendung nach Anspruch 13, bei der es sich bei der Aurora-Kinase um Aurora-A-Kinase oder Aurora-B-Kinase handelt.

16. Verwendung einer Verbindung nach einem der Ansprüche 1 bis 10 oder eines pharmazeutisch annehmbaren Salzes davon bei der Herstellung eines Arzneimittels zur Behandlung von hyperproliferativen Erkrankungen wie Krebs und insbesondere Kolorektal-, Brust-, Lungen-, Prostata-, Bauchspeicheldrüsen- oder Blasen- und Nierenkrebs oder Leukämien oder Lymphomen.

17. Verwendung einer Verbindung nach einem der Ansprüche 1 bis 10 oder eines pharmazeutisch annehmbaren Salzes davon und eines Chemotherapeutikums, das aus einer oder mehreren der folgenden Kategorien von Antitumormitteln ausgewählt ist:
(i) antiproliferative/antineoplastische Arzneistoffe und Kombinationen davon;
(ii) Cytostatika;
(iii) Mittel, die die Krebszelleninvasion inhibieren;
(iv) Inhibitoren der Wachstumsfaktorfunktion;
(v) antiangiogene Mittel;
(vi) gefäßschädigende Mittel;
(vii) Antisense-Therapien;
(viii) Gentherapie-Ansätze;
(ix) Immuntherapie-Ansätze;
bei der Herstellung eines Arzneimittels zur Behandlung von Krebs.

18. Verfahren zur Herstellung einer Verbindung der Formel (I) gemäß Anspruch 1 oder eines pharmazeutisch annehmbaren Salzes davon, bei dem man eine Verbindung der Formel (II) durch Phosphorylierung einer entsprechenden Hydroxygruppe in eine Verbindung der Formel (I) umwandelt: 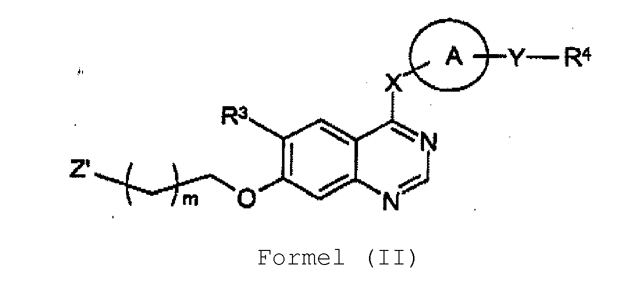 wobei A, X, m, Y, R³ und R⁴ die für Formel (I) angegebene Bedeutung besitzen; Z' für eine unter -NR^{1'}R^{2'}, Hydroxy, C₃₋₆-Cycloalkyl, das durch Hydroxy oder durch Hydroxy substituiertes C₁₋₄-Alkyl substituiert ist, und einem 4- bis 7-gliedrigen Ring, der über ein Kohlenstoffatom gebunden ist, ein Stickstoffatom und gegebenenfalls ein weiteres Stickstoffatom enthält, gesättigt, ungesättigt oder teilweise gesättigt sein kann, an Kohlenstoff oder Stickstoff durch Hydroxy oder durch Hydroxy substituiertes C₁₋₄-Alkyl substituiert ist und gegebenenfalls ferner an Kohlenstoff oder Stickstoff durch 1, 2 oder 3 Halogen- oder C₁₋₄-Alkylgruppen substituiert ist, ausgewählte. Gruppe steht; R^{1'} für eine unter -COR^{8'}, -CONR^{8'}R⁹ und C₁₋₆-Alkyl, das durch Hydroxy substituiert ist und gegebenenfalls ferner durch 1 oder 2 Halogen- oder Methoxygruppen substituiert ist, ausgewählte Gruppe steht; R^{2'} für eine unter Wasserstoff, - COR¹⁰, -CONR¹⁰R¹¹ und C₁₋₆-Alkyl, das gegebenenfalls durch 1, 2 oder 3 Halogen- oder C₁₋₄-Alkoxygruppen, -S(O)ₚR¹¹ (wobei p für 0, 1 oder 2 steht) oder Hydroxy substituiert ist, ausgewählte Gruppe steht oder R^{2'} für eine unter C₂₋₆-Alkenyl, C₂₋₆-Alkinyl, C₃₋₆-Cycloalkyl und C₃₋₆-Cycloalkyl-C₁₋₄-alkyl ausgewählte Gruppe steht; oder R^{1'} und R^{2'} gemeinsam mit dem Stickstoffatom, an das sie gebunden sind, einen 4- bis 7-gliedrigen Ring bilden, der gegebenenfalls ein weiteres Stickstoffatom enthalten kann, gesättigt, ungesättigt oder teilweise gesättigt sein kann, an Kohlenstoff oder Stickstoff durch eine unter Hydroxy und durch Hydroxy oder -NR^{8'}R⁹ substituiertem C₁₋₄-Alkyl ausgewählte Gruppe substituiert ist und gegebenenfalls ferner an Kohlenstoff oder Stickstoff durch 1, 2 oder 3 Halogen- oder C₁₋₄-Alkylgruppen substituiert ist; und R^{8'} für C₁₋₄-Alkyl, das durch Hydroxy substituiert ist und gegebenenfalls ferner durch 1 oder 2 Halogen- oder Methoxygruppen substituiert ist, steht;
und danach gegebenenfalls:
(i) eine Verbindung der Formel (I) in eine andere Verbindung der Formel (I) umwandelt und/oder
(ii) gegebenenfalls vorhandene Schutzgruppen abspaltet und/oder
(iii) ein pharmazeutisch annehmbares Salz davon bildet.

## Revendications

1. Composé de formule (I): 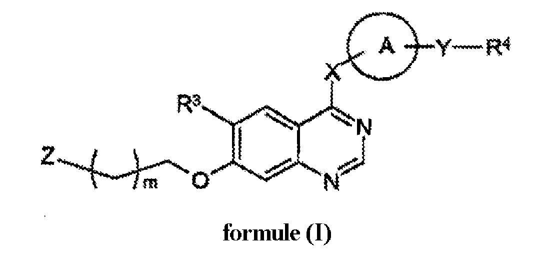
dans laquelle **A** est un hétéroaryle à 6 chaînons contenant un atome d'azote et contenant, éventuellement, un ou deux atomes d'azote supplémentaires ;
**X** est O, S, S(O), S(O)₂ ou NR¹⁴ ;
**m** est 0, 1, 2, 3, ou 4 ;
**Y** est un groupement choisi parmi O, NR⁵CO, CONR⁵, CR⁶R⁷CONR⁵ et CR⁶R⁷NR⁵ ;
**Z** est un groupement choisi parmi -NR¹R², phosphonooxy, C₃₋₆cycloalkyle, lequel C₃₋₆cycloalkyle est substitué par phosphonooxy ou C₁₋₄alkyle substitué par phosphonooxy, et un cycle à 4 à 7 chaînons lié par l'intermédiaire d'un atome de carbone contenant un atome d'azote et contenant, éventuellement, un atome d'azote supplémentaire, lequel cycle peut être saturé, insaturé ou partiellement saturé, lequel cycle est substitué sur le carbone ou l'azote par phosphonooxy ou C₁₋₄alkyle (substitué par phosphonooxy) et lequel cycle est, éventuellement, encore substitué sur le carbone ou l'azote par 1, 2 ou 3 groupements halogéno ou C₁₋₄alkyle ;
**R¹** est un groupement choisi parmi -COR⁸, -CONR⁸R⁹ et C₁₋₆alkyle, lequel C₁₋₆alkyle est substitué par phosphonooxy et, éventuellement, encore substitué par 1 ou 2 groupements halogéno ou méthoxy ;
**R²** est un groupement choisi parmi hydrogène, -COR¹⁰, -CONR¹⁰R¹¹ et C₁₋₆alkyle, lequel C₁₋₆alkyle est éventuellement substitué par 1, 2 ou 3 groupements halogéno ou C₁₋₄alcoxy, -S(O)ₚR¹¹ (où p est 0, 1 ou 2) ou phosphonooxy, ou R² est un groupement choisi parmi C₂₋₆alcényle, C₂₋₆alcynyle, C₃₋₆cycloalkyle et C₃₋₆-cycloalkyl-C₁₋₄alkyle ;
ou **R¹** et **R²,** ensemble avec l'azote auquel ils sont liés, forment un cycle à 4 à 7 chaînons contenant éventuellement un atome d'azote supplémentaire, lequel cycle peut être saturé, insaturé ou partiellement saturé, lequel cycle est substitué sur le carbone ou l'azote par un groupement choisi parmi phosphonooxy et C₁₋₄alkyle substitué par phosphonooxy ou -NR⁸R⁹, et lequel cycle est, éventuellement, encore substitué sur le carbone ou l'azote par 1, 2 ou 3 groupements halogéno ou C₁₋₄alkyle ;
**R³** est un groupement choisi parmi hydrogène, halogéno, cyano, nitro, C₁₋₆alcoxy, C₁₋₆alkyle, -OR¹², -CHR¹²R¹³, -OC(O)R¹², -C(O)R¹², -NR¹²C(O)R¹³, -C(O)NR¹²R¹³, -NR¹²SO₂R¹³ et -NR¹²R¹³ ;
**R⁴** est hydrogène ou un groupement choisi parmi C₁₋₄alkyle, hétéroaryle, hétéroaryl-C₁₋₄alkyle, aryle et aryl-C₁₋₄alkyle, lequel groupement est éventuellement substitué par 1, 2 ou 3 substituants choisis parmi halogéno, méthyle, éthyle, cyclopropyle et éthynyle ;
**R⁵** est un groupement choisi parmi hydrogène, C₁₋₄alkyle, C₂₋₄alcényle, C₂₋₄alcynyle, C₃₋₆cycloalkyle et C₃₋₆cycloalkyl-C₁₋₄alkyle ;
**R⁶** et **R⁷** sont choisis indépendamment parmi hydrogène, halogéno, C₁₋₄alkyle, C₃₋₆cycloalkyle, hydroxy et C₁₋₄alcoxy ;
**R⁸** est C₁₋₄alkyle substitué par phosphonooxy et, éventuellement, encore substitué par 1 ou 2 groupements halogéno ou méthoxy ;
**R⁹** est choisi parmi hydrogène et C₁₋₄alkyle ;
**R¹⁰** est choisi parmi hydrogène et C₁₋₄alkyle, lequel C₁₋₄alkyle est éventuellement substitué par halogéno, C₁₋₄alcoxy, S(O)_{q} (où q est 0, 1 ou 2) ou phosphonooxy ;
**R¹¹**, **R¹², R¹³** et **R¹⁴** sont choisis indépendamment parmi hydrogène, C₁₋₄alkyle et hétérocyclyle ;
ou un sel pharmaceutiquement acceptable de celui-ci.

2. Composé selon la revendication 1, **caractérisé en ce que** A est un groupement de formule (a), (b), (c) ou (d) : 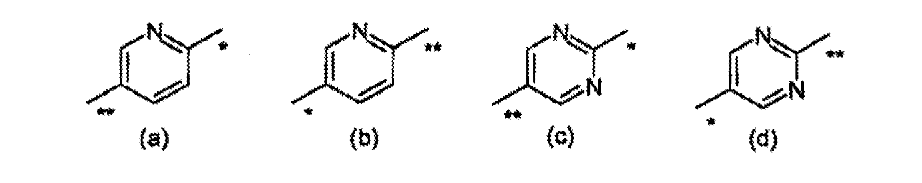 dans lesquelles * est le point d'attachement du groupement X de formule (I) et ** est le point d'attachement du groupement Y de formule (I) ; ou un sel pharmaceutiquement acceptable de celui-ci.

3. Composé selon la revendication 2, **caractérisé en ce que** A est un groupement de formule (b) ou (d), telle que définie dans la revendication 2 ; ou un sel pharmaceutiquement acceptable de celui-ci.

4. Composé selon l'une quelconque des revendications 1, 2 ou 3, **caractérisé en ce que** X est NH ; ou un sel pharmaceutiquement acceptable de celui-ci.

5. Composé selon l'une quelconque des revendications précédentes, **caractérisé en ce que** Z est un groupement choisi parmi -NR¹R², phosphonooxy, cyclopropyle, lequel cyclopropyle est substitué par C₁₋₄alkyle substitué par phosphonooxy, et un cycle pipéridine ou pipérazine lié par l'intermédiaire d'un carbone, lequel cycle est substitué sur le carbone ou l'azote par phosphonooxy ou C₁₋₄alkyle substitué par phosphonooxy ; ou un sel pharmaceutiquement acceptable de celui-ci.

6. Composé selon l'une quelconque des revendications précédentes, **caractérisé en ce que** R¹ est C₁₋₅alkyle substitué par phosphonooxy et R² est hydrogène, C₁₋₅alkyle, C₂₋₄alcynyle ou C₃₋₆cycloalkyle ; ou un sel pharmaceutiquement acceptable de celui-ci.

7. Composé selon l'une quelconque des revendications 1 à 5, **caractérisé en ce que** R¹ et R², ensemble avec l'azote auquel ils sont liés, forment un cycle pipéridine, pyrrolidine ou pipérazine, qui est substitué sur le carbone ou l'azote par un groupement choisi parmi phosphonooxy, phosphonooxyméthyle et 2-phosphonooxyéthyle et où le cycle est, éventuellement, encore substitué sur le carbone ou l'azote par 1 ou 2 méthyle.

8. Composé selon l'une quelconque des revendications précédentes, **caractérisé en ce que** R³ est méthoxy ou hydrogène ; ou un sel pharmaceutiquement acceptable de celui-ci.

9. Composé selon l'une quelconque des revendications précédentes, **caractérisé en ce que** R⁴ est phényle ou benzyle éventuellement substitué par 1 ou 2 fluoro ou chloro ; ou un sel pharmaceutiquement acceptable de celui-ci.

10. Composé selon la revendication 1, choisi parmi :
le dihydrogénophosphate de 3-[(3-{[4-({6-[(3-chlorobenzyl)oxy]pyridin-3-yl}amino)-6-méthoxyquinazolin-7-yl]oxy}propyl)amino]-3-méthylbutyle ;
le dihydrogénophosphate de 3-[(3-{[4-({6-[(3-chlorobenzoyl)amino]pyridin-3-yl}amino)-6-méthoxyquinazolin-7-yl]oxy}propyl)amino]-3-méthylbutyle ;
le dihydrogénophosphate de 2-[(3-{[4-({6-[(3-chlorobenzoyl)amino]pyridin-3-yl}amino)-6-méthoxyquinazolin-7-yl]oxy}propyl)(éthyl)amino]éthyle ;
le dihydrogénophosphate de 2-[1-(3-{[4-({6-[(3-chlorobenzoyl)amino]pyridin-3-yl}amino)-6-méthoxyquinazolin-7-yl]oxy}propyl)pipéridin-2-yl]éthyle;
le dihydrogénophosphate de [(2R)-1-(3-{[4-({6-[(3-chlorobenzoyl)amino]pyridin-3-yl}amino)-6-méthoxyquinazolin-7-yl]oxy}propyl)pyrrolidin-2-yl]méthyle ;
le dihydrogénophosphate de 2-[1-(3-{[4-({6-[(3-chlorobenzoyl)amino]pyridin-3-yl}amino)-6-méthoxyquinazolin-7-yl]oxy}propyl)pipéridin-4-yl]éthyle ;
le dihydrogénophosphate de 2-[éthyl(3-{[4-({6-[(3-fluorobenzoyl)amino]pyridin-3-yl}amino)-6-méthoxyquinazolin-7-yl]oxy}propyl)amino]éthyle ;
le dihydrogénophosphate de 2-[(3-{[4-({6-[(3,4-difluorobenzoyl)amino]pyridin-3-yl}amino)-6-méthoxyquinazolin-7-yl]oxy}propyl)(isopropyl)amino]éthyle ;
le dihydrogénophosphate de (3-{[4-({6-[(3-chlorobenzoyl)amino]pyridin-3-yl}amino)-6-méthoxyquinazolin-7-yl]oxy}propyl)pipéridin-4-yle ;
le dihydrogénophosphate de 4-{[4-({6-[(3-chlorobenzoyl)amino]pyridin-3-yl}amino)-6-méthoxyquinazolin-7-yl]oxy}butyle ;
le dihydrogénophosphate de 2-[(3-{[4-({6-[(3-chlorobenzoyl)amino]pyridin-3-yl}amino)-6-méthoxyquinazolin-7-yl]oxy}propyl)(méthyl)amino]éthyle ;
le dihydrogénophosphate de [1-(3-{[4-({6-[(3-chlorobenzoyl)amino]pyridin-3-yl}amino)-6-méthoxyquinazolin-7-yl]oxy}propyl)pipéridin-2-yl]méthyle ;
le dihydrogénophosphate de 2-[(5-{[4-({6-[(3-chlorobenzoyl)amino]pyridin-3-yl}amino)-6-méthoxyquinazolin-7-yl]oxy}pentyl)(éthyl)amino]éthyle ;
le dihydrogénophosphate de 4-[(3-{[4-({6-[(3-chlorobenzoyl)amino]pyridin-3-yl}amino)-6-méthoxyquinazolin-7-yl]oxy}propyl)(éthyl)amino]butyle ;
le dihydrogénophosphate de 2-[(3-{[4-({6-[(3-fluorobenzoyl)amino]pyridin-3-yl}amino)-6-méthoxyquinazolin-7-yl]oxy}propyl)(méthyl)amino]éthyle ;
le dihydrogénophosphate de 2-[(3-{[4-({6-[(3-chlorobenzoyl)amino]pyridin-3-yl}amino)-6-méthoxyquinazolin-7-yl]oxy}propyl)(isobutyl)amino]éthyle ;
le dihydrogénophosphate de 2-[(3-{[4-({6-[(3-chlorobenzoyl)amino]pyridin-3-yl}amino)-6-méthoxyquinazolin-7-yl]oxy}propyl)(cyclopropyl)amino]-éthyle ;
le dihydrogénophosphate de [1-(3-{[4-({6-[(3-chlorobenzoyl)amino]pyridin-3-yl}amino)-6-méthoxyquinazolin-7-yl]oxy}propyl)pipéridin-4-yl]méthyle ;
le dihydrogénophosphate de 2-[4-(3-{[4-({6-[(3-chlorobenzoyl)amino]pyridin-3-yl}amino)-6-méthoxyquinazolin-7-yl]oxy}propyl)pipérazin-1-yl]éthyle ;
le dihydrogénophosphate de [(2*S*)-1-(3-{[4-({6-[(3-chlorobenzoyl)amino]pyridin-3-yl}amino)-6-méthoxyquinazolin-7-yl]oxy}propyl)pyrrolidin-2-yl]méthyle ;
le dihydrogénophosphate de 2-[(3-{[4-({6-[(3-chlorobenzoyl)amino]pyridin-3-yl}amino)-6-méthoxyquinazolin-7-yl]oxy}propyl)(cyclobutyl)amino]-éthyle ;
le dihydrogénophosphate de 2-[(3-{[4-({6-[(3-chlorobenzoyl)amino]pyridin-3-yl}amino)-6-méthoxyquinazolin-7-yl]oxy}propyl)(prop-2-yn-1-yl)amino]-éthyle ;
le dihydrogénophosphate de 2-[(3-{[4-({2-[(3-chloro-4-fluorobenzoyl)amino]pyrimidin-5-yl}amino)-6-méthoxyquinazolin-7-yl]oxy}propyl)(cyclohexyl)-amino]éthyle ;
le dihydrogénophosphate de 2-[(3-{[4-({2-[(3-chloro-4-fluorobenzoyl)amino]pyrimidin-5-yl}amino)-6-méthoxyquinazolin-7-yl]oxy}propyl)(éthyl)amino]-éthyle ;
le dihydrogénophosphate de 3-{[4-({2-[(3-chlorobenzoyl)amino]pyrimidin-5-yl}amino)-6-méthoxyquinazolin-7-yl]oxy}propyle ;
le dihydrogénophosphate de 1-[3-({4-[(2-{[(3-chloro-4-fluorophényl)amino]méthyl}pyrimidin-5-yl)amino]-6-méthoxyquinazolin-7-yl}oxy)propyl]pipéridin-4-yle ;
le dihydrogénophosphate de 3-[(3-{[4-({2-[(3-chloro-4-fluorobenzyl)oxy]pyrimidin-5-yl}amino)-6-méthoxyquinazolin-7-yl]oxy}propyl)amino]-3-méthylbutyle ;
le dihydrogénophosphate de 2-[(3-{[4-({2-[(3-chlorobenzoyl)amino]pyrimidin-5-yl}amino)-6-méthoxyquinazolin-7-yl]oxy}propyl)(2,2-diméthylpropyl)-amino]éthyle ;
le dihydrogénophosphate de [2-({[4-({2-[(3-chloro-4-fluorobenzoyl)amino]pyrimidin-5-yl}amino)-6-méthoxyquinazolin-7-yl]oxy}méthyl)cyclopropyl]méthyle ; et
le dihydrogénophosphate de 2-[4-({[4-({2-[(3-chloro-4-fluorobenzoyl)amino]pyrimidin-5-yl}amino)-6-méthoxyquinazolin-7-yl]oxy}méthyl)pipéridin-1-yl]-éthyle ;
ou un sel pharmaceutiquement acceptable de ceux-ci.

11. Composition pharmaceutique comprenant un composé selon l'une quelconque des revendications précédentes, ou un sel pharmaceutiquement acceptable de celui-ci, en association avec un diluant ou un support pharmaceutiquement acceptable.

12. Composé selon l'une quelconque des revendications 1 à 10, ou un sel pharmaceutiquement acceptable de celui-ci, destiné à être utilisé comme médicament.

13. Composé selon l'une quelconque des revendications 1 à 10, ou un sel pharmaceutiquement acceptable de celui-ci, destiné à être utilisé dans le traitement du cancer.

14. Utilisation d'un composé selon l'une quelconque des revendications 1 à 10, dans la préparation d'un médicament destiné au traitement d'une maladie dans laquelle l'inhibition d'une ou plusieurs kinases Aurora est bénéfique.

15. Utilisation selon la revendication 13, **caractérisée en ce que** la kinase Aurora est la kinase Aurora-A ou la kinase Aurora-B.

16. Utilisation d'un composé selon l'une quelconque des revendications 1 à 10, ou d'un sel pharmaceutiquement acceptable de celui-ci, dans la préparation d'un médicament destiné au traitement de maladies hyper-prolifératives, telles que le cancer et, en particulier, le cancer colorectal, du sein, du poumon, de la prostate, du pancréas ou de la vessie et du rein, ou les leucémies ou les lymphomes.

17. Utilisation d'un composé selon l'une quelconque des revendications 1 à 10, ou d'un sel pharmaceutiquement acceptable de celui-ci, et d'un agent chimiothérapeutique choisi parmi une ou plusieurs des catégories suivantes d'agents antitumoraux :
(i) les médicaments anti-prolifératifs/antinéoplasiques et les combinaisons de ceux-ci ;
(ii) les agents cytostatiques ;
(iii) les agents inhibant l'invasion de cellules cancéreuses ;
(iv) les inhibiteurs du fonctionnement des facteurs de croissance ;
(v) les agents anti-angiogéniques ;
(vi) les agents endommageant le système vasculaire ;
(vii) les thérapies anti-sens ;
(viii) les approches de thérapie génique ; et
(ix) les approches d'immunothérapie ;
dans la fabrication d'un médicament destiné à être utilisé dans le traitement du cancer.

18. Procédé de préparation d'un composé de formule (I), tel que défini dans la revendication 1, ou un sel pharmaceutiquement acceptable de celui-ci, lequel procédé comprend la transformation d'un composé de formule (II) en un composé de formule (I) par phosphorylation d'un groupement hydroxy approprié : 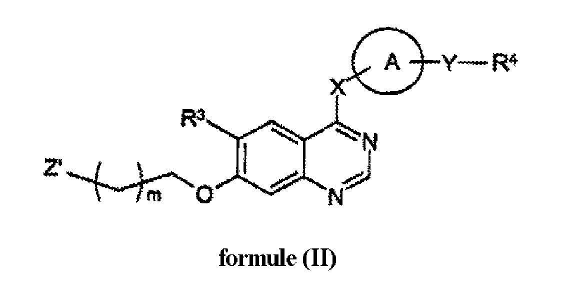 dans laquelle A, X, m, Y, R³ et R⁴ sont tels que définis pour la formule (I) ; et **Z'** est un groupement choisi parmi -NR^{1'}R^{2'}, hydroxy, C₃₋₆cycloalkyle, lequel C₃₋₆cycloalkyle est substitué par hydroxy ou C₁₋₄alkyle substitué par hydroxy, et un cycle à 4 à 7 chaînons lié par l'intermédiaire d'un atome de carbone, contenant un atome d'azote et contenant, éventuellement, un atome d'azote supplémentaire, lequel cycle peut être saturé, insaturé ou partiellement saturé, et lequel cycle est substitué sur le carbone ou l'azote par hydroxy ou C₁₋₄alkyle substitué par hydroxy, et lequel cycle est, éventuellement, encore substitué sur le carbone ou l'azote par 1, 2 ou 3 groupements halogéno ou C₁₋₄alkyle ; **R^{1'}** est un groupement choisi parmi -COR^{8'}, -CONR^{8'}R⁹ et C₁₋₆alkyle, lequel C₁₋₆alkyle est substitué par hydroxy et, éventuellement, encore substitué par 1 ou 2 groupements halogéno ou méthoxy ; **R^{2'}** est un groupement choisi parmi hydrogène, -COR¹⁰, -CONR¹⁰R¹¹ et C₁₋₆alkyle, lequel C₁₋₆alkyle est éventuellement substitué par 1, 2 ou 3 groupements halogéno ou C₁₋₄alcoxy, -S(O)ₚR¹¹ (où p est 0, 1 ou 2) ou hydroxy, ou R^{2'} est un groupement choisi parmi C₂₋₆alcényle, C₂₋₆alcynyle, C₃₋₆cycloalkyle et C₃₋₆cycloalkyl-C₁₋₄alkyle ; ou **R^{1'}** et **R^{2'}** ensemble avec l'azote auquel ils sont liés, forment un cycle à 4 à 7 chaînons contenant éventuellement un atome d'azote supplémentaire, lequel cycle peut être saturé, insaturé ou partiellement saturé, et lequel cycle est substitué sur le carbone ou l'azote par un groupement choisi parmi hydroxy et C₁₋₄alkyle, lequel C₁₋₄alkyle est substitué par hydroxy ou -NR^{8'}R⁹, et lequel cycle est, éventuellement, encore substitué sur le carbone ou l'azote par 1, 2 ou 3 groupements halogéno ou C₁₋₄alkyle ; et où **R**^{8'} est C₁₋₄alkyle substitué par hydroxy et, éventuellement, encore substitué par 1 ou 2 groupements halogéno ou méthoxy ;
et, ensuite, le cas échéant :
(i) la transformation d'un composé de formule (I) en un autre composé de formule (I) ; et/ou
(ii) l'élimination de tous les groupements protecteurs ; et/ou
(iii) la formation d'un sel pharmaceutiquement acceptable de celui-ci.
